# EUROPÄISCHE PATENTSCHRIFT

(11) **EP 3 689 324 B1**
(45) Veröffentlichungstag und Bekanntmachung des Hinweises auf die Patenterteilung: **20.11.2024**
(21) Anmeldenummer: 20150849.6
(22) Anmeldetag: 09.01.2020
(51) Int. Cl.: A61K 8/42, A61Q 19/00, A61P 17/00, C07D 333/20, C07D 409/12, C11D 3/34, A61K 31/192, C11D 3/32, A61K 8/49, A61K 31/381, A61P 1/02, A61P 17/02, A61P 31/02, C07D 317/60, C07C 235/20

(54) **NEUE KÜHLSTOFFE UND ZUBEREITUNGEN, DIE DIESE ENTHALTEN**
NEW COOLING AGENTS AND PREPARATIONS COMPRISING THEM
NOUVEAUX AGENTS RAFRAICHISSANTS ET PREPARATIONS LES CONTENANT

(30) Priorität: 04.02.2019 EP 19155329
(43) Veröffentlichungstag der Anmeldung: 05.08.2020
(73) Patentinhaber: Symrise AG, 37603 Holzminden Niedersachsen (DE)
(72) Erfinder: Join, Benoît, 37603 Holzminden (DE); Siegel, Sven, 37671 Höxter (DE); Brodhage, Rahim, 37671 Höxter (DE)
(74) Vertreter: Fabry, Bernd

(56) Entgegenhaltungen:
- WO-A1-2004/000789
- WO-A2-2012/061698
- DE-U1- 202012 013 357
- ALAIN NONCOVICH ET AL: "Discovery and development of a novel class of phenoxyacetyl amides as highly potent TRPM8 agonists for use as cooling agents", BIOORGANIC & MEDICINAL CHEMISTRY LETTERS, vol. 27, no. 16, 1 August 2017 (2017-08-01), AMSTERDAM, NL, pages 3931 - 3938, XP055405217, ISSN: 0960-894X, DOI: 10.1016/j.bmcl.2017.04.003
- ORDONEZ ET AL: "A convenient method for the preparation of chiral phosphonoacetamides and their Horner-Wadsworth-Emmons reaction", TETRAHEDRON ASYMMETRY, PERGAMON PRESS LTD, OXFORD, GB, vol. 18, no. 20, 29 October 2007 (2007-10-29), pages 2427 - 2436, XP022324332, ISSN: 0957-4166, DOI: 10.1016/J.TETASY.2007.09.033
- BROGGINI G ET AL: "Stereoselective intramolecular cycloadditions of homochiral N-alkenoyl aryl azides", TETRAHEDRON ASYMMETRY, PERGAMON PRESS LTD, OXFORD, GB, vol. 12, no. 8, 21 May 2001 (2001-05-21), pages 1201 - 1206, XP004245868, ISSN: 0957-4166, DOI: 10.1016/S0957-4166(01)00190-2

## Beschreibung

### GEBIET DER ERFINDUNG

Die Erfindung befindet sich auf dem Gebiet der physiologischen Kühlstoffe und betrifft neue Vertreter dieser Gruppe, Verfahren zu ihrer Herstellung sowie unterschiedliche Zubereitungen, die diese enthalten.

### TECHNOLOGISCHER HINTERGRUND

Physiologische Kühlwirkstoffe werden regelmäßig eingesetzt, um einen kühlen sensorischen Eindruck auf der Haut bzw. Schleimhaut, zum Beispiel auf der Schleimhaut im Mund-, Nasen- und/oder Rachenraum hervorzurufen, wobei allerdings tatsächlich keine physikalische Abkühlung wie zum Beispiel bei der Verdunstung von Lösungsmitteln stattfindet. Als physiologische Kühlwirkstoffe können sowohl Einzelkomponenten als auch Mischungen verwendet werden. Dabei ist zu berücksichtigen, dass nicht alle Verbindungen, die *in vitro* Rezeptoren beeinflussen, die (auch) an der Vermittlung einer physiologischen Kühlwirkung beteiligt sind, tatsächlich einen solchen Effekt *in vivo* auf der Haut oder auf Schleimhäuten erzeugen. Insbesondere wird ein solcher Effekt nicht immer identisch verlaufen. Das bedeutet zum Beispiel, dass die Stärke der vermittelten physiologischen Kühlwirkung sowie der Verlauf der Stärke der Kühlwirkung gegen Zeitdauer nicht alleine aus der Tatsache geschlossen werden können, dass eine bestimmte Verbindung ein Agonist eines an der Vermittlung eines Kühleindruckes beteiligten Rezeptors ist.

Der bekannteste physiologisch wirksame Kühlwirkstoff ist L-Menthol, der aber einige Nachteile besitzt, z. B. starker Geruchseindruck, eine hohe Flüchtigkeit und in höheren Konzentrationen einen bitteren und/oder scharfen Eigengeschmack, bzw. eine Haut reizende Wirkung.

### RELEVANTER STAND DER TECHNIK

Es wurde daher schon früher nach starken Kühlwirkstoffen gesucht, die nicht die nachteiligen Eigenschaften des L-Menthols aufweisen. So wurden z.B. Milchsäureester von Menthol(en) gemäß DE 2608226 A1 (H&R) und gemischte Carbonate mit Menthol(en) und Polyolen gemäß DE 4226043 A1 (SYMRISE) und Menthonketale gemäß EP 0507190 B1 (SYMRISE) beschrieben.

Menthylmonoester von Disäuren nach US 5,725,865 und US 5,843,466 (V MANE FILS) sind zwar interessante natürlich vorkommende Alternativen, können aber in sensorischen Tests nicht die Stärke der vorher beschriebenen Kühlwirkstoffe erreichen.

In J. Soc. Cosmet. Chem. 29, 185-200 (1978) wurden die Ergebnisse einer Studie an ca. 1200 Verbindungen präsentiert, bei der die Verbindungen L-Menthancarbonsäure-*N-*ethylamid ("WS3") und insbesondere *N*α-(L-Menthancarbonyl)-glycinethylester ("WS5") als die stärksten Kühlwirkstoffe gefunden wurden. Letzterer hat bei starker Wirkung aber den Nachteil, hydrolyseempfindlich zu sein und dabei die entsprechende freie Säure *N*α-(L-Menthancarbonyl)-glycin zu bilden, die selbst nur noch eine sehr schwache Kühlwirkung zeigt. Trotz der beschriebenen ausführlichen Untersuchungen ist eine systematische Vorhersage zu den Eigenschaften von potentiellen Kühlwirkstoffen, insbesondere zu deren Bitterkeit und/oder deren anderen trigeminalen Effekten nicht möglich und auch nicht beschrieben. So sind auch viele unter die Klasse der Menthancarbonsäureamide fallende Moleküle zwar stark kühlend, zeigen jedoch häufig gleichzeitig ausgeprägt bittere Noten wie z.B. die Menthancarbonsäure-N-(alkyloxyalkyl)amide nach JP 2004 059474 A2 (HASEGAWA) oder sind zusätzlich stark reizend, wie etwa der auch als WS5 bezeichnete N-[[5-Methyl-2-(1-methylethyl)cyclohexyl]carbonyl]glycinethylester gemäß US 2005 0222256 A1 (MILENNIUM SPECIALTY CHEM).

*N*α-(Menthancarbonyl)alkyloxyalkylamide wurden in JP 2004 059474 A2 (HASEGAWA) beschrieben. Diese haben bei starker Kühlwirkung und hoher Hydrolysestabilität jedoch den Nachteil, stark bitter zu sein, und sie sind somit in Nahrungsmitteln und auch in der Gesichtspflege dienenden kosmetischen Produkten nicht einsetzbar. Des Weiteren sind Menthylglyoxylate und ihre Hydrate in JP 2005 343795 A2 (TAKASAGO) als Kühlsubstanzen beschrieben worden.

Übersichten zu den bisher hergestellten und verwendeten Kühlwirkstoffen findet man in Perfumer & Flavorist 32(10), 20-35 (2007) sowie in der Monographie "Chemistry and Technology of Flavors and Fragrances", Blackwell Publishing Ltd, Oxford 2005, p. 212 - 222.

Der Kälte-Menthol-Rezeptor TRPM8 (auch bezeichnet als Cold-Membrane Receptor (CMR)1) gehört zur Familie der "Transient Receptor Potential Ion Channels", wird spezifisch in einer speziellen Gruppe von Neuronen exprimiert und bildet in der Zellmembran Poren aus (jeweils 4 Einheiten lagern sich dabei zu einem Tetramer zusammen), die selektiv Ca2+ Ionen passieren lassen. Das Protein weist 6 Transmembrandomänen auf und einen cytoplasmatischen C- sowie N-Terminus. Durch niedrige Temperaturen (bevorzugt 10 bis 25 °C) wird dieser Rezeptor stimuliert, es kommt zu einer Signaltransduktion, die vom Nervensystem als Kältegefühl interpretiert wird. Der Rezeptor ist erstmals 2002 als Kälterezeptor in mehreren Publikationen beschrieben worden.

Kühlende Verbindungen, wie z.B. Menthol, spielen bereits seit langem eine wichtige Rolle in der Geschmacks- und Riechstoffindustrie, um eine Assoziation mit Frische und Sauberkeit zu erzeugen. Für die Verbindung Menthol ist gezeigt worden, dass sie als ein natürlicher Modulator des Rezeptors TRPM8 wirkt. Durch Applikation von Menthol wird TRPM8 aktiviert, wodurch ein Ca²⁺-Einstrom in die Kälte-sensitiven Neuronen bewirkt wird. Das dadurch erzeugte elektrische Signal wird schließlich als Kältegefühl wahrgenommen. Überhöhte Menthol-Konzentrationen führen zu Irritation und einer anästhetischen Wirkung. Darüber hinaus sind in verschiedenen Publikationen Mentholderivate mit ähnlicher Wirkung beschrieben worden (vgl. *J*. *Soc. Cosmet. Chem.* 29, S. 185-200 (1978).

Es gibt auch vereinzelte, strukturell mit Menthol nicht verwandte Verbindungen, die eine signifikante TRPM8-Modulation bewirken, wie z.B. der Kühlstoff WS-23 oder in der Patentanmeldung WO 2007 019719 A1 (GIVAUDAN) aufgeführten Verbindungen.

Viele der bisher gefundenen Modulatoren von TRPM8 weisen jedoch Mängel in Bezug auf Wirkstärke, Wirkdauer, Haut-/Schleimhautreizung, Geruch, Geschmack, Löslichkeit sowie Flüchtigkeit auf.

Gegenstand der WO 2010 02694 A1 (BASF) sind einzelne Verbindungen zur Modulation des TRPM8-Rezeptors vorgeschlagen. So sind beispielsweise die folgenden Verbindungen offenbart:

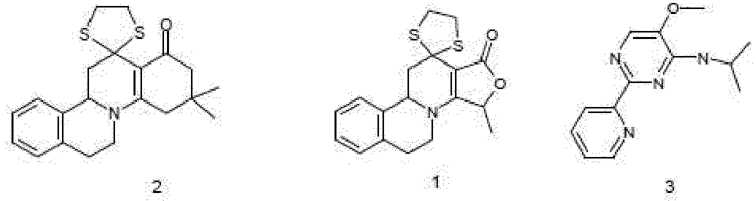

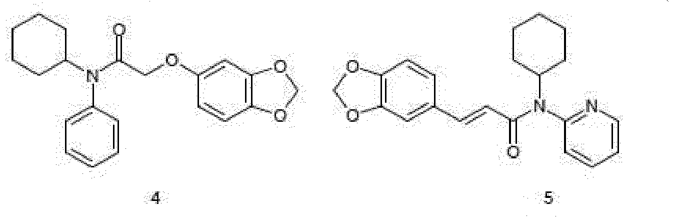

Diese Verbindungen sind auch kommerziell erhältlich:
- Verbindung 1 unter CAS Nummer: 99602-94-5 (3R-cis-Form) 20
- Verbindung 2 unter CAS Nummer: 165753-08-2
- Verbindung 3 unter CAS Nummer: 338771-57-6
- Verbindung 4 unter CAS Nummer: 878942-21-3
- Verbindung 5 unter CAS Nummer: 748783-13-3 (ohne Stereochemie)

Weitere Verbindungen zur Modulation des TRPM8-Rezeptors werden sowohl in der WO 2011 061330 A2 (BASF) vorgeschlagen werden.

Spezielle Kühlstoffe mit der Carboxamidstruktur (I) 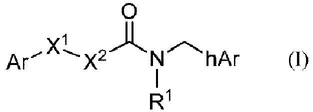 sind daneben auch aus der WO 2012 061698 A1 (SENOMYX) bekannt.

BIOORGANIC & MEDICAL CHEMISTRY LETTERS 27(16) (2017**)** stellt einen Übersichtsartikel zu einer Klasse von TRPM8 Antagonisten und deren Einsatz als Kühlstoffe dar.

WO 2004 000789 A1 (ELI LILLY) betrifft pharmazeutische Wirkstoffe, beispielsweise gegen Diabetes, die der Formel (I) folgen: 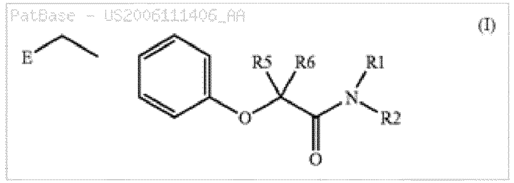 Auch hier gibt es eine zufällige Vorwegnahme durch die Strukturen 24, 26, 27 und 32, die

TETRAHEDRON LETTERS 18(20) p2427-2436 (2007**)** ist ein Aufsatz zu chiralen Phosphonoacetamiden. Die Stoffe haben keinen Bezug zu physiologischen Kühlstoffen.

TETRAHEDRON LETTERS 12(8) (2001**)** ist ebenfalls ein Aufsatz, der homochirale N-Alkenoxylarylazide betrifft. Auch hier besteht kein Zusammenhang mit physiologischen Kühlstoffen.

### AUFGABE DER ERFINDUNG

Primäre Aufgabe der vorliegenden Erfindung war es, neue Substanzen zu identifizieren, die eine besondere physiologische Kühlwirkung besitzen, vorzugsweise solche, die zu einer Modulation des TRPM8-Rezeptors führen, welche als Alternativen, vorzugsweise als geeignetere Mittel, zu den bisher bekannten Modulatoren einsetzbar sind. Solche Verbindungen sollten sich insbesondere auch für Anwendungen im Bereich Kosmetik, Ernährung, Textil, OTC-Produkte (z.B. Brandsalbe), Pharmaka (z.B. Tumorbehandlung, Blasenschwäche) oder Verpackungen eignen. Die anzugebenden Verbindungen bzw. Mischungen von Verbindungen sollten vorzugsweise einen möglichst schwachen Eigengeschmack zeigen, insbesondere wenig oder gar nicht bitter schmecken sowie möglichst nicht reizend sein.

Für die Lösung der erfindungsgemäßen Aufgabe waren dabei vor allem Mittel mit Wirkstoffen gesucht, die eine besonders langandauernde Kühlempfindung vermitteln können. Bevorzugt sollten diese Mittel darüber hinaus besonders intensive und/oder schnell einsetzende Kühleindrücke vermitteln können.

Auf der Mundschleimhaut zeigen viele der oben erwähnten konventionellen, im Stand der Technik bekannten Kühlsubstanzen alle ein mehr oder weniger identisches Kühlverhalten. Das durch sie vermittelte kühlende Frischempfinden setzt nach etwa 0,5 Minuten ein, flacht dann aber nach einem Höhepunkt bei 3 bis 5 Minuten wieder relativ schnell ab, wobei die Kühlung insgesamt für höchstens 30 Minuten deutlich wahrnehmbar ist und erfahrungsgemäß in Intensität und Dauer nur wenig durch eine Veränderung der Dosierung zu beeinflussen ist. Auf Seiten der Verbraucher besteht aber der Wunsch nach einer insbesondere lange andauernden Kühlwirkung, die für den Verwender mit einem entsprechenden Frische- und Wohlgefühl verbunden ist.

Eine weitere Aufgabe hat darin bestanden, geschmackliche Fehlnoten, die viele Aromen, insbesondere Süßstoffe wie etwa Vertreter der Gruppe der Stevioside aufweisen, auszugleichen. Dies betrifft insbesondere deren bittereren, adstringierenden und metallischen Nachgeschmack.

### BESCHREIBUNG DER ERFINDUNG

Ein erster Gegenstand der Erfindung betrifft Kühlstoffe ausgewählt aus der Gruppe, die gebildet wird von:

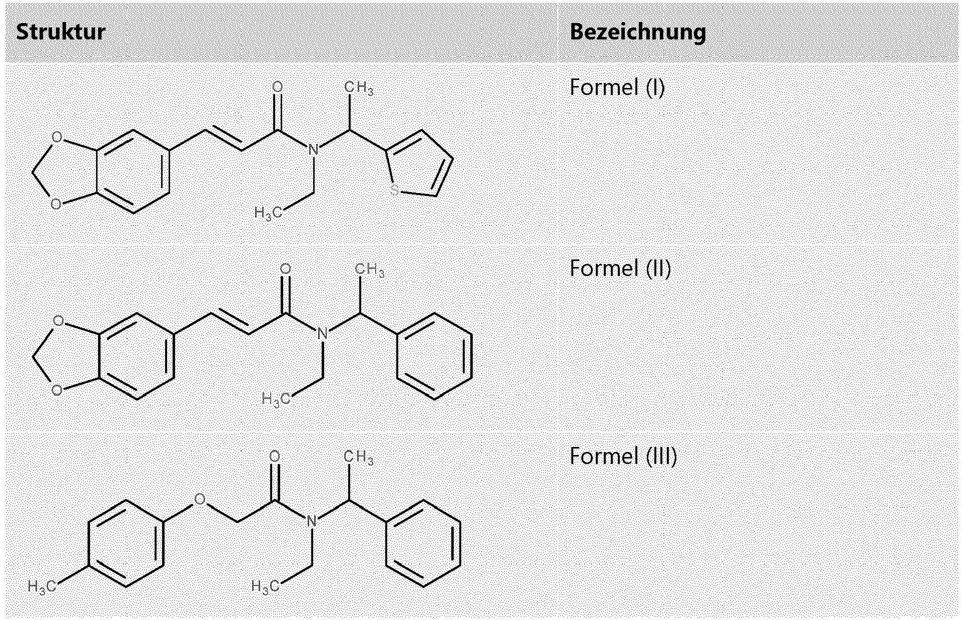

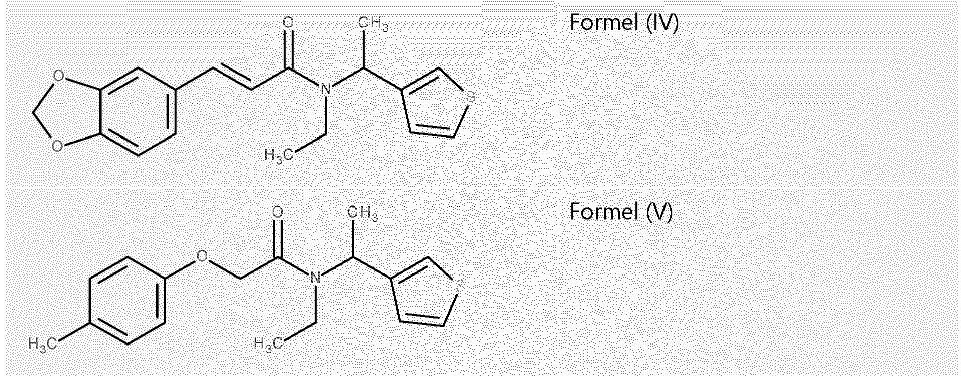

Überraschenderweise wurde gefunden, dass die erfindungsgemäßen Verbindungen die gemeinsame Eigenschaft haben, *in vivo* schon in niedriger Dosierung eine besonders lange Kühlwirkung auf Haut oder Schleimhaut zu erzielen. Dies war weder für die in dieser Anmeldung erwähnten TRPM8-Agonisten vorhersagbar, noch trifft es auf alle dieser Agonisten zu.

Bisher gab es im Stand der Technik keine Hinweise dazu, dass speziell die erfindungsgemäß zu verwendenden Verbindungen eine besonders geeignete Kühlwirkung zu vermitteln in der Lage sind. Um die langandauernde Kühlwirkung zu quantifizieren, können Vergleichsversuche mit Menthan-3-carbonsäure-N-ethylamid durchgeführt werden. Für diese Vergleichsversuche tauscht der Fachmann die erfindungsgemäß einzusetzende Verbindung oder die Verbindungen durch Menthan-3-carbonsäure-N-ethylamid (auch WS3), vorzugsweise in gleicher Konzentration, aus. Dann werden die Kühlwirkungen der jeweiligen Mittel miteinander verglichen. Sofern die erfindungsgemäß einzusetzenden Verbindungen im zu untersuchenden Mittel in einer Konzentration von höher als 100 ppm enthalten sind, ist es bevorzugt, dass für die Evaluierung, ob die Kühlwirkung gegenüber WS3 verlängert ist, das zu testende Mittel so zu verdünnen, dass die erfindungsgemäßen einzusetzenden Verbindungen in einer Konzentration von 100 ppm vorliegen. Selbstverständlich ist der gleiche Verdünnungsschritt auch für das Vergleichsmittel vorzunehmen, das WS3 enthält.

In diesem Zusammenhang ist bevorzugt, dass in den entsprechenden Vergleichen die Kühlwirkung der Mittel mit den erfindungsgemäß einzusetzenden Verbindungen vorzugsweise um wenigstens 10 Minuten, bevorzugt um wenigstens 15 Minuten, weiter bevorzugt wenigstens 20 Minuten und besonders bevorzugt wenigstens 30 Minuten gegenüber den Vergleichsversuchen verlängert ist.

Ebenso überraschend war, dass die erfindungsgemäßen Kühlstoffe in der Lage sind, die bekannten geschmacklichen Nachteile von Aromen, speziell auch von Süßstoffen wie etwa den Steviosiden zu maskieren. Dabei wird insbesondere der stechende, bittere und metallische Nachgeschmack schon bei Zugabe geringer Mengen überdeckt.

Schließlich wurde gefunden, dass die erfindunggemäßen Kühlstoffe in der Lage sind, die Stabilität von Emulsionen, speziell gegen Entmischung, zu verbessern.

### Erfindungsgemäße physiologische Kühlstoffe

Die erfindungsgemäßen physiologischen Kühlstoffe sind zwar noch nicht aus dem Stand der Technik bekannt, lassen sich jedoch nach allgemein bekannten Standardverfahren der präparativen organischen Chemie herstellen.

Im ersten Teil der Reaktion erfolgt die Umsetzung eines Ketons mit einer Aminverbindung unter Wasserabspaltung. Im zweiten Teil der Synthese wird eine entsprechende Carbonsäure in das Säurechlorid überführt und dieses dann mit der Aminverbindung aus dem ersten Schritt kondensiert.

Die zweistufige Reaktion ist im folgenden Schema verallgemeinert dargestellt:

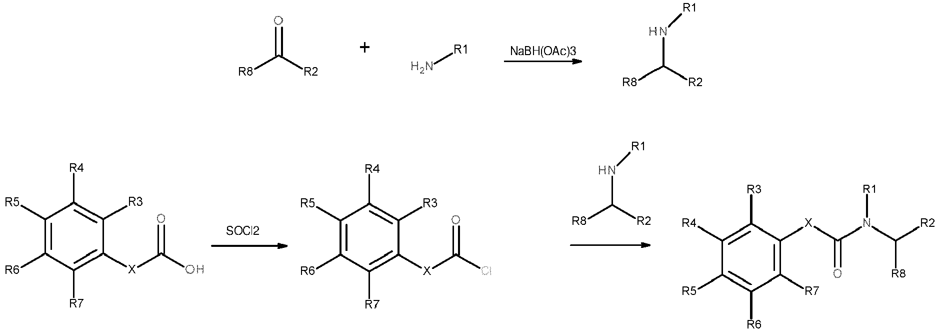

### Physiologische Kühlstoffmischungen

Ein weiterer Gegenstand der Erfindung betrifft eine physiologische Kühlstoffmischung enthaltend, bestehend aus oder im Wesentlichen bestehend aus:
(a) ein, zwei, drei oder mehreren Kühlstoffen der Formeln (I) bis (V) und gegebenenfalls
(b1) mindestens einem weiteren physiologischen Kühlstoff.

Der besondere Vorteil dieser Mischungen ist darin zu sehen, dass eine synergistische Verstärkung der Kühlleistung stattfindet.

Geeignete zusätzliche Kühlstoffe, die die Komponente (b1) bilden, sind ausgewählt aus der Gruppe, die gebildet wird von Menthol, Menthol Methyl Ether, Menthone Glyceryl Acetal (FEMA GRAS¹ 3807), Menthone Glyceryl Ketal (FEMA GRAS 3808), Menthyl Lactate (FEMA GRAS 3748), Menthol Ethylene Glycol Carbonate (FEMA GRAS 3805), Menthol Propylene Glycol Carbonate (FEMA GRAS 3806), Menthyl-N-ethyloxamat, Monomethyl Succinate (FEMA GRAS 3810), Monomenthyl Glutamate (FEMA GRAS 4006), Menthoxy-1,2-propanediol (FEMA GRAS 3784), Menthoxy-2-methyl-1,2-propandiol (FEMA GRAS 3849) sowie den Menthancarbonsäureestern und -amiden WS-3, WS-4, WS-5, WS-12, WS-14 und WS-30 sowie deren Gemischen.

Ein erster wichtiger Vertreter der Stoffe, die die Komponente (b1) bilden, stellt das Monomenthyl Succinate (FEMA GRAS 3810) dar, das als Stoff bereits 1963 von Brown & Williamson Tobacco Corp. patentiert worden **(**US 3,111,127**)** und als Kühlmittel Gegenstand der Schutzrechte US 5,725,865 und 5,843,466 (V.Mane Fils) ist. Sowohl das Succinat als auch das analoge Monomenthyl Glutarate (FEMA GRAS 4006) stellen wichtige Vertreter von Monomenthylestern auf Basis von Di- und Polycarbonsäuren dar:

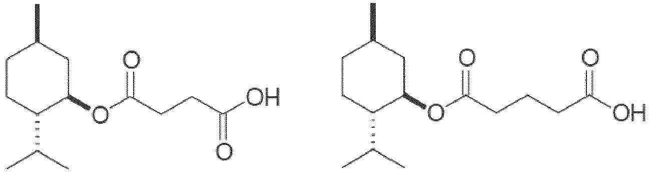

Beispiele für Anwendungen dieser Stoffe finden sich beispielsweise in den Druckschriften WO 2003 043431 (Unilever) oder EP 1332772 A1 (IFF).

Die nächste wichtige Gruppe von im Sinne der Erfindung bevorzugten Mentholverbindungen umfasst Carbonatester von Menthol und Polyolen, wie beispielsweise Glykolen, Glycerin oder Kohlenhydraten, wie beispielsweise Menthol Ethylenglycol Carbonate (FEMA GRAS 3805 = Frescolat^{®} MGC), Menthol Propylenglycol Carbonate (FEMA GRAS 3784 = Frescolat^{®} MPC), Menthol 2-Methyl-1,2-propandiol Carbonate (FEMA GRAS 3849) oder den entsprechenden Zuckerderivaten:
¹ FEMA steht für "Flavor and Extracts Manufacturers Association" und GRAS ist definiert als "Generally Regarded As Safe". Eine FEMA GRAS Bezeichnung bedeutet, dass die so gekennzeichnete Substanz nach Standardmethode getestet und für toxikologisch unbedenklich erachtet wird.

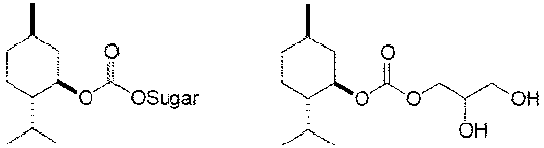

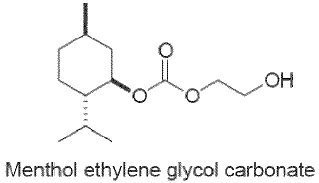

Die Verwendung derartiger Stoffe als Kühlstoff für Zigaretten ist beispielsweise Gegenstand der Druckschrift US 3,419,543 (Mold et al.) aus dem Jahre 1968; die Anwendung als physiologisches Kühlmittel wird in DE 4226043 A1 (H&R) beansprucht.

Im Sinne der Erfindung bevorzugt sind die Mentholverbindungen Menthyl Lactate (FEMA GRAS 3748 = Frescolat^{®} ML) und insbesondere das Menthone Glyceryl Acetal (FEMA GRAS 3807) bzw. Menthone Glyceryl Ketal (FEMA GRAS 3808), das unter der Bezeichnung Frescolat^{®} MGA vermarktet wird.

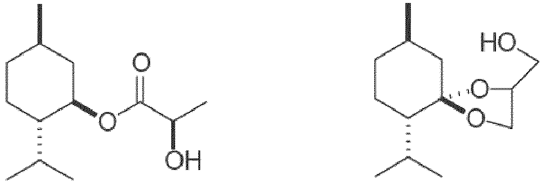

Erstere Struktur wird durch Veresterung von Milchsäure mit Menthol, letztere durch Acetalisierung von Menthon mit Glycerin gewonnen (vgl. DE 2608226 A1, H&R). In diese Gruppe von Verbindungen gehört auch das 3-(l-Menthoxy)-1,2,propandiol, das auch als Cooling Agent 10 bekannt ist (FEMA GRAS 3784, vgl. US 6,328,982, TIC), sowie das 3-(l-Menthoxy)-2-methyl-1,2,propandiol (FEMA GRAS 3849), das über eine zusätzliche Methylgruppe verfügt.

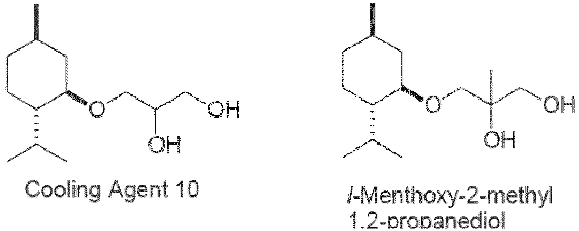

Die Herstellung des 3-(l-Menthoxy)-1,2,propandiol erfolgt beispielsweise ausgehend von Menthol nach dem folgenden Schema (vgl. US 4,459,425, Takagaso):

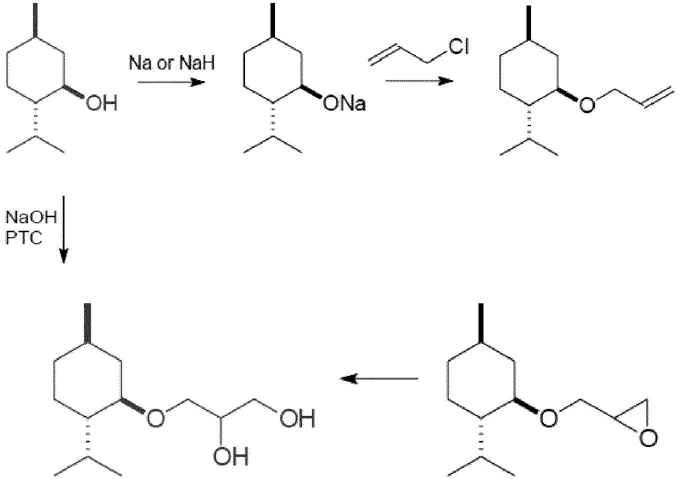

Alternative Routen, bei denen in der ersten Stufe Menthol mit Epichlorhydrin umgesetzt wird, wird in US 6,407,293 und US 6,515,188 (Takagaso) beschrieben. Im Folgenden wird eine Übersicht der bevorzugten Mentholverbindungen gegeben, die sich durch eine CO-Bindung auszeichnen:

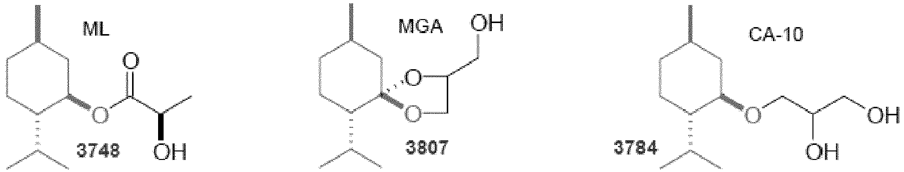

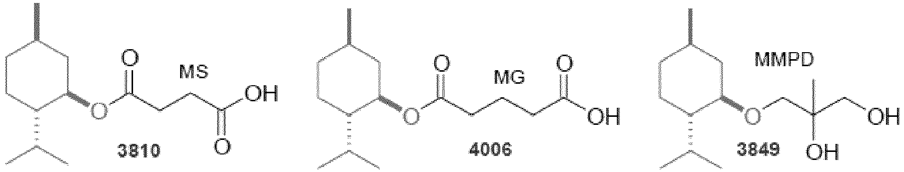

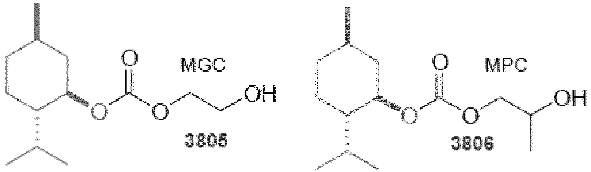

Als ganz besonders vorteilhaft haben sich unter diesen Stoffen Menthone Glyceryl Acetal/Ketal sowie das Menthyl Lactate sowie Menthol Ethylene Glycol Carbonate bzw. Menthol Propylene Glycol Carbonatw erwiesen, die die Anmelderin unter den Bezeichnungen Frescolat^{®} MGA, Frescolat^{®} ML, Frecolat^{®} MGC und Frescolat^{®} MPC vertreibt.

In den 70er Jahren des vergangenen Jahrhunderts wurden erstmals Mentholverbindungen entwickelt, die in der 3-Stellung über eine C-C-Bindung verfügen und von denen ebenfalls eine Reihe von Vertretern im Sinne der Erfindung eingesetzt werden können. Diese Stoffe werden im Allgemeinen als WS-Typen bezeichnet. Grundkörper ist ein Mentholderivat, bei dem die Hydroxyl- gegen eine Carboxylgruppe ersetzt ist (WS-1). Von dieser Struktur leiten sich alle weiteren WS-Typen ab, wie beispielsweise die ebenfalls im Sinne der Erfindung bevorzugten Spezies WS-3, WS-4, WS-5, WS-12, WS-14 und WS-30. Die beiden nachfolgenden Schaubilder zeigen die Synthesewege auf:

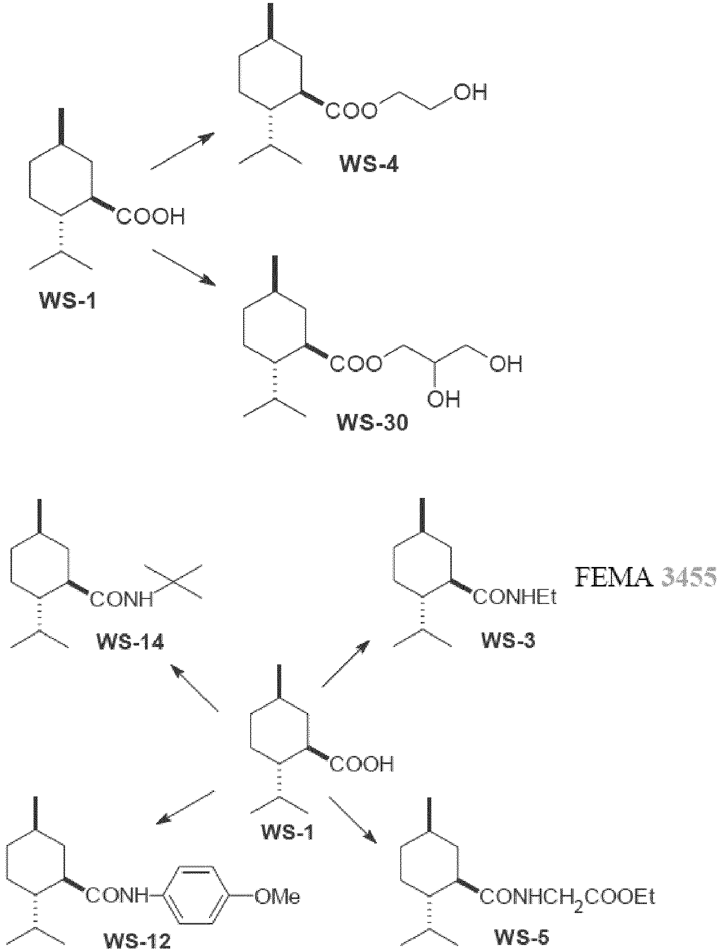

Die sich von WS-1 ableitenden Ester werden beispielsweise in US 4,157,384**,** die entsprechenden N-substituierten Amide in J. Soc. Cosmet. Chem. S. 185-200 (1978) beschrieben.

Weiterhin besonders bevorzugt ist der Kühlstoff 2-(p-tolyloxy)-N-(1H-pyrazol-5-yl)-N-((thiophen-2-yl)methyl)acetamid

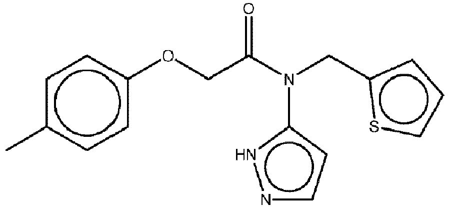

Die erfindungsgemäßen Mischungen können die Komponenten (a) und (b1) im Gewichtsverhältnis von etwa 1:99 bis etwa 99:1, vorzugsweise etwa 10:90 bis etwa 90:10, weiter bevorzugt etwa 25:75 bis etwa 75:25 und insbesondere etwa 40:60 bis 60:40 enthalten.

### AROMAZUBEREITUNGEN

Ein weiterer Gegenstand der Erfindung betrifft Aromazubereitungen enthaltend, bestehend aus oder im Wesentlichen bestehend aus
(a) ein, zwei, drei oder mehreren Kühlstoffen der Formeln (I) bis (V) und
(b2) mindestens einem Aromastoff.

Der besondere Vorteil dieser Mischungen ist darin zu sehen, dass die Kühlstoffe in der Lage sind, bereits in kleinen Konzentrationen unangenehme Geschmackseindrücke der Aromen, speziell von Süßstoffen zu maskieren.

Die erfindungsgemäßen Zubereitungen können einen oder mehrere Aromastoffe enthalten, die ausgewählt sind von der Gruppe, die gebildet wird von Acetophenon, Allylcapronat, alpha-lonon, beta-lonon, Anisaldehyd, Anisylacetat, Anisylformiat, Benzaldehyd, Benzothiazol, Benzylacetat, Benzylalkohol, Benzylbenzoat, beta-lonon, Butylbutyrat, Butylcapronat, Butylidenphthalid, Carvon, Camphen, Caryophyllen, Cineol, Cinnamylacetat, Citral, Citronellol, Citronellal, Citronellylacetat, Cyclohexylacetat, Cymol, Damascon, Decalacton, Dihydrocumarin, Dimethylanthranilat, Dimethylanthranilat, Dodecalacton, Ethoxyethylacetat, Ethylbuttersäure, Ethylbutyrat, Ethylcaprinat, Ethylcapronat, Ethylcrotonat, Ethylfuraneol, Ethylguajakol, Ethylisobutyrat, Ethylisovalerianat, Ethyllactat, Ethylmethylbutyrat, Ethylpropionat, Eucalyptol, Eugenol, Ethylheptylat, 4-(p-Hydroxyphenyl)-2-butanon, gamma-Decalacton, Geraniol, Geranylacetat, Geranylacetat, Grapefruitaldehyd, Methyldihydrojasmonat (z.B. Hedion^{®}), Heliotropin, 2-Heptanon, 3-Heptanon, 4-Heptanon, trans-2-Heptenal, cis-4-Heptenal, trans-2-Hexenal, cis-3-Hexenol, trans-2-Hexensäure, trans-3-Hexensäure, cis-2-Hexenylacetat, cis-3-Hexenylacetat, cis-3-Hexenylcapronat, trans-2-Hexenylcapronat, cis-3-Hexenylformiat, cis-2-Hexylacetat, cis-3-Hexylacetat, trans-2-Hexylacetat, cis-3-Hexylformiat, para-Hydroxybenzylaceton, Isoamylalkohol, Isoamylisovalerianat, Isobutylbutyrat, Isobutyraldehyd, Isoeugenolmethylether, Isopropylmethylthiazol, Laurinsäure, Leavulinsäure, Linalool, Linalooloxid, Linalylacetat, Menthol, Menthofuran, Methylanthranilat, Methylbutanol, Methylbuttersäure, 2-Methylbutylacetat, Methylcapronat, Methylcinnamat, 5-Methylfurfural, 3,2,2-Methylcyclopentenolon, 6,5,2-Methylheptenon, Methyldihydrojasmonat, Methyljasmonat, 2-Methylmethylbutyrat, 2-Methyl-2-Pentenolsäure, Methylthiobutyrat, 3,1-Methylthiohexanol, 3-Methylthiohexylacetat, Nerol, Nerylacetat, trans,trans-2,4-Nonadienal, 2,4-Nonadienol, 2,6-Nonadienol, 2,4-Nonadienol, Nootkaton, delta Octalacton, gamma Octalacton, 2-Octanol, 3-Octanol, 1,3-Octenol, 1-Octylacetat, 3-Octylacetat, Palmitinsäure, Paraldehyd, Phellandren, Pentandion, Phenylethylacetat, Phenylethylalkohol, Phenylethylalkohol, Phenylethylisovalerianat, Piperonal, Propionaldehyd, Propylbutyrat, Pulegon, Pulegol, Sinensal, Sulfurol, Terpinen, Terpineol, Terpinolen, 8,3-Thiomenthanon, 4,4,2-Thiomethylpentanon, Thymol, delta-Undecalacton, gamma-Undecalacton, Valencen, Valeriansäure, Vanillin, Acetoin, Ethylvanillin, Ethylvanillinisobutyrat (= 3-Ethoxy-4-isobutyryloxybenzaldehyd), 2,5-Dimethyl-4-hydroxy-3(2H)-furanon und dessen Abkömmlinge (dabei vorzugsweise Homofuraneol (= 2-Ethyl-4-hydroxy-5-methyl-3(2H)-furanon), Homofuronol (= 2-Ethyl-5-methyl-4-hydroxy-3(2H)-furanon und 5-Ethyl-2-methyl-4-hydroxy-3(2H)-furanon), Maltol und Maltol-Abkömmlinge (dabei vorzugsweise Ethylmaltol), Cumarin und Cumarin-Abkömmlinge, gamma-Lactone (dabei vorzugsweise gamma-Undecalacton, gamma-Nonalacton, gamma-Decalacton), delta-Lactone (dabei vorzugsweise 4-Methyldeltadecalacton, Massoilacton, Deltadecalacton, Tuberolacton), Methylsorbat, Divanillin, 4-Hydroxy-2(oder 5)-ethyl-5(oder 2)-methyl-3(2H)furanon, 2-Hydroxy-3-methyl-2-cyclopentenon, 3-Hydroxy-4,5-dimethyl-2(5H)-furanon, Essigsäureisoamylester, Buttersäureethylester, Buttersäure-n-butylester, Buttersäureisoamylester, 3-Methyl-buttersäureethylester, n-Hexansäureethylester, n-Hexansäureallylester, n-Hexansäure-n-butylester, n-Octansäureethylester, Ethyl-3-methyl-3-phenylglycidat, Ethyl-2-trans-4-cis-decadienoat, 4-(p-Hydroxyphenyl)-2-butanon, 1,1-Dimethoxy-2,2,5-trimethyl-4-hexan, 2,6-Dimethyl-5-hepten-1-al und Phenylacetaldehyd, 2-Methyl-3-(methylthio)furan, 2-Methyl-3-furanthiol, bis(2-Methyl-3-furyl)disulfid, Furfurylmercaptan, Methional, 2-Acetyl-2-thiazolin, 3-Mercapto-2-pentanon, 2,5-Dimethyl-3-furanthiol, 2,4,5-Trimethylthiazol, 2-Acetylthiazol, 2,4-Dimethyl-5-ethylthiazol, 2-Acetyl-1-pyrrolin, 2-Methyl-3-ethylpyrazin, 2-Ethyl-3,5-dimethylpyrazin, 2-Ethyl-3,6-dimethylpyrazin, 2,3-Diethyl-5-methylpyrazin, 3-Isopropyl-2-methoxypyrazin, 3-Isobutyl-2-methoxypyrazin, 2-Acetylpyrazin, 2-Pentylpyridin, (E,E)-2,4-Decadienal, (E,E)-2,4-Nonadienal, (E)-2-Octenal, (E)-2-Nonenal, 2-Undecenal, 12-Methyltridecanal, 1-Penten-3-on, 4-Hydroxy-2,5-dimethyl-3(2H)-furanon, Guajakol, 3-Hydroxy-4,5-dimethyl-2(5H)-furanon, 3-Hydroxy-4-methyl-5-ethyl-2(5H)-furanon, Zimtaldehyd, Zimtalkohol, Methylsalicylat, Isopulegol sowie (hier nicht explizit genannte) Stereoisomere, Enantiomere, Stellungsisomere, Diastereomere, cis/trans-Isomere bzw. Epimere dieser Substanzen.

Im Sinne der vorliegenden Erfindung kommen als Aromastoffe insbesondere auch künstliche wie auch natürliche Süßstoffe und Süßstoffverstärker in Betracht. Dies können ausgewählt sein aus der Gruppe, die gebildet wird von
- Zuckeralkohole (z.B. Erythritol, Threitol, Arabitol, Ribotol, Xylitol, Sorbitol, Mannitol, Dulcitol, Lactitol);
- Proteinen (z.B. Miraculin, Monellin, Thaumatin, Curculin, Brazzein);
- Synthetischen Süßstoffen (z.B. Magap, Natriumcyclamat, Acesulfam K, Neohesperidin Dihydrochalcon, Saccharin Natriumsalz, Aspartam, Superaspartam, Neotam, Alitam, Sucralose, Stevioside, Rebaudioside, Lugduname, Carrelame, Sucrononate, Sucrooctate, Monatin, Phenylodulcin);
- Süß schmeckendne Aminosäuren (z.B. Glycin, D-Leucin, D-Threonin, D-Asparagin, D-Phenylalanin, D-tryptophn, L-Prolin);
- süß schmeckenden niedermolekularen Substanzen, wie z.B. Hernandulcin, Dihydrochalconglykoside, Glycyrrhizin, Glycerrhetinsäure, ihre Derivate und Salze, Extrakte von Lakritz (Glycyrrhizza glabra ssp.), Lippia dulcis Extrakte, Momordica ssp. Extrakte oder
- pflanzlichen Extrakten wie z.B. Momordica grosvenori [Luo Han Guo] und die daraus gewonnenen Mogroside, Hydrangea dulcis oder Stevia ssp. (z.B. Stevia rebaudiana) Extrakte bzw. daraus gewonnene Stevioside.

Die erfindungsgemäßen Zubereitungen können die Komponenten (a) und (b2) im Gewichtsverhältnis von etwa 1:99 bis etwa 99:1, vorzugsweise etwa 10:90 bis etwa 90:10, weiter bevorzugt etwa 25:75 bis etwa 75:25 und insbesondere etwa 40:60 bis 60:40 enthalten.

### KAPSELN

In einer weiter bevorzugten Ausführungsform können die Kühlstoffe, die Mischungen oder der Zubereitungen in verkapselter Form vorliegen. Dies ist beispielsweise dann von besonderem Interesse, wenn die mit den Kühlstoffen beladenen Kapseln auf textile Oberflächen, beispielsweise als Bestandteil von Weichspülern oder Wäschenachbehandlungsmittel aufgebracht werden oder eine Ausrüstung durch Zwangsapplikation beispielsweise auf Strumpfhosen erfolgt.

Hierunter sind sphärische Aggregate zu verstehen, die mindestens einen festen oder flüssigen Kern enthalten, der von mindestens einer kontinuierlichen Hülle umschlossen ist. Die Duftstoffe können durch Überzugsmaterialien verkapselt werden und dabei als Makrokapseln mit Durchmessern von etwa 0,1 bis etwa 5 mm oder Mikrokapseln mit Durchmessern von etwa 0,0001 bis etwa 0,1 mm vorliegen.

### Überzugsmaterialien

Geeignete Überzugsmaterialien sind dabei beispielsweise Stärken, einschließlich deren Abbauprodukten sowie chemisch oder physikalisch erzeugten Derivaten (insbesondere Dextrine und Maltodextrine), Gelatine, Gummi Arabicum, Agar-Agar, Ghatti Gum, Gellan Gum, modifizierte und nicht-modifizierte Cellulosen, Pullulan, Curdlan, Carrageenane, Alginsäure, Alginate, Pektin, Inulin, Xanthan Gum und Mischungen von zwei oder mehreren dieser Substanzen.

Das feste Verkapselungsmaterial ist vorzugsweise eine Gelatine (insbesondere Schweine-, Rind-, Geflügel- und/oder Fischgelatine), wobei diese vorzugsweise einen Schwellfaktor von größer oder gleich 20, vorzugsweise von größer oder gleich 24 aufweist. Unter diesen Stoffen ist Gelatine besonders bevorzugt, da sie gut verfügbar ist und mit unterschiedlichen Schwellfaktoren bezogen werden kann.

Ebenfalls bevorzugt sind Maltodextrine (insbesondere auf Basis von Getreide, speziell Mais, Weizen, Tapioka oder Kartoffeln), die vorzugsweise DE-Werte im Bereich von 10 bis 20 aufweisen. Weiterhin bevorzugt sind Cellulosen (z.B. Celluloseether), Alginate (z.B. Natriumalginat), Carrageenan (z.B. beta-, jota-, lambda- und/oder kappa-Carrageenan), Gummi Arabicum, Curdlan und/oder Agar Agar.

Ebenfalls bevorzugt sind Alginatkapseln wie sie beispielsweise in den folgenden Schriften ausführlich beschrieben werden: EP 0389700 A1**,** US 4,251,195**,** US 6,214,376**,** WO 2003 055587 oder WO 2004 050069 A1**.**

In einer weiteren bevorzugten Ausführungsform besteht die Hülle der Kapseln aus Melamin-Formaldehydharzen oder Koazervationsprodukten aus kationischen Monomeren oder Biopolymeren (wie z.B. Chitosan) und anionischen Monomeren, wie beispielsweise (Meth)Acrylaten oder Alginaten.

### Verkapselungsverfahren

Bei den Kapseln handelt es sich im Allgemeinen um mit filmbildenden Polymeren umhüllte feindisperse flüssige oder feste Phasen, bei deren Herstellung sich die Polymere nach Emulgierung und Koazervation oder Grenzflächenpolymerisation auf dem einzuhüllenden Material niederschlagen. Nach einem anderen Verfahren werden geschmolzene Wachse in einer Matrix aufgenommen ("microsponge"), die als Mikropartikel zusätzlich mit filmbildenden Polymeren umhüllt sein können. Nach einem dritten Verfahren werden Partikel abwechselnd mit Polyelektrolyten unterschiedlicher Ladung beschichtet ("layer-by-layer"-Verfahren). Die mikroskopisch kleinen Kapseln lassen sich wie Pulver trocknen. Neben einkernigen Mikrokapseln sind auch mehrkernige Aggregate, auch Mikrosphären genannt, bekannt, die zwei oder mehr Kerne im kontinuierlichen Hüllmaterial verteilt enthalten. Ein- oder mehrkernige Mikrokapseln können zudem von einer zusätzlichen zweiten, dritten etc. Hülle umschlossen sein. Die Hülle kann aus natürlichen, halbsynthetischen oder synthetischen Materialien bestehen. Natürlich Hüllmaterialien sind beispielsweise Gummi Arabicum, Agar-Agar, Agarose, Maltodextrine, Alginsäure bzw. ihre Salze, z.B. Natrium- oder Calciumalginat, Fette und Fettsäuren, Cetylalkohol, Collagen, Chitosan, Lecithine, Gelatine, Albumin, Schellack, Polysaccharide, wie Stärke oder Dextran, Polypeptide, Proteinhydrolysate, Sucrose und Wachse. Halbsynthetische Hüllmaterialien sind unter anderem chemisch modifizierte Cellulosen, insbesondere Celluloseester und -ether, z.B. Celluloseacetat, Ethylcellulose, Hydroxypropylcellulose, Hydroxypropylmethylcellulose und Carboxymethylcellulose, sowie Stärkederivate, insbesondere Stärkeether und -ester. Synthetische Hüllmaterialien sind beispielsweise Polymere wie Polyacrylate, Polyamide, Polyvinylalkohol oder Polyvinylpyrrolidon.

Beispiele für Mikrokapseln des Stands der Technik sind folgende Handelsprodukte (in Klammern angegeben ist jeweils das Hüllmaterial) : *Hallcrest Microcapsules* (Gelatine, Gummi Arabicum), *Coletica Thalaspheres* (maritimes Collagen), *Lipotec Millicapseln* (Alginsäure, Agar-Agar), *Induchem Unispheres* (Lactose, mikrokristalline Cellulose, Hydroxypropylmethylcellulose); *Unicerin C30* (Lactose, mikrokristalline Cellulose, Hydroxypropylmethylcellulose), *Kobo Glycospheres* (modifizierte Stärke, Fettsäureester, Phospholipide), *Softspheres* (modifiziertes Agar-Agar) und *Kuhs Probiol Nanospheres* (Phospholipide) sowie *Primaspheres* und *Primasponges* (Chitosan, Alginate) und *Primasys* (Phospholipide).

Chitosanmikrokapseln und Verfahren zu ihrer Herstellung sind aus dem Stand der Technik hinlänglich bekannt **[**WO 01/01926**,** WO 01/01927**,** WO 01/01928**,** WO 01/01929**].** Mikrokapseln mit mittleren Durchmessern im Bereich von 0,0001 bis 5, vorzugsweise 0,001 bis 0,5 und insbesondere 0,005 bis 0,1 mm, bestehend aus einer Hüllmembran und einer die Wirkstoffe enthaltenden Matrix, können beispielsweise erhalten werden, indem man
(a) aus Gelbildnern, kationischen Polymeren und Wirkstoffen eine Matrix zubereitet,
(b) gegebenenfalls die Matrix in einer Ölphase dispergiert,
(c) die dispergierte Matrix mit wässrigen Lösungen anionischer Polymere behandelt und gegebenenfalls dabei die Ölphase entfernt.

Die Schritte (a) und (c) sind dabei insofern austauschbar, als man anstelle der kationischen Polymeren in Schritt (a) anionische Polymere einsetzt und umgekehrt.

Man kann die Kapseln auch erzeugen, indem man den Wirkstoff abwechselnd mit Schichten aus unterschiedlich geladenen Polyelektrolyten einhüllt (layer-by-layer-Technologie). In diesem Zusammenhang sei auf das Europäische Patent EP 1064088 B1 (Max-Planck Gesellschaft) verwiesen.

### KOSMETISCHE UND/ODER PHARMAZEUTISCHE ZUBEREITUNGEN

Ein weiterer Gegenstand der Erfindung betrifft kosmetische bzw. pharmazeutische Zubereitungen, die entweder einen oder mehrere der erfindungsgemäßen Kühlstoffe oder eine Zubereitung enthalten, welche einen Gehalt an diesen und weiteren Kühlstoffen aufweist. Dieser Gehalt beträgt vorzugsweise etwa 0,1 bis etwa Gew.-% und insbesondere etwa 0,5 bis 2 Gew.-% - bezogen auf die fertige Zubereitung.

Bei diesen Mitteln kann es sich beispielsweise um Hautpflegemittel, Körperpflegemittel, Haarpflegemittel, Sonnenschutzmittel oder dekorative Kosmetik handeln. Als pharmazeutische Mittel kommen beispielsweise Erkältungssäfte, Wundsalben oder Wundsprays in Frage. Ebenfalls möglich ist die Einarbeitung der Stoffe in Pflaster oder Tabletten, insbesondere wenn diese Wirkstoffe enthalten, die selbst einen unangenehmen Geschmack aufweisen.

Die erfindungsgemäßen Zubereitungen können weitere typische Hilfs- und Zusatzstoffe enthalten, wie beispielsweise milde Tenside, Ölkörper, Emulgatoren, Perlglanzwachse, Konsistenzgeber, Verdickungsmittel, Überfettungsmittel, Stabilisatoren, Polymere, Siliconverbindungen, Fette, Wachse, Lecithine, Phospholipide, UV-Lichtschutzfaktoren, Feuchthaltemittel, biogene Wirkstoffe, Antioxidantien, Deodorantien, Antitranspirantien, Antischuppenmittel, Filmbildner, Quellmittel, Insektenrepellentien, Selbstbräuner, Tyrosininhibitoren (Depigmentierungsmittel), Hydrotrope, Solubilisatoren, Konservierungsmittel, Parfümöle, Farbstoffe und dergleichen enthalten.

### Tenside

Als oberflächenaktive Stoffe können anionische, nichtionische, kationische und/oder amphotere bzw. zwitterionische Tenside enthalten sein, deren Anteil an den Mitteln üblicherweise bei etwa 1 bis 70, vorzugsweise 5 bis 50 und insbesondere 10 bis 30 Gew.-% beträgt.

Typische Beispiele für anionische Tenside sind Seifen, Alkylbenzolsulfonate, Alkansulfonate, Olefinsulfonate, Alkylethersulfonate, Glycerinethersulfonate, α-Methylestersulfonate, Sulfofettsäuren, Alkylsulfate, Alkylethersulfate, Glycerinethersulfate, Fettsäureethersulfate, Hydroxymischethersulfate, Monoglycerid(ether)sulfate, Fettsäureamid(ether)sulfate, Mono- und Dialkylsulfosuccinate, Mono- und Dialkylsulfosuccinamate, Sulfotriglyceride, Amidseifen, Ethercarbonsäuren und deren Salze, Fettsäureisethionate, Fettsäuresarcosinate, Fettsäuretauride, N-Acylaminosäuren, Alkyloligoglucosidsulfate, Proteinfettsäurekondensate (insbesondere pflanzliche Produkte auf Weizenbasis) und Alkyl(ether)phosphate. Sofern die anionischen Tenside Polyglycoletherketten enthalten, können diese eine konventionelle, vorzugsweise jedoch eine eingeengte Homologenverteilung aufweisen. Besonders bevorzugt sind in diesem Zusammenhang:
(a) Acylaminosäuresalze wie beispielsweise Acylglutamate, beispielsweise Natriumacylglutamat, Di-TEA-Palmitoylaspartat und Natriumcapryl/Caprinsäure-Glutamat, Acylpeptide, beispielsweise Palmitoyl-hydrolysiertes Milchprotein, Natriumcocoylhydrolysiertes Sojaprotein und Natrium / Kaliumcocoyl-hydrolysiertes Kollagen und Alaninate;
(b) Acyllactylate, Lauroyllactylat, Caproyllactylat
(c) Sulfate, wie beispielsweise
   - Alkylethersulfate wie insbesondere Natrium-, Ammonium-, Magnesium-, MIPA-, TIPA-Laurethsulfat, Natriummyrethsulfat und Natrium-C12-13-Parethsulfat;
   - Alkylsulfate, beispielsweise Natrium-, Ammonium- und TEA-Laurylsulfat;
   - Glyceridsulfate, wie beispielsweise Natriumcoco-Monoglyceridsulfat,
   - Amidsulfate, wie beispielsweise Magnesium-PEG-3-Cocamidsulfat.
(d) Sulfonate, wie beispielsweise
   - Alkylsulfonate,
   - Alkylarylsulfonate, insbesondere Natrium-C12-14-olefinsulfonat,
(e) Sulfosuccinate, beispielsweise Dioctylnatriumsulfosuccinat, Dinatriumlaurethsulfosuccinat, Dinatriumlaurylsulfosuccinat und Dinatriumundecylenamido-MEA-Sulfosuccinat;
(f) Sulfoacetate, wie Natriumlaurylsulfoacetat;
(g) Sarcosinate, beispielsweise Myristoylsarcosin, TEA-Lauroylsarcosinat, Natriumlauroylsarcosinat und Natriumcocoylsarcosinat,
(I) Isethionate, wie beispielsweise Natrium / Ammoniumcocoylisethionat,
(h) Taurate, beispielsweise Natriumlauroyltaurat und Natriummethylcocoyltaurat,
(i) Carboxylate, beispielsweise
   - Seifen wie etwa TEA-Stearat,
   - Ethercarboxylate, wie etwa Natriumlaureth-13-carboxylat und Natrium-PEG-6-cocamidcarboxylat,
(j) Phosphate wie etwa Cetylphosphat (Mono-, Di-Cetyl und ihre Gemische), Kaliumcetylphosphat, (Mono-, Di-Cetyl und ihre Gemische), DEA-Cetylphosphat (Mono-, Di-Cetyl und ihre Mischungen), DEA-Oleth-10-phosphat und Dilaureth-4-phosphat.

Typische Beispiele für nichtionische Tenside sind Fettalkoholpolyglycolether, Alkylphenolpolyglycolether, Fettsäurepolyglycolester, Fettsäureamidpolyglycolether, Fettaminpolyglycolether, alkoxylierte Triglyceride, Mischether bzw. Mischformale, gegebenenfalls partiell oxidierte Alk(en)yloligoglykoside bzw. Glucoronsäurederivate, Fettsäure-N-alkylglucamide, Proteinhydrolysate (insbesondere pflanzliche Produkte auf Weizenbasis), Polyolfettsäureester, Zuckerester, Sorbitanester, Polysorbate und Aminoxide. Sofern die nichtionischen Tenside Polyglycoletherketten enthalten, können diese eine konventionelle, vorzugsweise jedoch eine eingeengte Homologenverteilung aufweisen.

Kationische Tenside enthalten mindestens ein N-Atom, das kovalent an 4 Alkyl- oder Arylgruppen gebunden ist. Dies führt unabhängig vom pH zu einer positiven Ladung. Alkylbetain, Alkylamidopropylbetain und Alkylamidopropylhydroxysulfaine sind vorteilhaft. Die verwendeten kationischen Tenside können weiterhin vorzugsweise ausgewählt sein aus der Gruppe bestehend aus quaternären Ammoniumverbindungen, insbesondere Benzyltrialkylammoniumchloriden oder -bromiden, wie beispielsweise Benzyldimethylstearylammoniumchlorid, sowie Alkyltrialkylammoniumsalzen, beispielsweise Cetyltrimethylammoniumchlorid oder -bromid, Alkyldimethyl-hydroxyethylammoniumchloriden oder Bromiden, Dialkyldimethylammoniumchloriden oder -bromiden, Alkylamidethyltrimethylammoniumethersulfaten, Alkylpyridiniumsalzen, beispielsweise Lauryl- oder Cetylpyridiniumchlorid, Imidazolinderivaten und Verbindungen mit einem kationischen Charakter, wie Aminoxiden, beispielsweise Alkyldimethylaminoxid oder Alkylaminoethyldimethylaminoxid. Insbesondere Cetyltrimethylammoniumsalze werden vorteilhaft verwendet. Besonders bevorzugt sind dabei:
- Alkylamine,
- Alkylimidazole,
- ethoxylierte Amine,
- quaternäre Ammoniumsalze;
- RNH₂CH₂CH₂COO⁻ (bei pH = 7)
- RNHCH₂CH₂COO⁻ B⁺ (bei pH = 12) B + = ein beliebiges gewünschtes Kation sowie
- Esterquats.

Typische Beispiele für amphotere bzw. zwitterionische Tenside sind Alkylbetaine, Alkylamidobetaine, Aminopropionate, Aminoglycinate, Imidazoliniumbetaine und Sulfobetaine. Bei den genannten Tensiden handelt es sich ausschließlich um bekannte Verbindungen.

Typische Beispiele für besonders geeignete milde, d.h. besonders hautverträgliche Tenside sind Fettalkoholpolyglycolethersulfate, Monoglyceridsulfate, Mono- und/oder Dialkylsulfosuccinate, Fettsäureisethionate, Fettsäuresarcosinate, Fettsäuretauride, Fettsäureglutamate, α-Olefinsulfonate, Ethercarbonsäuren, Alkyloligoglucoside, Fettsäureglucamide, Alkylamidobetaine, Amphoacetale und/oder Proteinfettsäurekondensate, letztere vorzugsweise auf Basis von Weizenproteinen.

### Ölkörper

Als Ölkörper kommen beispielsweise Guerbetalkohole auf Basis von Fettalkoholen mit 6 bis 18, vorzugsweise 8 bis 10 Kohlenstoffatomen, Ester von linearen C₆-C₂₂-Fettsäuren mit linearen oder verzweigten C₆-C₂₂-Fettalkoholen bzw. Ester von verzweigten C₆-C₁₃-Carbonsäuren mit linearen oder verzweigten C₆-C₂₂-Fettalkoholen, wie z.B. Myristylmyristat, Myristylpalmitat, Myristylstearat, Myristylisostearat, Myristyloleat, Myristylbehenat, Myristylerucat, Cetylmyristat, Cetylpalmitat, Cetylstearat, Cetylisostearat, Cetyloleat, Cetylbehenat, Cetylerucat, Stearylmyristat, Stearylpalmitat, Stearylstearat, Stearylisostearat, Stearyloleat, Stearylbehenat, Stearylerucat, Isostearylmyristat, Isostearylpalmitat, Isostearylstearat, Isostearylisostearat, Isostearyloleat, Isostearylbehenat, Isostearyloleat, Oleylmyristat, Oleylpalmitat, Oleylstearat, Oleylisostearat, Oleyloleat, Oleylbehenat, Oleylerucat, Behenylmyristat, Behenylpalmitat, Behenylstearat, Behenylisostearat, Behenyloleat, Behenylbehenat, Behenylerucat, Erucylmyristat, Erucylpalmitat, Erucylstearat, Erucylisostearat, Erucyloleat, Erucylbehenat und Erucylerucat. Besonders bevorzugt sin Cetearyl ethylhexanoate, Cetearylnonanoat, Stearylheptanoat und Stearylcaprylat sowie deren Gemische.

Daneben eignen sich Ester von linearen C₆-C₂₂-Fettsäuren mit verzweigten Alkoholen, insbesondere 2-Ethylhexanol, Ester von C₁₈-C₃₈-Alkylhydroxycarbonsäuren mit linearen oder verzweigten C₆-C₂₂-Fettalkoholen, insbesondere Dioctylmalate, Ester von linearen oder verzweigten C6-C13 Carbonsäuren mit linearen oder verzweigten C6-C13 Alkoholen, wie beispielsweise Ethylhexylisononanoat, Ester von linearen und/oder verzweigten Fettsäuren mit mehrwertigen Alkoholen (wie z.B. Propylenglycol, Dimerdiol oder Trimertriol) und/oder Guerbetalkoholen, Triglyceride auf Basis C₆-C₁₀-Fettsäuren, flüssige Mono-/Di-/Triglyceridmischungen auf Basis von C₆-C₁₈-Fettsäuren, Ester von C₆-C₂₂-Fettalkoholen und/oder Guerbetalkoholen mit aromatischen Carbonsäuren, insbesondere Benzoesäure, Ester von C₂-C₁₂-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, pflanzliche Öle, verzweigte primäre Alkohole, substituierte Cyclohexane, lineare und verzweigte C₆-C₂₂-Fettalkoholcarbonate, wie z.B. Dicaprylyl Carbonate (Cetiol^{®} CC), Guerbetcarbonate auf Basis von Fettalkoholen mit 6 bis 18, vorzugsweise 8 bis 10 C Atomen, Ester der Benzoesäure mit linearen und/oder verzweigten C₆-C₂₂-Alkoholen (z.B. Finsolv^{®} TN), lineare oder verzweigte, symmetrische oder unsymmetrische Dialkylether mit 6 bis 22 Kohlenstoffatomen pro Alkylgruppe, wie z.B. Dicaprylyl Ether (Cetiol^{®} OE), Ringöffnungsprodukte von epoxidierten Fettsäureestern mit Polyolen, Siliconöle (Cyclomethicone, Siliciummethicontypen u.a.) und/oder aliphatische bzw. naphthenische Kohlenwasserstoffe, wie z.B. wie Squalan, Squalen oder Dialkylcyclohexane in Betracht.

Die Einsatzmenge kann hier bezogen auf die Endformulierung zwischen 5 und 80 Gew.-%, vorzugsweise zwischen 10 und 50 Gew.-% und insbesondere zwischen 20 und 40 Gew.-% liegen.

### Emulgatoren

Als Emulgatoren kommen beispielsweise nichtionogene Tenside aus mindestens einer der folgenden Gruppen in Frage:
- Anlagerungsprodukte von 2 bis 30 Mol Ethylenoxid und/ oder 0 bis 5 Mol Propylenoxid an lineare Fettalkohole mit 8 bis 22 C-Atomen, an Fettsäuren mit 12 bis 22 C-Atomen, an Alkylphenole mit 8 bis 15 C-Atomen in der Alkylgruppe sowie Alkylamine mit 8 bis 22 Kohlenstoffatomen im Alkylrest;
- Alkyl- und/oder Alkenyloligoglykoside mit 8 bis 22 Kohlenstoffatomen im Alk(en)ylrest und deren ethoxylierte Analoga;
- Anlagerungsprodukte von 1 bis 15 Mol Ethylenoxid an Ricinusöl und/oder gehärtetes Ricinusöl;
- Anlagerungsprodukte von 15 bis 60 Mol Ethylenoxid an Ricinusöl und/oder gehärtetes Ricinusöl;
- Partialester von Glycerin und/oder Sorbitan mit ungesättigten, linearen oder gesättigten, verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid;
- Partialester von Polyglycerin (durchschnittlicher Eigenkondensationsgrad 2 bis 8), Polyethylenglycol (Molekulargewicht 400 bis 5000), Trimethylolpropan, Pentaerythrit, Zuckeralkoholen (z.B. Sorbit), Alkylglucosiden (z.B. Methylglucosid, Butylglucosid, Laurylglucosid) sowie Polyglucosiden (z.B. Cellulose) mit gesättigten und/oder ungesättigten, linearen oder verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid;
- Mischester aus Pentaerythrit, Fettsäuren, Citronensäure und Fettalkohol und/oder Mischester von Fettsäuren mit 6 bis 22 Kohlenstoffatomen, Methylglucose und Polyolen, vorzugsweise Glycerin oder Polyglycerin.
- Wollwachsalkohole;
- Polysiloxan-Polyalkyl-Polyether-Copolymere bzw. entsprechende Derivate;
- Block-Copolymere z.B. Polyethylenglycol-30 Dipolyhydroxystearate;
- Polymeremulgatoren, z.B. Pemulen-Typen (TR-1,TR-2) von Lubrizol oder Cosmedia^{®} SP von BASF;
- Polyalkylenglycole sowie
- Glycerincarbonat.

Im Folgenden werden besonders geeignete Emulgatoren näher erläutert:

**Alkoxylate.** Die Anlagerungsprodukte von Ethylenoxid und/oder von Propylenoxid an Fettalkohole, Fettsäuren, Alkylphenole oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/ oder Propylenoxid und Substrat, mit denen die Anlagerungsreaktion durchgeführt wird, entspricht. C_{12/18}-Fettsäuremono- und -diester von Anlagerungsprodukten von Ethylenoxid an Glycerin sind als Rückfettungsmittel für kosmetische Zubereitungen bekannt.

**Alkyl- und/oder Alkenyloligoglykosid.** Alkyl- und/oder Alkenyloligoglycoside, ihre Herstellung und ihre Verwendung sind aus dem Stand der Technik bekannt. Ihre Herstellung erfolgt insbesondere durch Umsetzung von Glucose oder Oligosacchariden mit primären Alkoholen mit 8 bis 18 Kohlenstoffatomen. Bezüglich des Glycosidrestes gilt, daß sowohl Monoglycoside, bei denen ein cyclischer Zuckerrest glycosidisch an den Fettalkohol gebunden ist, als auch oligomere Glycoside mit einem Oligomerisationsgrad bis vorzugsweise etwa 8 geeignet sind. Der Oligomerisierungsgrad ist dabei ein statistischer Mittelwert, dem eine für solche technischen Produkte übliche Homologenverteilung zugrunde liegt.

**Partialglyceride.** Typische Beispiele für geeignete Partialglyceride sind Hydroxystearinsäuremonoglycerid, Hydroxystearinsäurediglycerid, Isostearinsäuremonoglycerid, Isostearinsäurediglycerid, Ölsäuremonoglycerid, Ölsäurediglycerid, Ricinolsäuremoglycerid, Ricinolsäurediglycerid, Linolsäuremonoglycerid, Linolsäurediglycerid, Linolensäuremonoglycerid, Linolensäurediglycerid, Erucasäuremonoglycerid, Erucasäurediglycerid, Weinsäuremonoglycerid, Weinsäurediglycerid, Citronensäuremonoglycerid, Citronendiglycerid, Äpfelsäuremonoglycerid, Äpfelsäurediglycerid sowie deren technische Gemische, die untergeordnet aus dem Herstellungsprozeß noch geringe Mengen an Triglycerid enthalten können. Ebenfalls geeignet sind Anlagerungsprodukte von 1 bis 30, vorzugsweise 5 bis 10 Mol Ethylenoxid an die genannten Partialglyceride.

**Sorbitanester.** Als Sorbitanester kommen Sorbitanmonoisostearat, Sorbitansesquiisostearat, Sorbitan-diisostearat, Sorbitantriisostearat, Sorbitanmonooleat, Sorbitansesquioleat, Sorbitan-dioleat, Sorbitantrioleat, Sorbitanmonoerucat, Sorbitansesquierucat, Sorbitandierucat, Sorbitantrierucat, Sorbitanmonoricinoleat, Sorbitansesquiricinoleat, Sorbitandiricinoleat, Sorbitantriricinoleat, Sorbitanmonohydroxystearat, Sorbitansesquihydroxystearat, Sorbitandihydroxystearat, Sorbitantrihydroxystearat, Sorbitanmonotartrat, Sorbitansesqui-tartrat, Sorbitanditartrat, Sorbitantritartrat, Sorbitanmonocitrat, Sorbitansesquicitrat, Sorbitandicitrat, Sorbitantricitrat, Sorbitanmonomaleat, Sorbitansesquimaleat, Sorbitan-dimaleat, Sorbitantrimaleat sowie deren technische Gemische. Ebenfalls geeignet sind Anlagerungsprodukte von 1 bis 30, vorzugsweise 5 bis 10 Mol Ethylenoxid an die genannten Sorbitanester.

**Polyglycerinester.** Typische Beispiele für geeignete Polyglycerinester sind Polyglyceryl-2 Dipolyhydroxystearate (Dehymuls^{®} PGPH), Polyglycerin-3-Diisostearate (Lameform^{®} TGI), Polyglyceryl-4 Isostearate (Isolan^{®} GI 34), Polyglyceryl-3 Oleate, Diisostearoyl Polyglyceryl-3 Diisostearate (Isolan^{®} PDI), Polyglyceryl-3 Methylglucose Distearate (Tego Care^{®} 450), Polyglyceryl-3 Beeswax (Cera Beilina^{®}), Polyglyceryl-4 Caprate (Polyglycerol Caprate T2010/90), Polyglyceryl-3 Cetyl Ether (Chimexane^{®} NL), Polyglyceryl-3 Distearate (Cremophor^{®} GS 32) und Polyglyceryl Polyricinoleate (Admul^{®} WOL 1403) Polyglyceryl Dimerate Isostearate sowie deren Gemische. Beispiele für weitere geeignete Polyolester sind die gegebenenfalls mit 1 bis 30 Mol Ethylenoxid umgesetzten Mono-, Di- und Triester von Trimethylolpropan oder Pentaerythrit mit Laurinsäure, Kokosfettsäure, Talgfettsäure, Palmitinsäure, Stearinsäure, Ölsäure, Behensäure und dergleichen.

**Anionische Emulgatoren.** Typische anionische Emulgatoren sind aliphatische Fettsäuren mit 12 bis 22 Kohlenstoffatomen, wie beispielsweise Palmitinsäure, Stearinsäure oder Behensäure, sowie Dicarbonsäuren mit 12 bis 22 Kohlenstoffatomen, wie beispielsweise Azelainsäure oder Sebacinsäure.

Ebenfalls geeignet sind Mono-, Di- und Trialkylphosphate sowie Mono-, Di- und/oder Tri-PEG-alkylphosphate und deren Salze, wie etwa Cetylphosphat Kaliumsalz sowie die Citratester, insbesondere Glyceryloleatcitrat und Glycerylstearylcitrat.

**Amphotere und kationische Emulgatoren.** Weiterhin können als Emulgatoren zwitterionische Tenside verwendet werden. Als zwitterionische Tenside werden solche oberflächenaktiven Verbindungen bezeichnet, die im Molekül mindestens eine quartäre Ammoniumgruppe und mindestens eine Carboxylat- und eine Sulfonatgruppe tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acylaminopropyl-N,N-dimethylammoniumglycinate, beispielsweise das Kokosacylaminopropyldimethyl-ammoniumglycinat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Besonders bevorzugt ist das unter der CTFA-Bezeichnung Cocamidopropyl Betaine bekannte Fettsäureamid-Derivat. Ebenfalls geeignete Emulgatoren sind ampholytische Tenside. Unter ampholytischen Tensiden werden solche oberflächenaktiven Verbindungen verstanden, die außer einer C_{8/18}-Alkyl- oder Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe.. Besonders bevorzugte ampholytische Tenside sind das N-Kokosalkylaminopropionat, das Kokosacylaminoethylaminopropionat und das C_{12/18}-Acylsarcosin. Schließlich kommen auch Kationtenside als Emulgatoren in Betracht, wobei solche vom Typ der Esterquats, vorzugsweise methylquaternierte Difettsäuretriethanolaminester-Salze, besonders bevorzugt sind.

Die Einsatzmenge der Emulgatoren liegt typisch im Bereich von etwa 0,5 bis etwa 10 Gew.-% und vorzugsweise etwa 1 bis etwa 5 Gew.-%.

### Fette und Wachse

Typische Beispiele für Fette sind Glyceride, d.h. feste oder flüssige pflanzliche oder tierische Produkte, die im Wesentlichen aus gemischten Glycerinestern höherer Fettsäuren bestehen, als Wachse kommen u.a. natürliche oder synthetische Wachse, wie z.B. Candelillawachs, Carnaubawachs, Japanwachs, Espartograswachs, Korkwachs, Guarumawachs, Reiskeimölwachs, Zuckerrohrwachs, Ouricurywachs, Montanwachs, Bienenwachs, Schellackwachs, Walrat, Lanolin (Wollwachs), Bürzelfett, Ceresin, Ozokerit (Erdwachs), Petrolatum, Paraffinwachse, Mikrowachse; chemisch modifizierte Wachse (Hartwachse), wie z.B. Montanesterwachse, Sasolwachse, hydrierte Jojobawachse sowie synthetische Wachse, wie z.B. Polyalkylenwachse und Polyethylenglycolwachse in Frage. Neben den Fetten kommen als Zusatzstoffe auch fettähnliche Substanzen, wie Lecithine und Phospholipide in Frage. Unter der Bezeichnung Lecithine versteht der Fachmann diejenigen Glycero-Phospholipide, die sich aus Fettsäuren, Glycerin, Phosphorsäure und Cholin durch Veresterung bilden. Lecithine werden in der Fachwelt daher auch häufig als Phosphatidylcholine (PC). Als Beispiele für natürliche Lecithine seien die Kephaline genannt, die auch als Phosphatidsäuren bezeichnet werden und Derivate der 1,2-Diacyl-sn-glycerin-3-phosphorsäuren darstellen. Dem gegenüber versteht man unter Phospholipiden gewöhnlich Mono- und vorzugsweise Diester der Phosphorsäure mit Glycerin (Glycerinphosphate), die allgemein zu den Fetten gerechnet werden. Daneben kommen auch Sphingosine bzw. Sphingolipide in Frage.

### Perlglanzwachse

Als Perlglanzwachse kommen beispielsweise in Frage: Alkylenglycolester, speziell Ethylenglycoldistearat; Fettsäurealkanolamide, speziell Kokosfettsäurediethanolamid; Partialglyceride, speziell Stearinsäuremonoglycerid; Ester von mehrwertigen, gegebenenfalls hydroxy-substituierte Carbonsäuren mit Fettalkoholen mit 6 bis 22 Kohlenstoffatomen, speziell langkettige Ester der Weinsäure; Fettstoffe, wie beispielsweise Fettalkohole, Fettketone, Fettaldehyde, Fettether und Fettcarbonate, die in Summe mindestens 24 Kohlenstoffatome aufweisen, speziell Lauron und Distearylether; Fettsäuren wie Stearinsäure, Hydroxystearinsäure oder Behensäure, Ringöffnungsprodukte von Olefinepoxiden mit 12 bis 22 Kohlenstoffatomen mit Fettalkoholen mit 12 bis 22 Kohlenstoffatomen und/oder Polyolen mit 2 bis 15 Kohlenstoffatomen und 2 bis 10 Hydroxylgruppen sowie deren Mischungen

### Konsistenzgeber und Verdickungsmittel

Als Konsistenzgeber kommen in erster Linie Fettalkohole oder Hydroxyfettalkohole mit 12 bis 22 und vorzugsweise 16 bis 18 Kohlenstoffatomen und daneben Partialglyceride, Fettsäuren oder Hydroxyfettsäuren in Betracht. Bevorzugt ist eine Kombination dieser Stoffe mit Alkyloligoglucosiden und/oder Fettsäure-N-methylglucamiden gleicher Kettenlänge und/oder Polyglycerinpoly-12-hydroxystearaten. Geeignete Verdickungsmittel sind beispielsweise Aerosil-Typen (hydrophile Kieselsäuren), Polysaccharide, insbesondere Xanthan-Gum, Guar-Guar, Agar-Agar, Alginate und Tylosen, Carboxymethylcellulose und Hydroxyethyl- und Hydroxypropylcellulose, ferner höhermolekulare Polyethylenglycolmono- und -diester von Fettsäuren, Polyacrylate, (z.B. Carbopole^{®} und Pemulen-Typen von Lubrizol; Synthalene^{®} von Sigma; Keltrol-Typen von Kelco; Sepigel-Typen von Seppic; Salcare-Typen von BASF), Polyacrylamide, Polymere, Polyvinylalkohol und Polyvinylpyrrolidon. Als besonders wirkungsvoll haben sich auch Bentonite, wie z.B. Bentone^{®} Gel VS-5PC (Elementis) erwiesen, bei dem es sich um eine Mischung aus Cyclopentasiloxan, Disteardimonium Hectorit und Propylencarbonat handelt. Weiter in Frage kommen Tenside, wie beispielsweise ethoxylierte Fettsäureglyceride, Ester von Fettsäuren mit Polyolen wie beispielsweise Pentaerythrit oder Trimethylolpropan, Fettalkoholethoxylate mit eingeengter Homologenverteilung oder Alkyloligoglucoside sowie Elektrolyte wie Kochsalz und Ammoniumchlorid.

### Überfettungsmittel und Stabilisatoren

Als Überfettungsmittel können Substanzen wie beispielsweise Lanolin und Lecithin sowie polyethoxylierte oder acylierte Lanolin- und Lecithinderivate, Polyolfettsäureester, Monoglyceride und Fettsäurealkanolamide verwendet werden, wobei die letzteren gleichzeitig als Schaumstabilisatoren dienen.

Als Stabilisatoren können Metallsalze von Fettsäuren, wie z.B. Magnesium-, Aluminium- und/oder Zinkstearat bzw. -ricinoleat eingesetzt werden.

### Polymere

Geeignete kationische Polymere sind beispielsweise kationische Cellulosederivate, wie z.B. eine quaternierte Hydroxyethylcellulose, die unter der Bezeichnung Polymer JR 400^{®} von Dow erhältlich ist, kationische Stärke, Copolymere von Diallylammoniumsalzen und Acrylamiden, quaternierte Vinylpyrrolidon/Vinylimidazol-Polymere, wie z.B. Luviquat^{®} (BASF), Kondensationsprodukte von Polyglycolen und Aminen, quaternierte Kollagenpolypeptide, wie beispielsweise Lauryldimonium Hydroxypropyl Hydrolyzed Collagen (Lamequat^{®}L/BASF), quaternierte Weizenpolypeptide, Polyethylenimin, kationische Siliconpolymere, wie z.B. Amodimethicone, Copolymere der Adipinsäure und Dimethylaminohydroxypropyldiethylentriamin (Cartaretine^{®}/Sandoz), Copolymere der Acrylsäure mit Dimethyl-diallylammoniumchlorid (Merquat^{®} 550/Lubrizol), Polyaminopolyamide sowie deren vernetzte wasserlöslichen Polymere, kationische Chitinderivate wie beispielsweise quaterniertes Chitosan, gegebenenfalls mikrokristallin verteilt, Kondensationsprodukte aus Dihalogenalkylen, wie z.B. Dibrombutan mit Bisdialkylaminen, wie z.B. Bis-Dimethylamino-1,3-propan, kationischer Guar-Gum, wie z.B. Jaguar^{®} CBS, Jaguar^{®} C-17, Jaguar^{®} C-16 (Solvay), quaternierte Ammoniumsalz-Polymere, wie z.B. Mirapol^{®} A-15, Mirapol^{®} AD-1, Mirapol^{®} AZ-1 (Solvay).

Als anionische, zwitterionische, amphotere und nichtionische Polymere kommen beispielsweise Vinylacetat/Crotonsäure-Copolymere, Vinylpyrrolidon/Vinylacrylat-Copolymere, Vinylacetat/Butylmaleat/ Isobornylacrylat-Copolymere, Methylvinylether/Maleinsäureanhydrid-Copolymere und deren Ester, unvernetzte und mit Polyolen vernetzte Polyacrylsäuren, Acrylamidopropyltrimethylammoniumchlorid/ Acrylat-Copolymere, Octylacrylamid/Methylmeth-acrylat/tert.Butylaminoethylmethacrylat/2-Hydroxypropylmethacrylat-Copolymere, Polyvinylpyrrolidon, Vinylpyrrolidon/Vinylacetat-Copolymere, Vinylpyrrolidon/ Dimethylaminoethylmethacrylat/Vinylcaprolactam-Terpolymere sowie gegebenenfalls derivatisierte Celluloseether und Silicone in Frage.

### Silikonverbindungen

Geeignete Siliconverbindungen sind beispielsweise Dimethylpolysiloxane, Methylphenylpolysiloxane, cyclische Silicone sowie amino-, fettsäure-, alkohol-, polyether-, epoxy-, fluor-, glykosid- und/oder alkylmodifizierte Siliconverbindungen, die bei Raumtemperatur sowohl flüssig als auch harzförmig vorliegen können. Weiterhin geeignet sind Simethicone, bei denen es sich um Mischungen aus Dimethiconen mit einer durchschnittlichen Kettenlänge von 200 bis 300 Dimethylsiloxan-Einheiten und hydrierten Silicaten handelt.

### UV-Lichtschutzfilter

Es wurde gefunden, dass die erfindungsgemäßen Diester insbesondere in der Lage sind, die Klebrigkeit, die für viele UV-Filter typisch ist, zu überwinden. Ein weiterer Gegenstand der vorliegenden Erfindung betrifft daher Zubereitungen, die neben den Diestern weiterhin wenigstens einen UV-Filter enthalten. Insbesondere sind solche Zubereitungen bevorzugt, welche
(a) 1,3-Propandiol dicaprylate/caprate und
(b) mindestens einen UV-Lichtschutzfilter

mit der Maßgabe enthalten, dass die Komponente (a) vollständig auf pflanzlicher Basis hergestellt worden ist. Ebenfalls typisch sind Zubereitungen enthaltend
   (a) etwa 0,1 bis etwa 30 Gew.-% 1,3-Propandiol dicaprylate/caprate und
   (b) etwa 1 bis etwa 50 Gew.-% UV-Lichtschutzfilter
mit der Maßgabe, dass sich die Mengenangaben mit Lösungsmitteln und weiteren kosmetischen Hilfs- und Zusatzstoffen zu 100 Gew.-% addieren.

Unter UV-Lichtschutzfilter (synonym auch öfters als Lichtschutzfaktoren bezeichnet) sind beispielsweise bei Raumtemperatur flüssig oder kristallin vorliegende organische Substanzen zu verstehen, die in der Lage sind, ultraviolette Strahlen zu absorbieren und die aufgenommene Energie in Form langwelliger Strahlung, z.B. Wärme wieder abzugeben. Üblicherweise sind die UV-Lichtschutzfilter in Mengen von 0,1 bis 50 und vorzugsweise 1 bis 45 Gew.-% zugegen.

Als typische UV-A-Filter kommen insbesondere Derivate des Benzoylmethans in Frage, wie beispielsweise 1-(4'-tert.Butylphenyl)-3-(4'-methoxyphenyl)propan-1,3-dion, 4-tert.-Butyl-4'-methoxydibenzoylmethan (Parsol^{®} 1789), 2-(4-Diethylamino-2-hydroxybenzoyl)-benzoic acid hexylester (Uvinul^{®} A Plus), 1-Phenyl-3-(4'-isopropylphenyl)-propan-1,3-dion sowie Enaminverbindungen. Besonders bevorzugt sind:
- Terephthalylidenedibornanesulphonic acid and salts (Mexoryl^{®}SX);
- Hexyl 2-(4-diethylamino-2-hydroxybenzoyl)benzoate (Uvinul^{®} A Plus); 2,2'-(1,4-phenylene)bis-[1H-benzimidazole-4,5-disulfonic acid], disodium salt (Neo Heliopan^{®} AP);
- Menthyl anthranilate (Neo Heliopan^{®}MA);
- Avobenzone (Neo Heliopan^{®} 357)
und deren Gemische.

UVB-Filter können öllöslich oder wasserlöslich sein. Als öllösliche Substanzen sind z.B. zu nennen:
- Octocylene;
- Homosalate;
- Octisalate;
- p-Aminobenzoesäure;
- Ethyl p-aminobenzoate+25EO;
- 2-Ethylhexyl p-dimethylaminobenzoat;
- Triethanolaminsalicylate (Neo Heliopan^{®} TS);
- Menthyl anthranilat (Neo Heliopan^{®}MA);
- 2-Ethylhexyl p-methoxycinnamat (Neo Heliopan^{®}AV);
- Isoamyl p-methoxycinnamat (Neo Heliopan^{®}E 1000);
- 2-Phenylbenzimidazolsulfonsäure (Neo Heliopan^{®} Hydro) und deren Salze;
- 3-(4'-Trimethylammonium)benzylidenebornan-2-one methylsulfat;
- 3-(4'-Sulpho)benzylidenebornan-2-on und dessen Salze;
- 3-(4'-Methylbenzyliden)-d,1-campher (Neo Heliopan^{®}MBC);
- N-[(2,4)-[2-(oxoborn-3-ylidene)methyl]benzyl]acrylamid polymer;
- 4,4'-[(6-[4-(1,1-dimethyl)aminocarbonyl)phenylamino]-1,3,5-triazine-2,4-diyl) diimino] bis(benzoesäure 2-ethylhexyl ester) (Uvasorb^{®}HEB);
- Benzylidenemalonate-polysiloxan (Parsol^{®}SLX);
- Tris(2-ethylhexyl)4,4',4"-(1,3,5-triazine-2,4,6-triyltriimino)tribenzoat (Uvinul^{®}T150);
- Methoxy propylamino cyclohexenylidene ethoxyethyl cyanoacetate
und deren Gemische.

Geeignete Breitbandfilter umfassen beispielsweise:
- 2-hydroxy-4-methoxybenzophenon-5-sulfonsäure (sulisobenzone, benzophenone-4) und deren Salze;
- 2-hydroxy-4-methoxybenzophenon (Neo Heliopan^{®} BB);
- Dinatrium 2,2'-dihydroxy-4,4'-dimethoxy-5,5'-disulphobenzophenon;
- Phenol,-(2H-benzotriazol-2-yl-4-methyl-6-(2-methyl-3-(1,3,3,3-tetramethyl-1-(trimethylsilyl)oxy)disiloxyanyl)propyl), (Mexoryl^{®}XL);
- 2,2'-methylenebis(6-(2H-benztriazol-2-yl)-4-1,1,3,3-tetramethylbutyl)-phenol (Tinosorb^{®}M);
- Tris-Biphenyl Triazine (Tinosorb^{®} A2B);
- Bemotrizinol (Neo Heliopan^{®}BMT);
- 2,4-bis[[(4-(3-sulphonato)-2-hydroxypropyloxy)-2-hydroxy]phenyl]-6-(4-methoxyphenyl)-1,3,5-triazin Natriumsalz;
- 2,4-bis[[(3-(2-propyloxy)-2-hydroxypropyloxy)-2-hydroxy]phenyl]-6-(4-methoxyphenyl)-1,3,5-triazin;
- 2,4-bis[[4-(2-ethylhexyloxy)-2-hydroxy]phenyl]-6-[4-(2-methoxyethyl¬carbonyl)phenylamino]-1,3,5-triazin;
- 2,4-bis[[4-(3-(2-propyloxy)-2-hydroxypropyloxy)-2-hydroxy]phenyl]-6-[4-(2-ethyl carboxyl)phenylamino]-1,3,5-triazin;
- 2,4-bis[[4-(2-ethylhexyloxy)-2-hydroxy]phenyl]-6-(1-methylpyrrol-2-yl)-1,3,5-triazin;
- 2,4-bis[[4-tris(trimethylsiloxysilylpropyloxy)-2-hydroxy]phenyl]-6-(4-methoxy phenyl)-1,3,5-triazin;
- 2,4-bis[[4-(2"-methylpropenyloxy)-2-hydroxy]phenyl]-6-(4-methoxyphenyl)-1,3,5-triazin;
- 2,4-bis[[4-(1',1',1',3',5',5',5'-heptamethylsiloxy-2"-methylpropyloxy)-2 hydroxy]phenyl]-6-(4-methoxyphenyl)-1,3,5-triazin;
- (5,6,5',6'-tetraphenyl-3,3'-(1,4-Phenylene)bis(1,2,4-triazine)
sowie deren Gemische.

Die UV-A und UV-B-Filter können selbstverständlich auch in Mischungen eingesetzt werden. Besonders günstige Kombinationen bestehen aus den Derivate des Benzoylmethans,, z.B. 4-tert.-Butyl-4'-methoxydibenzoylmethan (Parsol^{®} 1789) und 2-Cyano-3,3-phenylzimtsäure-2-ethyl-hexylester (Octocrylene) in Kombination mit Ester der Zimtsäure, vorzugsweise 4-Methoxyzimtsäure-2-ethylhexylester und/oder 4-Methoxyzimtsäurepropylester und/oder 4-Methoxyzimtsäureisoamylester. Vorteilhaft werden derartige Kombinationen mit wasserlöslichen Filtern wie z.B. 2-Phenylbenzimidazol-5-sulfonsäure und deren Alkali-, Erdalkali-, Ammonium-, Alkylammonium-, Alkanolammonium- und Glucammoniumsalze kombiniert.

### Pigmente, speziell Lichtschutzpigmente

Neben den genannten löslichen Stoffen kommen für diesen Zweck auch unlösliche Lichtschutzpigmente, nämlich feindisperse Metalloxide bzw. Salze in Frage. Beispiele für geeignete Metalloxide sind insbesondere Zinkoxid und Titandioxid und daneben Oxide des Eisens, Zirkoniums, Siliciums, Mangans, Aluminiums und Cers sowie deren Gemische. Als Salze können Silicate (Talk), Bariumsulfat oder Zinkstearat eingesetzt werden. Die Oxide und Salze werden in Form der Pigmente für hautpflegende und hautschützende Emulsionen und dekorative Kosmetik verwendet. Die Partikel sollten dabei einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pigmente können auch oberflächenbehandelt, d.h. hydrophilisiert oder hydrophobiert vorliegen. Typische Beispiele sind gecoatete Titandioxide, wie z.B. Titandioxid T 805 (Degussa) oder Eusolex^{®} T2000, Eusolex^{®} T, Eusolex^{®} T-ECO, Eusolex^{®} T-S, Eusolex^{®} T-Aqua, Eusolex^{®} T-45D (alle Merck), Uvinul TiOz (BASF). Als hydrophobe Coatingmittel kommen dabei vor allem Silicone und dabei speziell Trialkoxyoctylsilane oder Simethicone in Frage. In Sonnenschutzmitteln werden bevorzugt sogenannte Mikro- oder Nanopigmente eingesetzt. Vorzugsweise wird mikronisiertes Zinkoxid wie z.B. Z-COTE^{®} oder Z-COTE HP1^{®} verwendet.

### Feuchthaltemittel

Feuchthaltemittel dienen zur weiteren Optimierung der sensorischen Eigenschaften der Zusammensetzung sowie zur Feuchtigkeitsregulierung der Haut. Gleichzeitig wird die Kältestabilität der erfindungsgemäßen Zubereitungen, insbesondere im Falle von Emulsionen, erhöht. Die Feuchthaltemittel sind üblicherweise in einer Menge von 0,1 bis 15 Gew.-%, vorzugsweise 1 bis 10 Gew.-%, und insbesondere 5 bis 10 Gew.-% enthalten.

Erfindungsgemäß geeignet sind u.a. Aminosäuren, Pyrrolidoncarbonsäure, Milchsäure und deren Salze, Lactitol, Harnstoff und Harnstoffderivate, Harnsäure, Glucosamin, Kreatinin, Spaltprodukte des Kollagens, Chitosan oder Chitosansalze/-derivate, und insbesondere Polyole und Polyolderivate (z. B. Glycerin, Diglycerin, Triglycerin, Ethylenglycol, Propylenglycol, Butylenglycol, Erythrit, 1,2,6-Hexantriol, Polyethylenglycole wie PEG-4, PEG-6, PEG-7, PEG-8, PEG-9, PEG-10, PEG-12, PEG-14, PEG-16, PEG-18, PEG-20), Zucker und Zuckerderivate (u.a. Fructose, Glucose, Maltose, Maltitol, Mannit, Inosit, Sorbit, Sorbitylsilandiol, Sucrose, Trehalose, Xylose, Xylit, Glucuronsäure und deren Salze), ethoxyliertes Sorbit (Sorbeth-6, Sorbeth-20, Sorbeth-30, Sorbeth-40), Honig und gehärteter Honig, gehärtete Stärkehydrolysate sowie Mischungen aus gehärtetem Weizenprotein und PEG-20-Acetatcopolymer. Erfindungsgemäß bevorzugt geeignet als Feuchthaltemittel sind Glycerin, Diglycerin, Triglycerin und Butylenglycol.

### Biogene Wirkstoffe und Antioxidantien

Unter biogenen Wirkstoffen sind beispielsweise Tocopherol, Tocopherolacetat, Tocopherolpalmitat, Ascorbinsäure, (Desoxy)Ribonucleinsäure und deren Fragmentierungsprodukte, β-Glucane, Retinol, Bisabolol, Allantoin, Phytantriol, Panthenol, AHA-Säuren, Aminosäuren, Ceramide, Pseudoceramide, essentielle Öle, Pflanzenextrakte, wie z.B. Prunusextrakt, Bambaranussextrakt und Vitaminkomplexe zu verstehen.

Antioxidantien unterbrechen die photochemische Reaktionskette, welche ausgelöst wird, wenn UV-Strahlung in die Haut eindringt. Typische Beispiele hierfür sind Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Butioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Äpfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Superoxid-Dismutase, Zink und dessen Derivate (z.B. ZnO, ZnSO₄) Selen und dessen Derivate (z.B. Selen-Methionin), Stilbene und deren Derivate (z.B. Stilbenoxid, trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

### Deodorantien und keimhemmende Mittel

Kosmetische Deodorantien (Desodorantien) wirken Körpergerüchen entgegen, überdecken oder beseitigen sie. Körpergerüche entstehen durch die Einwirkung von Hautbakterien auf apokrinen Schweiß, wobei unangenehm riechende Abbauprodukte gebildet werden. Dementsprechend enthalten Deodorantien Wirkstoffe, die als keimhemmende Mittel, Enzyminhibitoren, Geruchsabsorber oder Geruchsüberdecker fungieren.

**Keimhemmende Mittel.** Als keimhemmende Mittel sind grundsätzlich alle gegen grampositive Bakterien wirksamen Stoffe geeignet, wie z. B. 2-Methyl-5-cyclohexylpentanol, 1,2-Decylenglycol, 4-Hydroxybenzoesäure und ihre Salze und Ester, N-(4-Chlorphenyl)-N'-(3,4-dichlorphenyl)harnstoff, 2,4,4'-Trichlor-2'-hydroxy-diphenylether (Triclosan), 4-Chlor-3,5-dimethyl-phenol, 2,2'-Methylen-bis(6-brom-4-chlorphenol), 3-Methyl-4-(1-methylethyl)-phenol, 2-Benzyl-4-chlorphenol, 3-(4-Chlorphenoxy)-1,2-propandiol, 3-lod-2-propinylbutylcarbamat, Chlorhexidin, 3,4,4'-Trichlorcarbanilid (TTC), antibakterielle Riechstoffe, Thymol, Thymianöl, Eugenol, Nelkenöl, Menthol, Minzöl, Farnesol, Phenoxyethanol, Glycerinmonocaprinat, Glycerinmonocaprylat, Glycerinmonolaurat (GML), Diglycerinmonocaprinat (DMC), Salicylsäure-N-alkylamide wie z. B. Salicylsäure-n-octylamid oder Salicylsäure-n-decylamid.

**Enzyminhibitoren.** Als Enzyminhibitoren sind beispielsweise Esteraseinhibitoren geeignet. Hierbei handelt es sich vorzugsweise um Trialkylcitrate wie Trimethylcitrat, Tripropylcitrat, Triisopropylcitrat, Tributylcitrat und insbesondere Triethylcitrat (Hydagen^{®} CAT). Die Stoffe inhibieren die Enzymaktivität und reduzieren dadurch die Geruchsbildung. Weitere Stoffe, die als Esteraseinhibitoren in Betracht kommen, sind Sterolsulfate oder -phosphate, wie beispielsweise Lanosterin-, Cholesterin-, Campesterin-, Stigmasterin- und Sitosterinsulfat bzw -phosphat, Dicarbonsäuren und deren Ester, wie beispielsweise Glutarsäure, Glutarsäuremonoethylester, Glutarsäurediethylester, Adipinsäure, Adipinsäuremonoethylester, Adipinsäurediethylester, Malonsäure und Malonsäurediethylester, Hydroxycarbonsäuren und deren Ester wie beispielsweise Citronensäure, Äpfelsäure, Weinsäure oder Weinsäurediethylester, sowie Zinkglycinat.

**Geruchsabsorber.** Als Geruchsabsorber eignen sich Stoffe, die geruchsbildende Verbindungen aufnehmen und weitgehend festhalten können. Sie senken den Partialdruck der einzelnen Komponenten und verringern so auch ihre Ausbreitungsgeschwindigkeit. Wichtig ist, daß dabei Parfums unbeeinträchtigt bleiben müssen. Geruchsabsorber haben keine Wirksamkeit gegen Bakterien. Sie enthalten beispielsweise als Hauptbestandteil ein komplexes Zinksalz der Ricinolsäure oder spezielle, weitgehend geruchsneutrale Duftstoffe, die dem Fachmann als "Fixateure" bekannt sind, wie z. B. Extrakte von Labdanum bzw. Styrax oder bestimmte Abietinsäurederivate. Als Geruchsüberdecker fungieren Riechstoffe oder Parfümöle, die zusätzlich zu ihrer Funktion als Geruchsüberdecker den Deodorantien ihre jeweilige Duftnote verleihen. Als Parfümöle seien beispielsweise genannt Gemische aus natürlichen und synthetischen Riechstoffen. Natürliche Riechstoffe sind Extrakte von Blüten, Stengeln und Blättern, Früchten, Fruchtschalen, Wurzeln, Hölzern, Kräutern und Gräsern, Nadeln und Zweigen sowie Harzen und Balsamen. Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, p-tert.-Butylcyclohexylacetat, Linalylacetat, Phenylethylacetat, Linalylbenzoat, Benzylformiat, Allylcyclohexylpropionat, Styrallylpropionat und Benzylsalicylat. Zu den Ethern zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labdanumöl und Lavandinöl. Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evernyl, Iraldein gamma, Phenylessigsäure, Geranylacetat, Benzylacetat, Rosenoxid, Romilat, Irotyl und Floramat allein oder in Mischungen, eingesetzt.

**Antitranspirantien.** Antitranspirantien (Antiperspirantien) reduzieren durch Beeinflussung der Aktivität der ekkrinen Schweißdrüsen die Schweißbildung, und wirken somit Achselnässe und Körpergeruch entgegen. Wässrige oder wasserfreie Formulierungen von Antitranspirantien enthalten typischerweise folgende Inhaltsstoffe:
- adstringierende Wirkstoffe,
- Ölkomponenten,
- nichtionische Emulgatoren,
- Coemulgatoren,
- Konsistenzgeber,
- Hilfsstoffe wie z. B. Verdicker oder Komplexierungsmittel und/oder
- nichtwässrige Lösungsmittel wie z. B. Ethanol, Propylenglykol und/oder Glycerin.
Als adstringierende Antitranspirant-Wirkstoffe eignen sich vor allem Salze des Aluminiums, Zirkoniums oder des Zinks. Solche geeigneten antihydrotisch wirksamen Wirkstoffe sind z.B. Aluminiumchlorid, Aluminiumchlorhydrat, Aluminiumdichlorhydrat, Aluminiumsesquichlorhydrat und deren Komplexverbindungen z. B. mit Propylenglycol-1,2. Aluminiumhydroxyallantoinat, Aluminiumchloridtartrat, Aluminium-Zirkonium-Trichloro-hydrat, Aluminium-Zirkonium-tetrachlorohydrat, Aluminium-Zirkonium-pentachlorohydrat und deren Komplexverbindungen z. B. mit Aminosäuren wie Glycin. Daneben können in Antitranspirantien übliche öllösliche und wasserlösliche Hilfsmittel in geringeren Mengen enthalten sein. Solche öllöslichen Hilfsmittel können z.B. sein:
- entzündungshemmende, hautschützende oder wohlriechende ätherische Öle,
- synthetische hautschützende Wirkstoffe und/oder
- öllösliche Parfümöle.

Übliche wasserlösliche Zusätze sind z.B. Konservierungsmittel, wasserlösliche Duftstoffe, pH-Wert-Stellmittel, z.B. Puffergemische, wasserlösliche Verdickungsmittel, z.B. wasserlösliche natürliche oder synthetische Polymere wie z.B. Xanthan-Gum, Hydroxyethylcellulose, Polyvinylpyrrolidon oder hochmolekulare Polyethylenoxide.

### Filmbildner

Gebräuchliche Filmbildner sind beispielsweise Chitosan, mikrokristallines Chitosan, quaterniertes Chitosan, Polyvinylpyrrolidon, Vinylpyrrolidon-Vinylacetat-Copolymerisate, Polymere der Acrylsäurereihe, quaternäre Cellulose-Derivate, hydrolysierte Jojobester, Kollagen, Hyaluronsäure bzw. deren Salze und ähnliche Verbindungen.

### Antischuppenwirkstoffe

Als Antischuppenwirkstoffe kommen Pirocton Olamin (1-Hydroxy-4-methyl-6-(2,4,4-trimythylpentyl)-2-(1H)-pyridinonmonoethanolaminsalz), Crinipan^{®} AD (Climbazole), Ketoconazol^{®}, (4-Acetyl-1-{-4-[2-(2.4-dichlorphenyl) r-2-(1H-imidazol-1-ylmethyl)-1,3-dioxylan-c-4-ylmethoxyphenyl}piperazin, Ketoconazol, Elubiol, Selendisulfid, Schwefel kolloidal, Schwefelpolyehtylenglykolsorbitanmonooleat, Schwefelrizinolpolyehtoxylat, Schwfel-teer Destillate, Salicylsäure (bzw. in Kombination mit Hexachlorophen), Undexylensäure Monoethanolamid Sulfosuccinat Na-Salz, Lamepon^{®} UD (Protein-Undecylensäurekondensat), Zinkpyrithion, Aluminiumpyrithion und Magnesiumpyrithion / Dipyrithion-Magnesiumsulfat in Frage.

### Quellmittel

Als Quellmittel für wässrige Phasen können Montmorillonite, Clay Mineralstoffe, Pemulen sowie alkylmodifizierte Carbopoltypen (Lubrizol) dienen. Weitere geeignete Polymere bzw. Quellmittel können der Übersicht von R.Lochhead in Cosm.Toil. 108, 95 (1993**)** entnommen werden.

### Insektenrepellentien

Als Insekten-Repellentien kommen N,N-Diethyl-m-toluamid, 1,2-Pentandiol oder Ethyl Butylacetylaminopropionate in Frage. Als Selbstbräuner eignet sich Dihydroxyaceton. Als Tyrosinhinbitoren, die die Bildung von Melanin verhindern und Anwendung in Depigmentierungsmitteln finden, kommen beispielsweise Arbutin, Ferulasäure, Kojisäure, Cumarinsäure und Ascorbinsäure (Vitamin C) in Frage.

### Inhaltsstoffe für Mund- und Zahnpflegemittel

Unter Zahnpasten oder Zahncremes werden im allgemeinen gelförmige oder pastöse Zubereitungen aus Wasser, Verdickungsmitteln, Feuchthaltemitteln, Schleif- oder Putzkörpern, Tensiden, Süßmitteln, Aromastoffen, deodorierenden Wirkstoffen sowie Wirkstoffen gegen Mund- und Zahnerkrankungen verstanden. In die erfindungsgemäßen Zahnpasten können alle üblichen Putzkörper, wie z. B. Kreide, Dicalciumphosphat, unlösliches Natriummetaphosphat, Aluminiumsilikate, Calciumpyrophosphat, feinteilige Kunstharze, Kieselsäuren, Aluminiumoxid und Aluminiumoxidtrihydrat eingesetzt werden.

Bevorzugt geeignete Putzkörper für die erfindungsgemäßen Zahnpasten sind vor allem feinteilige Xerogelkieselsäuren, Hydrogelkieselsäuren, Fällungskieselsäuren, Aluminiumoxid-trihydrat und feinteiliges alpha -Aluminiumoxid oder Mischungen dieser Putzkörper in Mengen von 15 bis 40 Gew.-% der Zahnpasta. Als Feuchthaltemittel kommen vorwiegend niedermolekulare Polyethylenglykole, Glycerin, Sorbit oder Mischungen dieser Produkte in Mengen bis zu 50 Gew.-% in Frage. Unter den bekannten Verdickungsmitteln sind die verdickenden, feinteiligen Gelkieselsäuren und Hydrokolloide, wie z. B. Carboxymethylcellulose, Hydroxyethylcellulose, Hydroxypropylguar, Hydroxyethylstärke, Polyvinylpyrrolidon, hochmolekulares Polyethylenglykol, Pflanzengummen wie Traganth, Agar-Agar, Carragheenmoos, Gummiarabicum, Xantham-Gum und Carboxyvinylpolymere (z. B. Carbopol^{®}-Typen) geeignet. Zusätzlich zu den Mischungen aus menthofuran und Mentholverbindungen können die Mund- und Zahnpflegemittel insbesondere oberflächenaktive Stoffe, bevorzugt anionische und nichtionische schaumstarke Tenside, wie die bereits oben genannten Stoffe, insbesondere aber Alkylethersulfat-Salze, Alkylpolyglucoside und deren Gemische.

Weitere übliche Zahnpastenzusätze sind:
- Konservierungsmittel und antimikrobielle Stoffe wie z. B. p- Hydroxybenzösäuremethyl-, -ethyl- oder -propylester, Natriumsorbat, Natriumbenzoat, Bromchlorophen, Phenylsalicylsäureester, Thymol und dergleichen;
- Antizahnsteinwirkstoffe, z. B. Organophosphate wie 1-Hydroxyethan- 1.1-diphosphonsäure, 1-Phosphonpropan-1,2,3-tricarbonsäure und andere, die z. B. aus US 3,488,419**,** DE 2224430 A1 und DE 2343196 A1 bekannt sind;
- andere karieshemmende Stoffe wie z. B. Natriumfluorid, Natriummonofluorphosphat, Zinnfluorid;
- Süßungsmittel, wie z. B. Saccharin-Natrium, Natrium-Cyclamat, Sucrose, Lactose, Maltose, Fructose oder Apartam^{®}, (L-Aspartyl- L-phenylalanin-methylester), Stivia-extrakte oder deren süßenden Bestandteile, insbesondere Ribeaudioside;
- Zusätzliche Aromen wie z. B. Eukalyptusöl, Anisöl, Fenchelöl, Kümmelöl, Methylacetat, Zimtaldehyd, Anethol, Vanillin, Thymol sowie Mischungen dieser und anderer natürlicher und synthetischer Aromen;
- Pigmente wie z. B. Titandioxid;
- Farbstoffe;
- Puffersubstanzen wie z. B. primäre, sekundäre oder tertiäre Alkaliphosphate oder Citronensäure/Natriumcitrat;
- wundheilende und entzündungshemmende Stoffe wie z. B. Allantoin, Harnstoff, Azulen, Kamillenwirkstoffe und Acetylsalicylsäurederivate.

Eine bevorzugte Ausführung der kosmetischen Zubereitungen sind Zahnpasten in Form einer wässrigen, pastösen Dispersion, enthaltend Poliermittel, Feuchthaltemittel, Viskositätsregulatoren und gegebenenfalls weitere übliche Komponenten, sowie die Mischung aus Menthofuran und Mentholverbindungen in Mengen von 0,5 bis 2 Gew.-% enthalten.

In Mundwässern ist eine Kombination mit wässrig-alkoholischen Lösungen verschiedener Grädigkeit von ätherischen Ölen, Emulgatoren, adstringierenden und tonisierenden Drogenauszügen, zahnsteinhemmenden, antibakteriellen Zusätzen und Geschmackskorrigentien ohne weiteres möglich. Eine weitere bevorzugte Ausführung der Erfindung ist ein Mundwasser in Form einer wässrigen oder wässrig-alkoholischen Lösung enthaltend die Mischung aus Menthofuran und Mentholverbindungen in Mengen von 0,5 bis 2 Gew.-%. In Mundwässern, die vor der Anwendung verdünnt werden, können mit, entsprechend dem vorgesehenen Verdünnungsverhältnis, höheren Konzentrationen ausreichende Effekte erzielt werden.

### Hydrotrope

Zur Verbesserung des Fließverhaltens können ferner Hydrotrope, wie beispielsweise Ethanol, Isopropylalkohol, oder Polyole eingesetzt werden; diese Stoffe entsprechen weitgehend den eingangs geschildern Trägern. Polyole, die hier in Betracht kommen, besitzen vorzugsweise 2 bis 15 Kohlenstoffatome und mindestens zwei Hydroxylgruppen. Die Polyole können noch weitere funktionelle Gruppen, insbesondere Aminogruppen, enthalten bzw. mit Stickstoff modifiziert sein. Typische Beispiele sind
- Glycerin;
- Alkylenglycole, wie beispielsweise Ethylenglycol, Diethylenglycol, Propylenglycol, Butylenglycol, Pentylenglycol, Caprylyl Glycol sowie Polyethylenglycole mit einem durchschnittlichen Molekulargewicht von 100 bis 1.000 Dalton;
- technische Oligoglyceringemische mit einem Eigenkondensationsgrad von 1,5 bis 10 wie etwa technische Diglyceringemische mit einem Diglyceringehalt von 40 bis 50 Gew.-%;
- Methyolverbindungen, wie insbesondere Trimethylolethan, Trimethylolpropan, Trimethylolbutan, Pentaerythrit und Dipentaerythrit;
- Niedrigalkylglucoside, insbesondere solche mit 1 bis 8 Kohlenstoffen im Alkylrest, wie beispielsweise Methyl- und Butylglucosid;
- Zuckeralkohole mit 5 bis 12 Kohlenstoffatomen, wie beispielsweise Sorbit oder Mannit,
- Zucker mit 5 bis 12 Kohlenstoffatomen, wie beispielsweise Glucose oder Saccharose;
- Aminozucker, wie beispielsweise Glucamin;
- Dialkoholamine, wie Diethanolamin oder 2-Amino-1,3-propandiol.

### Konservierungsmittel

Als Konservierungsmittel eignen sich beispielsweise Phenoxyethanol, Formaldehydlösung, Parabene, o-Cymen-5-ol, 4- Hydroxyacetophenon,Tropolon oder Sorbinsäure sowie die unter der Bezeichnung Surfacine^{®} bekannten Silberkomplexe und die in Anlage 6, Teil A und B der Kosmetikverordnung aufgeführten weiteren Stoffklassen.

### Parfümöle und Aromen

Als Parfümöle seien genannt Gemische aus natürlichen und synthetischen Riechstoffen. Natürliche Riechstoffe sind Extrakte von Blüten (Lilie, Lavendel, Rosen, Jasmin, Neroli, Ylang-Ylang), Stengeln und Blättern (Geranium, Patchouli, Petitgrain), Früchten (Anis, Koriander, Kümmel, Wacholder), Fruchtschalen (Bergamotte, Zitrone, Orangen), Wurzeln (Macis, Angelica, Sellerie, Kardamon, Costus, Iris, Calmus), Hölzern (Pinien-, Sandel-, Guajak-, Zedern-, Rosenholz), Kräutern und Gräsern (Estragon, Lemongras, Salbei, Thymian), Nadeln und Zweigen (Fichte, Tanne, Kiefer, Latschen), Harzen und Balsamen (Galbanum, Elemi, Benzoe, Myrrhe, Olibanum, Opoponax). Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethylmethylphenylglycinat, Allylcyclohexylpropionat, Styrallylpropionat und Benzylsalicylat. Zu den Ethern zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone, α-Isomethylionon und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labolanumöl und Lavandinöl. Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evernyl, Iraldein gamma, Phenylessigsäure, Geranylacetat, Benzylacetat, Rosenoxid, Romilllat, Irotyl und Floramat allein oder in Mischungen, eingesetzt.

Als Aromen kommen beispielsweise Pfefferminzöl, Krauseminzöl, Anisöl, Sternanisöl, Kümmelöl, Eukalyptusöl, Fenchelöl, Citronenöl, Wintergrünöl, Nelkenöl, Menthol und dergleichen in Frage.

### Farbstoffe

Als Farbstoffe können die für kosmetische Zwecke geeigneten und zugelassenen Substanzen verwendet werden, wie sie beispielsweise in der Publikation **"**Kosmetische Färbemittel" der Farbstoffkommission der Deutschen Forschungsgemeinschaft, Verlag Chemie, Weinheim, 1984, S.81-106 zusammengestellt sind. Beispiele sind Kochenillerot A (C.I. 16255), Patentblau V (C.I.42051), Indigotin (C.I.73015), Chlorophyllin (C.I.75810), Chinolingelb (C.I.47005), Titandioxid (C.I.77891), Indanthrenblau RS (C.I. 69800) und Krapplack (C.I.58000). Als Lumineszenzfarbstoff kann auch Luminol enthalten sein. Diese Farbstoffe werden üblicherweise in Konzentrationen von 0,001 bis 0,1 Gew.-%, bezogen auf die gesamte Mischung, eingesetzt.

### Zubereitungen

Bevorzugte erfindungsgemäße Zusammensetzungen sind ausgewählt aus der Gruppe der Produkte zur Behandlung, zum Schutz, zur Pflege und zur Reinigung der Haut und/oder der Haare oder als Schminkprodukt, entweder als Leave-on- oder Rinse-off-Produkte.

Die Formulierungen umfassen beispielsweise Dispersionen, Suspensionen, Cremes, Lotionen oder Milch, je nach Herstellungsmethode und Inhaltsstoffen, Gele (einschließlich Hydrogele, z.B. Hydrodispersionsgele, Oleogele), Sprays (z. B. Pumpsprays oder Sprays mit Treibmittel) Schäume oder Imprägnierlösungen für kosmetische Tücher, Seifen, Waschflüssigkeiten, Dusch- und Badezubereituneng, Badprodukte (Kapseln, Öl, Tabletten, Salze, Badesalze, Seifen usw.), Brausepräparatr, Hautpflegeprodukte, wie z.B. Emulsionen, Salben, Pasten, Gele (wie oben beschrieben), Öle, Balsame, Seren, Pulver (z. B. Gesichtspuder, Körperpulver), Masken, Stifte, Roll-on Stifte, Aerosole (schäumend, nicht schäumend oder nachschäumend), Deodorantien und / oder Antitranspirantien, Mundwasser und Mundspülungen, Insektenschutzmittel, Sonnenschutzmittel, After-Sun-Präparate, Rasiermittel, Aftershave-Balsame, Pre- und Aftershave-Lotionen, Enthaarungsmittel, Haarpflegeprodukte wie z Shampooe (einschließlich 2-in-1-Shampooe, Anti-Schuppen-Shampooe, Baby-Shampooe, Shampooe für trockene Kopfhaut, konzentriertes Shampooe), Conditioner, Haartoniken, Haarwasser, Haarspülungen, Stylingcremes, Pomaden, Dauerwell- und Festigungslotionen, Haarsprays, z.B. Stylinghilfen (zB Gel oder Wachs), Haarglättungsmittel (Entwirrungsmittel, Entspannungsmittel), Haarfärbemittel wie z.B. temporäre Haarfärbemittel, semipermanente Haarfärbemittel, permanente Haarfärbemittel, Haarconditioner, Haarschäume, Augenpflegeprodukte, Makeups, Make-up-Entferner oder Babyprodukte.

Besonders bevorzugt liegen die erfindungsgemäßen Formulierungen in Form einer Emulsion vor, insbesondere in Form einer W/O-, O/W-, W/O/W-, O/W/O-Emulsion, PIT-Emulsion, B. eine Pickering-Emulsion, eine Emulsion mit einem geringen Ölgehalt, eine Mikro- oder Nanoemulsion, ein Gel (einschließlich Hydrogel, Hydrodispersionsgel, Oleogel) oder einer Lösung vor.

Der Gesamtanteil der Hilfs- und Zusatzstoffe kann 1 bis 50, vorzugsweise 5 bis 40 Gew.-% - bezogen auf die Mittel - betragen. Die Herstellung der Mittel kann durch übliche Kalt - oder Heißprozesse erfolgen; vorzugsweise arbeitet man nach der Phaseninversionstemperatur-Methode.

### ORALE ZUBEREITUNGEN

Ein weiterer Gegenstand der Erfindung betrifft orale Zubereitungen, die entweder einen oder mehrere der erfindungsgemäßen Kühlstoffe oder eine Aromazubereitung enthalten, welche einen Gehalt an diesen Kühlstoffen aufweist. Dieser Gehalt beträgt vorzugsweise etwa 0,1 bis etwa Gew.-% und insbesondere etwa 0,5 bis 2 Gew.-% - bezogen auf die fertige Zubereitung.

Unter oralen Zubereitungen sind im Sinne der vorliegenden Erfindung zum einen Mund- und Zahnpflegemittel einschließlich Kaugummis und zum anderen Nahrungsmittelzubereitungen zu verstehen.

### Mund- und Zahnpflegemittel

Erfindungsgemäße oral konsumierbare süß schmeckende Produkte können auch der Mund- und Zahnreinigung und-pflege dienen. Beispiele hierfür sind Zahnpasten, Zahngele, Zahnpulver, Mundwässer und dergleichen. Unter Zahnpasten oder Zahncremes werden im allgemeinen gelförmige oder pastöse Zubereitungen aus Wasser, Verdickungsmitteln, Feuchthaltemitteln, Schleif- oder Putzkörpern, Tensiden, Süßmitteln, Aromastoffen, deodorierenden Wirkstoffen sowie Wirkstoffen gegen Mund- und Zahnerkrankungen verstanden. In die erfindungsgemäßen Zahnpasten können alle üblichen Putzkörper, wie z. B. Kreide, Dicalciumphosphat, unlösliches Natriummetaphosphat, Aluminiumsilikate, Calciumpyrophosphat, feinteilige Kunstharze, Kieselsäuren, Aluminiumoxid und Aluminiumoxidtrihydrat eingesetzt werden.

Bevorzugt geeignete Putzkörper für die erfindungsgemäßen Zahnpasten sind vor allem feinteilige Xerogelkieselsäuren, Hydrogelkieselsäuren, Fällungskieselsäuren, Aluminiumoxid-trihydrat und feinteiliges alpha -Aluminiumoxid oder Mischungen dieser Putzkörper in Mengen von 15 bis 40 Gew.-% der Zahnpasta. Als Feuchthaltemittel kommen vorwiegend niedermolekulare Polyethylenglykole, Glycerin, Sorbit oder Mischungen dieser Produkte in Mengen bis zu 50 Gew.-% in Frage. Unter den bekannten Verdickungsmitteln sind die verdickenden, feinteiligen Gelkieselsäuren und Hydrokolloide, wie z. B. Carboxymethylcellulose, Hydroxyethylcellulose, Hydroxypropylguar, Hydroxyethylstärke, Polyvinylpyrrolidon, hochmolekulares Polyethylenglykol, Pflanzengummen wie Traganth, Agar-Agar, Carragheenmoos, Gummi arabicum, Xantham-Gum und Carboxyvinylpolymere (z. B. Carbopol^{®}-Typen) geeignet. Zusätzlich zu den Mischungen aus menthofuran und Mentholverbindungen können die Mund- und Zahnpflegemittel insbesondere oberflächenaktive Stoffe, bevorzugt anionische und nichtionische schaumstarke Tenside, wie die bereits oben genannten Stoffe, insbesondere aber Alkylethersulfat-Salze, Alkylpolyglucoside und deren Gemische.

Weitere übliche Zahnpastenzusätze sind:
- Konservierungsmittel und antimikrobielle Stoffe wie z. B. p- Hydroxybenzösäuremethyl-, -ethyl- oder -propylester, Natriumsorbat, Natriumbenzoat, Bromchlorophen, Phenylsalicylsäureester, Thymol und dergleichen;
- Antizahnsteinwirkstoffe, z. B. Organophosphate wie 1-Hydroxyethan- 1.1-diphosphonsäure, 1-Phosphonpropan-1,2,3-tricarbonsäure und andere, die z. B. aus US 3,488,419**,** DE 2224430 A1 und DE 2343196 A1 bekannt sind;
- andere karieshemmende Stoffe wie z. B. Natriumfluorid, Natriummonofluorphosphat, Zinnfluorid;
- Süssungsmittel, wie z. B. Saccharin-Natrium, Natrium-Cyclamat, Sucrose, Lactose, Maltose, Fructose oder Apartam^{®}, (L-Aspartyl- L-phenylalanin-methylester), Stivia-extrakte oder deren süßenden Bestandteile, insbesondere Ribeaudioside;
- Zusätzliche Aromen wie z. B. Eukalyptusöl, Anisöl, Fenchelöl, Kümmelöl, Methylacetat, Zimtaldehyd, Anethol, Vanillin, Thymol sowie Mischungen dieser und anderer natürlicher und synthetischer Aromen;
- Pigmente wie z. B. Titandioxid;
- Farbstoffe;
- Puffersubstanzen wie z. B. primäre, sekundäre oder tertiäre Alkaliphosphate oder Citronensäure/Natriumcitrat;

wundheilende und entzündungshemmende Stoffe wie z. B. Allantoin, Harnstoff, Azulen, Kamillenwirkstoffe und Acetylsalicylsäurederivate.

Eine bevorzugte Ausführung der kosmetischen Zubereitungen sind Zahnpasten in Form einer wässrigen, pastösen Dispersion, enthaltend Poliermittel, Feuchthaltemittel, Viskositätsregulatoren und gegebenenfalls weitere übliche Komponenten, sowie die Mischung aus Menthofuran und Mentholverbindungen in Mengen von 0,5 bis 2 Gew.-% enthalten.

In Mundwässern ist eine Kombination mit wässrig-alkoholischen Lösungen verschiedener Grädigkeit von ätherischen Ölen, Emulgatoren, adstringierenden und tonisierenden Drogenauszügen, zahnsteinhemmenden, antibakteriellen Zusätzen und Geschmackskorrigentien ohne weiteres möglich. Eine weitere bevorzugte Ausführung der Erfindung ist ein Mundwasser in Form einer wässrigen oder wässrig-alkoholischen Lösung enthaltend die Mischung aus Menthofuran und Mentholverbindungen in Mengen von 0,5 bis 2 Gew.-%. In Mundwässern, die vor der Anwendung verdünnt werden, können mit, entsprechend dem vorgesehenen Verdünnungsverhältnis, höheren Konzentrationen ausreichende Effekte erzielt werden.

Zur Verbesserung des Fließverhaltens können ferner Hydrotrope, wie beispielsweise Ethanol, Isopropylalkohol, oder Polyole eingesetzt werden; diese Stoffe entsprechen weitgehend den eingangs geschildern Trägern. Polyole, die hier in Betracht kommen, besitzen vorzugsweise 2 bis 15 Kohlenstoffatome und mindestens zwei Hydroxylgruppen. Die Polyole können noch weitere funktionelle Gruppen, insbesondere Aminogruppen, enthalten bzw. mit Stickstoff modifiziert sein. Typische Beispiele sind
- Glycerin;
- Alkylenglycole, wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton;
- technische Oligoglyceringemische mit einem Eigenkondensationsgrad von 1,5 bis 10 wie etwa technische Diglyceringemische mit einem Diglyceringehalt von 40 bis 50 Gew.-%;
- Methyolverbindungen, wie insbesondere Trimethylolethan, Trimethylolpropan, Trimethylolbutan, Pentaerythrit und Dipentaerythrit;
- Niedrigalkylglucoside, insbesondere solche mit 1 bis 8 Kohlenstoffen im Alkylrest, wie beispielsweise Methyl- und Butylglucosid;
- Zuckeralkohole mit 5 bis 12 Kohlenstoffatomen, wie beispielsweise Sorbit oder Mannit,
- Zucker mit 5 bis 12 Kohlenstoffatomen, wie beispielsweise Glucose oder Saccharose;
- Aminozucker, wie beispielsweise Glucamin;
- Dialkoholamine, wie Diethanolamin oder 2-Amino-1,3-propandiol.

Als Konservierungsmittel eignen sich beispielsweise Phenoxyethanol, Formaldehydlösung, Parabene, Pentandiol oder Sorbinsäure sowie die unter der Bezeichnung Surfacine^{®} bekannten Silberkomplexe und die in Anlage 6, Teil A und B der Kosmetikverordnung aufgeführten weiteren Stoffklassen.

Als Parfümöle seien genannt Gemische aus natürlichen und synthetischen Riechstoffen. Natürliche Riechstoffe sind Extrakte von Blüten (Lilie, Lavendel, Rosen, Jasmin, Neroli, Ylang-Ylang), Stengeln und Blättern (Geranium, Patchouli, Petitgrain), Früchten (Anis, Koriander, Kümmel, Wacholder), Fruchtschalen (Bergamotte, Zitrone, Orangen), Wurzeln (Macis, Angelica, Sellerie, Kardamon, Costus, Iris, Calmus), Hölzern (Pinien-, Sandel-, Guajak-, Zedern-, Rosenholz), Kräutern und Gräsern (Estragon, Lemongras, Salbei, Thymian), Nadeln und Zweigen (Fichte, Tanne, Kiefer, Latschen), Harzen und Balsamen (Galbanum, Elemi, Benzoe, Myrrhe, Olibanum, Opoponax). Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethylmethylphenylglycinat, Allylcyclohexylpropionat, Styrallylpropionat und Benzylsalicylat. Zu den Ethern zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone, α-Isomethylionon und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labolanumöl und Lavandinöl. Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evernyl, Iraldein gamma, Phenylessigsäure, Geranylacetat, Benzylacetat, Rosenoxid, Romilllat, Irotyl und Floramat allein oder in Mischungen, eingesetzt.

Als Aromen, die die Komponente (b2) bilden, kommen insbesondere Pfefferminzöl, Krauseminzöl, Anisöl, Sternanisöl, Kümmelöl, Eukalyptusöl, Fenchelöl, Citronenöl, Wintergrünöl, Nelkenöl, Menthol und dergleichen in Frage.

### Kaugummis

Kaugummizusammensetzungen enthalten typischerweise eine wasserunlösliche und eine wasserlösliche Komponente.

Die wasserunlösliche Basis, die auch als "Gummibasis" bezeichnet wird. umfasst üblicherweise natürliche oder synthetische Elastomere, Harze, Fette und Öle, Weichmacher, Füllstoffe, Farbstoffe sowie gegebenenfalls Wachse. Der Anteil der Basis an der Gesamtzusammensetzung macht üblicherweise 5 bis 95, vorzugsweise 10 bis 50 und insbesondere 20 bis 35 Gew.-% aus. In einer typischen Ausgestaltungsform der Erfindung setzt sich die Basis aus 20 bis 60 Gew.-% synthetischen Elastomeren, 0 bis 30 Gew.-% natürlichen Elastomeren, 5 bis 55 Gew.-% Weichmachern, 4 bis 35 Gew.-% Füllstoffe und in untergeordneten Mengen Zusatzstoffe wie Farbstoffe, Antioxidantien und dergleichen zusammen, mit der Maßgabe, dass sie allenfalls in geringen Mengen wasserlöslich sind.

Als geeignete synthetische Elastomere kommen beispielsweise Polyisobutylene mit durchschnittlichen Molekulargewichten (nach GPC) von 10.000 bis 100.000 und vorzugsweise 50.000 bis 80.000, Isobutylen-Isopren-Copolymere ("Butyl Elastomere"), Styrol-Butadien-Copolymere (Styrol:Butadien-Verhältnis z.B. 1: 3 bis 3: 1), Polyvinylacetate mit durchschnittlichen Molekulargewichten (nach GPC) von 2.000 bis 90.000 und vorzugsweise 10.000 bis 65.000, Polyisoprene, Polyethylen, Vinylacetat-Vinyllaurat-Copolymere und deren Gemische. Beispiele für geeignete natürliche Elastomere sind Kautschuks wie etwa geräucherter oder flüssiger Latex oder Guayule sowie natürliche Gummistoffe wie Jelutong, Lechi caspi, Perillo, Sorva, Massaranduba balata, Massaranduba chocolate, Nispero, Rosindinba, Chicle, Gutta hang 1kang sowie deren Gemische. Die Auswahl der synthetischen und natürlichen Elastomere und deren Mischungsverhältnisse richtet sich im Wesentlichen danach, ob mit den Kaugummis Blasen erzeugt werden sollen ("bubble gums") oder nicht. Vorzugsweise werden Elastomergemische eingesetzt, die Jelutong, Chicle, Sorva und Massaranduba enthalten.

In den meisten Fällen erweisen sich die Elastomere in der Verarbeitung als zu hart oder zu wenig verformbar, so dass es sich als vorteilhaft erwiesen hat, spezielle Weichmacher mitzuverwenden, die natürlich insbesondere auch alle Anforderungen an die Zulassung als Nahrungsmittelzusatzstoffe erfüllen müssen. In dieser Hinsicht kommen vor allem Ester von Harzsäuren in Betracht, beispielsweise Ester von niederen aliphatischen Alkoholen oder Polyolen mit ganz oder teilweise gehärteten, monomeren oder oligomeren Harzsäuren. Insbesondere werden für diesen Zweck die Methyl-, Glycerin-, oder Pentareythritester sowie deren Gemische eingesetzt. Alternativ kommen auch Terpenharze in Betracht, die sich von alpha-Pinen, beta-Pinen, delta-Limonen oder deren Gemischen ableiten können.

Als Füllstoffe oder Texturiermittel kommen Magnesium- oder Calciumcarbonat, gemahlener Bimsstein, Silicate, speziell Magnesium- oder Aluminiumsilicate, Tone, Aluminiumoxide. Talkum, Titandioxid, Mono-, Di- und Tricalciumphosphat sowie Cellulosepolymere.

Geeignete Emulgatoren sind Talg, gehärteter Talg, gehärtete oder teilweise gehärtete pflanzliche Öle, Kakaobutter, Partialglyceride, Lecithin, Triacetin und gesättigte oder ungesättigte Fettsäuren mit 6 bis 22 und vorzugsweise 12 bis 18 Kohlenstoffatomen sowie deren Gemische.

Als Farbstoffe und Weißungsmittel kommen beispielsweise die für die Färbung von Lebensmitteln zugelassenen FD und C-Typen, Pflanzen- und Fruchtextrakte sowie Titandioxid in Frage.

Die Basismassen können Wachse enthalten oder wachsfrei sein; Beispiele für wachsfreie Zusammensetzungen finden sich unter anderem in der Patentschrift US 5,286,500**,** auf deren Inhalt hiermit ausdrücklich Bezug genommen wird.

Zusätzlich zu der wasserunlöslichen Gummibasis enthalten Kaugummizubereitungen regelmäßig einen wasserlösliche Anteil, der beispielsweise von Softener, Süßstoffen, Füllstoffen, Geschmacksstoffen, Geschmacksverstärkern, Emulgatoren, Farbstoffen, Säuerungsmitteln, Antioxidantien und dergleichen gebildet werden, hier mit der Maßgabe, dass die Bestandteile eine wenigstens hinreichende Wasserlöslichkeit besitzen. In Abhängigkeit der Wasserlöslichkeit der speziellen Vertreter können demnach einzelne Bestandteile sowohl der wasserunlöslichen wie auch der wasserlöslichen Phase angehören. Es ist jedoch auch möglich, Kombinationen beispielsweise eines wasserlöslichen und eines wasserunlöslichen Emulgators einzusetzen, wobei sich die einzelnen Vertreter, dann in unterschiedlichen Phasen befinden. Üblicherweise macht der wasserunlösliche Anteil 5 bis 95 und vorzugsweise 20 bis 80 Gew.-% der Zubereitung aus.

Wasserlösliche Softener oder Plastifiziermittel werden den Kaugummizusammensetzungen hinzugegeben um die Kaubarkeit und das Kaugefühl zu verbessern und sind in den Mischungen typischerweise in Mengen von 0,5 bis 15 Gew.-% zugegen. Typische Beispiele sind Glycerin, Lecithin sowie wässrige Lösungen von Sorbitol, gehärteten Stärkehydrolysaten oder Kornsirup.

Als Süßstoffe kommen sowohl zuckerhaltige wie zuckerfreie Verbindungen in Frage, die in Mengen von 5 bis 95, vorzugsweise 20 bis 80 und insbesondere 30 bis 60 Gew.-% bezogen auf die Kaugummizusammensetzung eingesetzt werden. Typische Saccharid-Süssstoffe sind Sucrose, Dextrose, Maltose, Dextrin, getrockneter Invertzucker, Fructose, Levulose, Galactose, Kornsirup sowie deren Gemische. Als Zuckerersatzstoffe kommen Sorbitol, Mannitol, Xylitol, gehärtete Stärkehydrolysate, Maltitol und deren Gemische in Frage. Weiterhin kommen als Zusatzstoffe auch sogenannte HIAS ("High Intensity Articifical Sweeteners") in Betracht, wie beispielsweise Sucralose, Aspartam, Acesulfamsalze, Alitam, Saccharin und Saccharinsalze, Cyclamsäure und deren Salze, Glycyrrhizine, Dihydrochalcone, Thaumatin, Monellin und dergleichen alleine oder in Abmischungen. Besonders wirksam sind auch die hydrophoben HIAS, die Gegenstand der internationalen Patentanmeldung WO 2002 091849 A1 (Wrigleys) sowie Stevia Extrakte und deren aktiven Bestandteile, insbesondere Ribeaudiosid A sind. Die Einsatzmenge dieser Stoffe hängt in erster Linie von ihrem Leistungsvermögen ab und liegt typischerweise im Bereich von 0,02 bis 8 Gew.-%.

Insbesondere für die Herstellung kalorienarmer Kaugummis eignen sich Füllstoffe wie beispielsweise Polydextrose, Raftilose, Rafitilin, Fructooligosaccharide (NutraFlora), Palatinoseoligosaaccharide, Guar Gum Hydrolysate (Sun Fiber) sowie Dextrine.

Die Auswahl an weiteren Geschmacksstoffen ist praktisch unbegrenzt und für das Wesen der Erfindung unkritisch. Üblicherweise liegt der Gesamtanteil aller Geschmacksstoffe bei 0,1 bis 15 und vorzugsweise 0,2 bis 5 gew.-% bezogen auf die Kaugummizusammensetzung. Geeignete weitere Geschmacksstoffe stellen beispielsweise essentielle Öle, synthetische Aromen und dergleichen dar, wie etwa Anisöl, Sternanisöl, Kümmelöl, Eukalyptusöl, Fenchelöl, Citronenöl, Wintergrünöl, Nelkenöl, und dergleichen, wie sie auch beispielsweise in Mund- und Zahnpflegemittel Verwendung finden.
Die Kaugummis können des weiteren Hilfs- und Zusatzstoffe enthalten, die beispielsweise für die Zahnpflege, speziell zur Bekämpfung von Plaque und Gingivitis geeignet sind, wie z.B. Chlorhexidin, CPC oder Trichlosan. Weiter können pH-Regulatoren (z.B. Puffer oder Harnstoff), Wirkstoffe gegen Karies (z.B. Phosphate oder Fluoride), biogene Wirkstoffe (Antikörper, Enzyme, Koffein, Pflanzenextrakte) enthalten sein, solange diese Stoffe für Nahrungsmittel zugelassen sind und nicht in unerwünschter Weise miteinander in Wechselwirkung treten.

### NAHRUNGSMITTEL

Vorzugsweise handelt hierbei um Backwaren, beispielsweise Brot, Trockenkekse, Kuchen, sonstiges Gebäck, Süßwaren (beispielsweise Schokoladen, Schokoladenriegelprodukte, sonstige Riegelprodukte, Fruchtgummi, Hart- und Weichkaramellen, Kaugummi), alkoholische oder nicht-alkoholische Getränke (beispielsweise Kaffee, Tee, Eistee, Wein, weinhaltige Getränke, Bier, bierhaltige Getränke, Liköre, Schnäpse, Weinbrände, (carbonisierte) fruchthaltige Limonaden, (carbonisierte) isotonische Getränke, (carbonisierte) Erfrischungsgetränke, Nektare, Schorlen, Obst- und Gemüsesäfte, Frucht- oder Gemüsesaftzubereitungen, Instantgetränke (beispielsweise Instant-Kakao-Getränke, Instant-Tee-Getränke, Instant-Kaffeegetränke, Instant-Fruchtgetränke), Fleischprodukte (beispielsweise Schinken, Frischwurst- oder Rohwurstzubereitungen, gewürzte oder marinierte Frisch- oder Pökelfleischprodukte), Eier oder Eiprodukte (Trockenei, Eiweiß, Eigelb), Getreideprodukte (beispielsweise Frühstückscerealien, Müsliriegel, vorgegarte Fertigreis-Produkte), Milchprodukte (beispielsweise Milchgetränke, Buttermilchgetränke, Milcheis, Joghurt, Kefir, Frischkäse, Weichkäse, Hartkäse, Trockenmilchpulver, Molke, Molkegetränke, Butter, Buttermilch, teilweise oder ganz hydrolisierte Milchprotein-haltige Produkte), Produkte aus Sojaprotein oder anderen Sojabohnen-Fraktionen (beispielsweise Sojamilch und daraus gefertigte Produkte, Fruchtgetränke mit Sojaprotein, Sojalecithin-haltige Zubereitungen, fermentierte Produkte wie Tofu oder Tempe oder daraus gefertigte Produkte), Produkte aus anderen pflanzlichen Proteinquellen, beispielsweise Haferprotein-Getränke, Fruchtzubereitungen (beispielsweise Konfitüren, Fruchteis, Fruchtsoßen, Fruchtfüllungen), Gemüsezubereitungen (beispielsweise Ketchup, Soßen, Trockengemüse, Tiefkühlgemüse, vorgegarte Gemüse, eingekochte Gemüse), Knabberartikel (beispielsweise gebackene oder frittierte Kartoffelchips oder Kartoffelteigprodukte, Extrudate auf Mais- oder Erdnussbasis), Produkte auf Fett- und Ölbasis oder Emulsionen derselben (beispielsweise Mayonnaise, Remoulade, Dressings), sonstige Fertiggerichte und Suppen (beispielsweise Trockensuppen, Instant-Suppen, vorgegarte Suppen), Gewürze, Würzmischungen sowie insbesondere Aufstreuwürzungen (englisch: Seasonings), die beispielsweise im Snackbereich Anwendung finden. Die Nahrungsmittel können neben den bereits oben genannten Aromen und Süßstoffen weitere Hilfs- und Zusatzstoffe enthalten, wie z.B.:

### Starterkulturen und Probiotische Mikroorganismen

Probiotische Mikroorganismen, auch als "Probiotics" bezeichnet, die die Gruppe (N) bilden, stellen lebende Mikroorganismen dar, die für den Wirt nützliche Eigenschaften besitzen. Gemäß der Definition der FAO/WHO handelt es sich um "lebende Mikroorganismen, die bei angemessener Dosierung dem Wirt einen Gesundheitsvorteil vermitteln". Milchsäurebakterien (LAB) und Bifidobakterien stellen die bekanntesten Probiotics dar; es können aber auch verschiedene Hefen und Bazillen Verwendung finden. Probiotics werden üblicherweise als Bestandteil von fermentierten Nahrungsmitteln aufgenommen, denen man spezielle Lebendkulturen zugesetzt hat, wie z.B. Joghurt, Sojajoghurt oder andere probiotische Nahrungsmittel. Darüber hinaus sind auch Tabletten, kapseln, Pulver und Sachets erhältlich, die die Mikroorganismen in gefriergetrockneter Form enthalten. Tabelle B gibt einen Überblick über handelsübliche Probiotics und den zugehörigen Auslobungen zur Gesundheit, die im Sinne der vorliegenden Erfindung als Komponente (b1) eingesetzt werden können.

**Tabelle A:**

| Probiotische Stoffe | | | |
|---|---|---|---|
| **Stamm** | **Bezeichnung** | **Hersteller** | **Auslobung** |
| *Bacillus coagulans* GBI-30,6086 | GanedenBC | Ganeden Biotech | Steigert die Immunantwort bei viraler Infektion |
| *Bifidobacterium animalis* subsp. *lactis* BB-12 | Probio-Tec Bifidobacterium BB-12 | Chr. Hansen | Klinische Studien am Menschen haben gezeigt, dass BB-12 alleine oder in Kombination das gastrotestinale System positiv beeinflusst. |
| *Bifidobacterium infantis* 35624 | Align | Procter & Gamble | In einer vorläufigen Studie wurde gezeigt, dass das Bakterium abdominale Schmerzen verringern kann. |
| *Lactobacillus acidophi-lus* NCFM | | Danisco | Aus einer Studie geht hervor, dass die Nebeneffekte von antibiotischen Behandlungen verringert werden |
| *Lactobacillus paraca*sei St11 (orNCC2461) | | | |
| *Lactobacillus johnsonii* La1 (= Lactobacillus LC1, *Lactobacillus johnsonii* NCC533) | | Nestlé | Verringert Gastritisbeschwerden und vemindert Entzündungen |
| *Lactobacillus plantarum* 299v | Good Belly/ ProViva/ProbiMage | Probi | Könnte IBS Symptome verbessern; jedoch noch mehr Studien erforderlich. |
| *Lactobacillus reute*ri American Type Culture CollectionlATTC 55730 *(Lactobacillus reuteri* SD2112) | | BioGaia | Erste Anzeichen für eine Wirksamkeit gegen Gangivitis, Fieber bei Kindern und Verminderung der Krankheitstage bei Erwachsenen. |
| *Lactobacillus reuteri* Protectis (DSM 17938, daughter strain of ATCC 55730) | | | |
| *Lactobacillus reuteri* Protectis (DSM 17938, daughter strain of ATCC 55730) | | | |
| *Saccharomyces boulardii* | DiarSafe and others | Wren Laboratories | Beschränkter Nachweis bei der Behandlung von akuten Durchfallerkrankungen. |
| *Lactobacillus rhamnosus* GR-1 & *Lactobacillus reuteri* RC-14 | Bion Flore Intime/ Jarrow Fem-Dophilus | Chr. Hansen | In einer Studie Nachweis der Wirksamkeit gegen Vaginitis. |
| *Lactobacillus acidophi-lus* NCFM *& Bifidobacterium bifidum* BB-12 | Florajen3 | American Lifeline, Inc | Erste Hinweise auf Wirksamkeit gegen CDAD |
| *Lactobacillus acidophi-lus* CL 1285 & *Lactobacillus casei* LBC80R | Bio-K+ CL1285 | Bio-K+ International | Hinweise auf Verbesserung bei der Verdauung, speziell im Hinblick auf Lactoseintoleranz. |
| *Lactobacillus plantarum* HEAL 9 & *Lactobacillus paracasei* 8700:2 | Bravo Friscus/ ProbiFrisk | Probi | Derzeit laufen Untersuchungen zur Wirksamkeit gegen Erkältungs-erkrankungen. |

Im Folgenden werden zwei weiteren Formen von Milchsäurebakterien genannt, die ebenfalls als Starterkulturen bzw. als Probiotics eingesetzt werden können:
- *Lactobacillus bulgaricus;*
- *Streptococcus thermophilus;*
- *Streptococcus thermophilus,*
- *Leuconostoc species,*
- *Lactococcus lactis subsp. lactis biovar diacetylactis,*
- *Lactococcus lactis subsp. lactis,*
- *Lactococcus lactis subsp. cremoris,* und
- *Bifido bakterium lactis B12,*

### Prebiotische Stoffe

In einer weiteren Ausgestaltung der Erfindung können die Zubereitungen des Weiteren Prebiotische Stoffe ("Prebiotics") enthalten, die die Gruppe H bilden. Prebiotics werden als unverdauliche Nahrungsbestandteile definiert, deren Verabreichung das Wachstum oder die Aktivität einer Reihe nützlicher Bakterien im Dickdarm stimuliert. Die Zugabe von prebiotischen Verbindungen verbessert die Stabilität der Anthocyanine gegenüber Abbauprozessen im Darmtrakt. Im Folgenden werden verschiedene Stoffe, insbesondere Kohlenhydrate, die als Prebiotics im Sinne der Erfindung besonders bevorzugt sind.

**Fructooligosaccharide.** Fructooligosaccharide oder abgekürzt FOS umfassen insbesondere kurzkettige Vertreter mit 3 bis 5 Kohlenstoffatomen, wie beispielsweise D-Fructose und D-Glucose. FOS, auch als Neozucker bezeichnet, werden kommerziell auf Basis von Saccharose und dem aus Pilzen gewonnenen Enzym Fructosyltransferase hergestellt. FOS unterstützen insbesondere das Wachstum von Bifidobakterien im Darm und werden vor allem in den USA zusammen mit probiotischen Bakterien in verschiedenen funktionalisierten Lebensmitteln vermarktet.

**Inuline.** Inuline zählen zu einer Gruppe von natürlich vorkommenden Fructose enthaltenden Oligosachhariden. Sie gehören zu einer Klasse von Kohlenhydraten, die als Fructane bezeichnet werden. Ihre Gewinnung erfolgt aus den Wurzeln der Chicoree-Pflanze (Cichorium intybus) oder so genannten Jerusalem-Artischocken. Inuline bestehen überwiegend aus Fructoseeinheiten und weisen typisch eine Glucoseeinheit als Endgruppe auf. Die Fructoseeinheiten sind dabei miteinander über eine beta-(2-1)glykosidische Bindung verknüpft. Der mittlere Polymerisationsgrad von Inulinen, die als Prebiotics im nahrungsmittelbereich Anwendung finden, liegt bei 10 bis 12. Inuline stimulieren ebenfalls das Wachstum von Bifidobakterien im Dickdarm.

**Isomaltooligosaccharide.** Bei dieser Gruppe handelt es sich um eine Mischung von alpha-D-verknüpften Glucoseoligomeren, einschließlich Isomaltose, Panose, isomaltotetraose, Isomaltopentaose, Nigerose, Kojibiose, Isopanose und höherer verzweigter Oligosaccharide. Isomaltooligosaccharide werden über verschiedene enzymatische Wege hergestellt. Sie stimulieren ebenfalls das Wachstum von Bifidobakterien und Lactobacillen im Dickdarm. Isoma-Itooligosaccharide werden speziell in Japan als Nahrungsmittelzusatzstoffe in funktionalisierten Lebensmitteln eingesetzt. Inzwischen finden sie auch in den USA Verbreitung.

**Lactilol.** Lactilol ist das Disaccharid der Lactulose. Seine medizinische Anwendung findet gegen Verstopfung und bei häpatischer Enzephalopathie. In Japan wird Lactilol als prebiotic eingesetzt. Es widersteht dem Abbau im oberen Verdauungstrakt, wird aber durch verschiedene Darmbakterien fermentiert, was zu einem Anstieg der Biomasse an Bifidobakterien und Lactobacillen im Darm führt. Lactilol ist auch unter der chemischen Bezeichnung 4-0-(beta-D-galactopyranosyl)-D-glucitol bekannt. Der medizinsche Anwendungsbereich von Lactilol in den USA ist wegen fehlender Studien beschränkt; in Europa wird es vorzugsweise als Süßstoff eingesetzt.

**Lactosucrose.** Lactosucrose ist ein Trisaccharid, das sich aus D-Galactose, D-Glucose und D-Fructose aufbaut. Lactosucrose wird durch enzymatischen Transfer des Galactosylrestes in der Lactose auf die Sucrose hergestellt. Es wird weder im Magen noch im oberen Teil des Darmtraktes abgebaut und wird ausschließlich von Bifidobakterien zu Wachstum konsumiert. Unter physiologischen Gesichtspunkten wirkt Lactosucrose als Stimulator für das Wachstum der Darmflora. Lactosucrose ist ebenfalls bekannt als 4G-beta-D-galactosucrose. Es ist in Japan als Nahrungsmittelzusatzstoff und als Bestandteil von funktionalisierten lebensmittel weit verbreitet, insbesondere auch als Zusatzstoff für Joghurts. Lactosucrose wird derzeit auch in den USA für einen ähnlichen Anwendungszweck getestet.

**Lactulose.** Lactulose ist ein halbsynthetisches Disaccharid aus D-Lactose und D-Fructose. Die Zucker sind über eine beta-glykosidische Bindung verknüpft, was sie resistent gegen Hydrolyse durch Verdauungsenzyme macht. Stattdessen wird Lactulose durch eine beschränkte Anzahl von Darmbakterien fermentiert, was zu einem Wachstum insbesondere von Lactobacillen und Bifidobakterien führt. Lactulose stellt in den USA ein verschreibungspflichtiges Medikament gegen Verstopfung und hepatische Enzephalopathie dar. In Japan hingegen wird es als Nahrungsmittelzusatzstoff und Bestandteil von funktionalisierten Lebensmitteln frei verkauft.

**Pyrodextrine.** Pyrodextrine umfassen eine Mischung von glucoseenthaltenden Oligosacchariden, die bei der Hydrolyse von Stärke gebildet werden. Pyrodextrine fördern die Proliferation von Bifidobakterien im Dickdarm. Auch sie werden im oberen Darmbereich nicht abgebaut.

**Sojaoligosaccharide.** Bei dieser Gruppe handelt es sich um Oligosaccharide, die im Wesentlichen nur in Sojabohnen und darüber noch in anderen Bohnen sowie Erbsen zu finden sind. Die beiden maßgeblichen Vertreter sind das Trisaccharid Raffinose und das Tetrasaccharid Stachyose. Raffinose setzt sich aus jeweils einem Molekül D-Galactose, D-Glucose und D-Fructose zusammen. Stachyose besteht aus zwei Molekülen D-Galactose sowie jeweils einem Molekül D-Glucose und D-Fructose. Sojaoligosacccharide stimulieren das Wachstum von Bifidobakterien im Dickdarm und werden in Japan bereits als Nahrungsmittelzusatzstoffe sowie in funktionalisierten Lebensmitteln eingesetzt. In den USA werden sie für diese Anwendung derzeit getestet.

**Transgalactooligosaccharide.** Transgalactooligosaccharide (TOS) stellen Mischungen von Oligosacchariden auf Basis D-Glucose und D-Galactose dar. TOS werden ausgehend von D-Lactose mit Hilfe des Enzyms Betaglucosidase aus Aspergillus oryzae hergestellt. Wie viele andere Prebiotics sind auch TOS im Dünndarm stabil und stimulieren das Wachstum von Bifidobakterien im Dickdarm. TOS werden sowohl bereits in Europa als auch in Japan als Nahrungsmittelzusatzstoffe vermarktet.

**Xylooligosaccharide.** Xylooligosaccharide enthalten beta-1,4-verknüpfte Xyloseeinheiten. Der Polymerisationsgrad der Xylooligosaccharide liegt zwischen 2 und 4. Sie werden durch enzymatische Hydrolyse des Polysaccharids Xylan erhalten. Sie werden bereits in Japan als Nahrungsmittelzusatzstoffe vermarktet, in den USA befinden sie sich noch in der Testphase.

**Biopolymere.** Geeignete Biopolymere, die ebenfalls als Prebiotics in Betracht kommen, wie beispielsweise Beta-Glucane, zeichnen sich dadurch aus, dass sie auf pflanzlicher Basis hergestellt werden, beispielsweise kommen als Rohstoffquellen Cerealien wie Hafer und Gerste, aber auch Pilze, Hefen und Bakterien in Frage. Außerdem geeignet sind mikrobiell hergestellte Zellwandsuspensionen oder ganze Zellen mit hohem Beta-Glucan Gehalt. Restliche Anteile an Monomeren weisen 1-3 und 1-4 oder 1-3 und 1-6 Verknüpfungen auf, wobei der Gehalt stark variieren kann. Vorzugsweise werden Beta-Glucane auf Basis von Hefen, insbesondere Saccharomyces, speziell Saccharomyces cerevisiae, erhalten. Andere geeignete Biopolymere sind Chitin und Chitinderivate, insbesondere Oligoglucosamin und Chitosan, das ein typisches Hydrokolloid darstellt.

**Galactooligosaccharide (GOS).** Galacto-Oligosaccharide werden durch die enzymatische Umwandlung von Lactose, einer Komponente von Rindermilch, erzeugt. GOS umfassen im Allgemeinen eine Kette von Galactose-Einheiten, die durch aufeinanderfolgende Transgalactosylierung-Reaktionen gebildet werden, und die eine terminale Glucoseeinheit aufweisen. Terminale Glucoseeinheiten werden zumeist durch eine frühzeitige Hydrolyse von GOS gebildet. Der Polymerisationsgrad der GOS kann ziemlich stark schwanken und reicht von 2 bis 8 Monomereinheiten. Eine Reihe von Faktoren bestimmt dabei den Aufbau und die Reihenfolge Monomereinheiten: die Enzym-Quelle, das Ausgangsmaterial (Lactose-Konzentration und Ursprung der Lactose), die am Prozess beteiligten Enzyme, Bedingungen bei der Verarbeitung und die Zusammensetzung des Mediums.

### Lebensmittelemulgatoren

Emulgatoren zeichnen sich durch die wichtige Eigenschaft aus, sowohl in Wasser als auch in Fett löslich zu sein. Emulgatoren bestehen meist aus einem fettlöslichen und einem wasserlöslichen Teil. Sie kommen immer dann zum Einsatz, wenn Wasser und Öl zu einer beständigen, homogenen Vermischung gebracht werden sollen.

Geeignete Emulgatoren, die in der lebensmittelverarbeitenden Industrie verwendet werden sind ausgewählt aus: Ascorbylpalmitat (E 304) Lezithin (E 322) Phosphorsäure (E 338) Natriumphosphat (E 339) Kaliumphosphat (E 340) Kalziumphosphat (E 341) Magnesiumorthophosphat (E 343) Propylenglykolalginat (E 405) Polyoxyethylen(8)stearat (E 430) Polyoxyethylenstearat (E 431) Ammoniumphosphatide (E 442) Natriumphosphat und Kaliumphosphat (E 450) Natriumsalze der Speisefettsäuren (E 470 a) Mono- und Diglyceride von Speisefettsäuren (E 471) Essigsäuremonoglyceride (E 472 a) Milchsäuremonoglyceride (E 472 b) Zitronensäuremonoglyceride (E 472 c) Weinsäuremonoglyceride (E 472 d) Diacetylweinsäuremonoglyceride (E 472 e) Zuckerester von Speisefettsäuren (E 473) Zuckerglyceride (E 474) Polyglyceride von Speisefettsäuren (E 475) Polyglycerin-Polyricinoleat (E 476) Propylenglykolester von Speisefettsäuren (E 477) Natriumstearoyllaktylat (E 481) Calciumstearoyl-2-lactylat (E 482) Stearyltartrat (E 483) Sorbitanmonostearat (E 491) Stearinsäure (E 570).

### Verdickungsmittel

Verdickungsmittel sind Stoffe, die in erster Linie in der Lage sind, Wasser zu binden. Durch Entzug von ungebundenem Wasser kommt es zur Erhöhung der Viskosität. Ab einer für jedes Verdickungsmittel charakteristischen Konzentration treten zu diesem Effekt auch noch Netzwerkeffekte auf, die zu einer meist überproportionalen Erhöhung der Viskosität führen. Man spricht in diesem Fall davon, dass Moleküle miteinander 'kommunizieren', d.h. verschlaufen. Bei den meisten Verdickungsmitteln handelt es sich um lineare oder verzweigte Makromoleküle (z. B. Polysaccharide oder Proteine), die durch intermolekulare Wechselwirkungen, wie Wasserstoffbrücken, hydrophobe Wechselwirkungen oder lonenbeziehungen miteinander interagieren können. Extremfälle von Dickungsmitteln sind Schichtsilikate (Bentonite, Hectorite) oder hydratisierte SiOz-Partikel, die als Teilchen dispergiert vorliegen und in ihrer festkörperartigen Struktur Wasser binden können bzw. aufgrund der beschriebenen Wechselwirkungen miteinander interagieren können. Beispiele sind:
- E 400: - Alginsäure
- E 401: - Natriumalginat
- E 402: - Kaliumalginat
- E 403: - Ammoniumalginat
- E 404: - Calciumalginat
- E 405: - Propylenglycolalginat
- E 406: - Agar Agar
- E 407: - Carrgeen, Furcelleran
- E 407: - Johannisbrotkernmehl
- E 412: - Guarkernmehl
- E 413: - Traganth
- E 414: - Gummi arabicum
- E 415: - Xanthan
- E 416: - Karaya (Indischer Traganth)
- E 417: - Tarakernmehl (Peruanisches Johannisbrotkernmehl)
- E 418: - Gellan
- E 440: - Pektin, Opekta
- E 440ii: - Amidiertes Pektin
- E 460: - Mikrokristalline Cellulose, Cellulosepulver
- E 461: - Methylcellulose
- E 462: - Ethylcellulose
- E 463: - Hydroxypropylcellulose
- E 465: - Methylethylcellulose
- E 466: - Carboxymethylcellulose, Natriumcarboxymethylcellulose

### Lebensmittelsäuren

Die Nahrungsmittel können Carbonsäuren enthalten. Säuren im Sinne der Erfindung sind bevorzugt in Lebensmitteln zulässige Säuren, insbesondere die hier genannten:
- E 260: - Essigsäure
- E 270: - Milchsäure
- E 290: - Kohlendioxid
- E 296: - Apfelsäure
- E 297: - Fumarsäure
- E 330: - Citronensäure
- E 331: - Natriumcitrat
- E 332: - Kaliumcitrat
- E 333: - Calciumcitrat
- E 334: - Weinsäure
- E 335: - Natriumtartrat
- E 336: - Kaliumtartrat
- E 337: - Natrium-Kaliumtartrat
- E 338: - Phosphorsäure
- E 353: - Metaweinsäure
- E 354: - Calciumtartrat
- E 355: - Adipinsäure
- E 363: - Bernsteinsäure
- E 380: - Triammoniumcitrat
- E 513: - Schwefelsäure
- E 574: - Gluconsäure
- E 575: - Glucono-delta-Lacton

### Säureregulatoren

Säureregulatoren sind Lebensmittelzusatzstoffe, die den Säuregrad oder die Basizität und damit den gewünschten pH-Wert eines Lebensmittels konstant halten. Es handelt sich meist um organische Säuren und deren Salze, Carbonate, seltener auch um anorganische Säuren und deren Salze. Der Zusatz eines Säureregulators verstärkt teils die Stabilität und Festigkeit des Lebensmittels, bewirkt eine erwünschte Ausfällung und verbessert die Wirkung von Konservierungsmitteln. Im Gegensatz zu Säuerungsmitteln werden sie nicht zur Geschmacksveränderung von Lebensmitteln benutzt. Ihre Wirkung beruht auf der Bildung eines Puffersystems im Lebensmittel, bei dem sich auf Zugabe von sauren oder basischen Stoffen der pH-Wert nicht oder nur geringfügig ändert. Beispiele sind:
- E 170: - Calciumcarbonat
- E 260-263: - Essigsäure und Acetate
- E 270: - Milchsäure
- E 296: - Äpfelsäure
- E 297: - Fumarsäure
- E 325-327: - Lactate (Milchsäure)
- E 330-333: - Citronensäure und Citrate
- E 334-337: - Weinsäure und Tartrate
- E 339-341: - Orthophosphate
- E 350-352: - Malate (Äpfelsäure)
- E 450-452: - Di-, Tri- und Polyphosphate
- E 500-504: - Carbonate (Kohlensäure)
- E 507: - Salzsäure und ChlorideE 513-517 Schwefelsäure und Sulfate
- E 524-528: - Hydroxide
- E 529-530: - Oxide
- E 355-357: - Adipinsäure und Adipate
- E 574-578: - Gluconsäure und Gluconate

### Vitamine

In einer weiteren Ausführungsform der vorliegenden Erfindung können die Nahrungsmittelzusatzstoffe als weitere fakultative Gruppe von Zusatzstoffen Vitamine enthalten. Vitamine verfügen über unterschiedlichste biochemische Wirkungsweisen. Einige wirken ähnlich wie Hormone und regulieren den Mineralmetabolismus (z.B. Vitamin D), oder wirken auf das Wachstum von Zellen und Gewebe sowie die Zelldifferenzierung (z.B. einige Formen des Vitamin A). Andere stellen Antioxidantien dar (z.B. Vitamin E und unter bestimmten Umständen auch Vitamin C). Die größte Zahl von Vitaminen (z.B. die B-Vitamine) stellen Vorstufen für enzymatische Co-Faktoren dar, die Enzyme dabei unterstützen, bestimmte Prozesse im Metabolismus zu katalysieren. In diesem Zusammenhang können Vitamin mitunter eng an die Enzyme gebunden sein, beispielsweise als Teil der prostetischen Gruppe: ein Beispiel hierfür ist Biotin, das ein Teil des Enzyms ist, welches für den Aufbau von Fettsäuren verantwortlich ist. Vitamine können andererseits auch weniger stark gebunden sein und dann als CoKatalysatoren wirken, beispielsweise als Gruppen, die sich leicht abspalten lassen und chemische Gruppen oder Elektronen zwischen den Molekülen transportieren. So transportiert beispielsweise Folsäure Methyl-, Formyl- und Methylengruppen in die Zelle. Obwohl ihre Unterstützung in Enzym-Substrat-Reaktionen wohl bekannt ist, sind auch ihre übrigen Eigenschaften für den Körper von großer Bedeutung.

Im Rahmen der vorliegenden Erfindung kommen als Vitamine Stoffe in Betracht, die ausgewählt sind aus der Gruppe bestehend aus
- Vitamin A (Retinol, Retinal, Betakarotin),
- Vitamin B₁ (Thiamin),
- Vitamin B₂ (Rioflavin),
- Vitamin B₃ (Niacin, Niacinamid),
- Vitamin B₅ (Panthothensäure),
- Vitamin B₆ (Pyridoxin, Pyridoxamin, Paridoxal),
- Vitamin B₇ (Biotin),
- Vitamin B₉ (Folsäure, Folinsäure),
- Vitamin B₁₂ (Cyanobalamin, Hydroxycobalmin, Methylcobalmin),
- Vitamin C (Ascorbinsäure),
- Vitamin D (Cholecalciferol),
- Vitamin E (Tocopherole, Tocotrienole) und
- Vitamin K (Phyllolchinon, Menachinon).
Die bevorzugten Vitamine sind neben der Ascorbinsäure die Gruppe der Tocopherole.

### Antioxidantien

In der Lebensmittelindustrie werden sowohl natürliche als auch künstliche Antioxidationsmittel verwendet. Natürliche und künstliche Antioxidantien unterscheiden sich in erster Linie dadurch, dass erstere natürlich in der Nahrung vorkommen und letztere künstlich hergestellt werden. So werden natürliche Antioxidationsmittel, so sie als Lebensmittelzusatzstoff eingesetzt werden sollen, beispielsweise aus Pflanzenölen gewonnen. Vitamin E - auch als Tocopherol bekannt - wird beispielsweise häufig aus Sojaöl hergestellt. Synthetische Antioxidantien wie das Propylgallat, das Octylgallat und das Dodecylgallat werden dagegen durch chemische Synthese gewonnen. Die Gallate können bei empfindlichen Personen Allergien auslösen. Weitere einsetzbare Antioxidantien in Zusammensetzungen der vorliegenden Erfindung sind: Schwefeldioxid, E 220 Sulfite Natriumsulfit, E 221 Natriumhydrogensulfit, E 222 Natriumdisulfit, E 223 Kaliumdisulfit, E 224 Kalziumsulfit, E 226 Kalziumhydrogensulfit, E 227 Kaliumhydrogensulfit, E 228 Milchsäure, E 270 Ascorbinsäure, E 300 Natrium-L-Ascorbat, E 301 Calcium-L-Ascorbat, E 302 Ascorbinsäureester, E 304 Tocopherol, E 306 Alpha-Tocopherol, E 307 Gamma-Tocopherol, E 308 Delta-Tocopherol, E 309 Propylgallat, E 310 Octygallat, E 311 Dodecylgallat, E 312 Isoascorbinsäure, E 315 Natriumisoascorbat, E 316 tertiär-Butylhydrochinon (TBHQ), E 319 Butylhydroxianisol, E 320 Butylhydroxitoluol, E 321 Lecithin, E 322 Citronensäure, E 330 Salze der Zitronensäure (E 331 & E 332) Natriumzitrat, E 331 Kaliumzitrat, E 332 Calcium-Dinatrium-EDTA, E 385 Diphosphate, E 450 Dinatriumdiphosphat, E 450a Trinatriumdiphosphat, E 450b Tetranatriumdiphosphat, E 450c Dikaliumdiphosphat, E 450d Trekaliumdiphosphat, E 450e Dikalziumdiphosphat, E 450f Kalziumdihydrogendiphosphst, E 450g Triphosphate, E 451 Pentanatriumtriphosphat, E 451a Pentakaliumtriphosphat, E 451b Polyphosphat, E 452 Natriumpolyphosphat, E 452a Kaliumpolyphosphat, E 452b Natriumkalziumpolyphosphat, E 452c Kalziumpolyphosphat, E 452d Zinn-II-Chlorid, E 512.

### Geschmacksverstärker

Diese Zubereitungen- wie auch die Aromamischungen- können des Weiteren zusätzliche Aromastoffe zum Verstärken eines salzigen, gegebenenfalls leicht sauren und/oder Umami-Geschmackseindrucks enthalten. Es werden somit die erfindungsgemäßen Produkte bzw. Aromamischungen in Kombination mit zumindest einer weiteren zur Verstärkung eines an-genehmen Geschmackseindrucks (salzig, Umami, gegebenenfalls leicht sauer) geeigneten Substanz verwendet. Hierbei bevorzugt sind salzig schmeckende Verbindungen und salzverstärkende Verbindungen. Bevorzugte Verbindungen sind in der WO 2007/045566 offenbart. Ferner bevorzugt sind Umami-Verbindungen wie in der WO 2008/046895 und EP 1 989 944 beschrieben sind.

Weiterhin können erfindungsgemäß bevorzugte Aromamischungen und Produkte auch Aromastoffe zur Maskierung von bitteren und/oder adstringierenden Geschmackseindrücken umfassen (Geschmackskorrigentien). Die (weiteren) Geschmackskorrigenzien werden z. B. aus der folgenden Liste ausgewählt: Nucleotide (z.B. Adenosin-5'-monophosphat, Cytidin-5'-monophosphat) oder deren pharmazeutisch akzeptable Salze, Lactisole, Natriumsalze (z.B. Natriumchlorid, Natriumlactat, Natriumcitrat, Natriumacetat, Natriumgluconoat), weitere Hydroxyflavanone (z.B. Eriodictyol, Homoeriodictyol oder deren Natriumsalze), insbesondere gemäß US 2002/0188019, Hydroxybenzoesäureamide nach DE 10 2004 041 496 (z.B. 2,4-Dihydroxybenzoesäurevanillylamid, 2,4-Dihydroxybenzoesäure-N-(4-hydroxy-3-methoxybenzyl)amid, 2,4,6-Trihydroxybenzoesäure-N-(4-hydroxy-3-methoxybenzyl)amid, 2-Hydroxy-benzoesäure-N-4-(hydroxy-3-methoxybenzyl)amid, 4-Hydroxybenzoesäure-N-(4-hydroxy-3-methoxybenzyl)amid, 2,4-Dihydroxybenzoesäure-N-(4-hydroxy-3-methoxybenzyl)amid-Mono-natriumsalz, 2,4-Dihydroxybenzoesäure-N-2-(4-hydroxy-3-methoxyphenyl)-ethylamid, 2,4-Dihydroxybenzoesäure-N-(4-hydroxy-3-ethoxybenzyl)amid, 2,4-Dihydroxybenzoesäure-N-(3,4-dihydroxybenzyl)amid und 2-Hydroxy-5-methoxy-N-[2-(4-hydroxy-3-methoxyphenyl)ethyl]amid (Aduncamid), 4-Hydroxybenzoesäurevanillylamid), bittermaskierende Hydroxydeoxybenzoine z.B. gemäß WO 2006/106023 (z.B. 2-(4-Hydroxy-3-methoxyphenyl)-1-(2,4,6-tri¬ihydroxyphenyl)ethanon, 1-(2,4-Dihydroxyphenyl)-2-(4-hydroxy-3-methoxyphenyl)ethanon, 1-(2-Hydroxy-4-methoxyphenyl)-2-(4-hydroxy-3-methoxyphenyl)ethanon), Aminosäuren (z.B. gamma-Aminobuttersäure nach WO 2005/096841 zur Verminderung oder Maskierung eines unangenehmen Geschmackseindrucks wie Bitterkeit), Äpfelsäureglycoside nach WO 2006/003107, salzig schmeckende Mischungen gemäß PCT/EP 2006/067120 Diacetyltrimere gemäß WO 2006/058893, Gemische von Molkeproteinen mit Lecithinen und/oder bittermaskierende Substanzen wie Gingerdione gemäß WO 2007/003527.

Bevorzugte Aromastoffe sind solche, die einen süßen Geruchseindruck verursachen, wobei der oder die weiteren Aromastoffe, die einen süßen Geruchseindruck verursachen, bevor-zugt ausgewählt sind aus der Gruppe bestehend aus:
Vanillin, Ethylvanillin, Ethylvanillinisobutyrat (= 3 Ethoxy-4-isobutyryloxy-benzaldehyd), Furaneol (2,5-Dimethyl-4-hydroxy-3(2H)-furanon) und Abkömmlinge (z.B. Homofuraneol, 2-Ethyl-4-hydroxy-5-methyl-3(2H)-furanon), Homofuronol (2-Ethyl-5-methyl-4-hydroxy-3(2H)-furanon und 5-Ethyl-2-methyl-4-hydroxy-3(2H)-furanon), Maltol und Abkömmlinge (z.B. Ethylmaltol), Cumarin und Abkömmlinge, gamma-Lactone (z.B. gamma-Undecalacton, gamma-Nonalacton), delta-Lactone (z.B. 4-Methyldeltalacton, Massoilacton, Deltadecalac-ton, Tuberolacton), Methylsorbat, Divanillin, 4-Hydroxy-2(oder 5)-ethyl-5(oder 2)-methyl-3(2H)furanon, 2-Hydroxy-3-methyl-2-cyclopentenone, 3-Hydroxy-4,5-dimethyl-2(5H)-furanon, Fruchtester und Fruchtlactone (z.B. Essigsäure-n-butylester, Essigsäureisoamylester, Propionsäureethylester, Buttersäureethylester, Buttersäure-n-butylester, Buttersäurei-soamylester, 3-Methyl-buttersäureethylester, n-Hexansäureethylester, n-Hexansäureallylester, n-Hexansäure-n-butylester, n-Octansäureethylester, Ethyl-3-methyl-3-phenylglycidat, Ethyl-2-trans-4-cis-decadienoat), 4-(p-Hydroxyphenyl)-2-butanon, 1,1-Dimethoxy-2,2,5-trimethyl-4-hexan, 2,6-Dimethyl-5-hepten-1-al, 4-Hydroxyzimtsäure, 4-Methoxy-3-hydroxyzimtsäure, 3-Methoxy-4-hydroxyzimtsäure, 2-Hydroxyzimtsäure, 2,4-Dihydroxybenzoesäure, 3-Hydroxybenzoesäure, 3,4-Dihydroxybenzoesäure, Vanillinsäure, Homovanillinsäure, Vanillomandelsäure und Phenylacetaldehyd.

### Wirkstoffe zur Maskierung von unangenehmen Geschmackseindrücken

Weiterhin können die oralen Zubereitungen auch weitere Stoffe umfassen, die ebenfalls zur Maskierung von bitteren und/oder adstringierenden Geschmackseindrücken dienen. Diese weiteren Geschmackskorrigenzien werden z. B. aus der folgenden Liste ausgewählt: Nucleotiden (z.B. Adenosin-5'-monophosphat, Cytidin-5'-monophosphat) oder deren physiologisch akzeptablen Salzen, Lactisolen, Natriumsalzen (z.B. Natriumchlorid, Natriumlactat, Natriumcitrat, Natriumacetat, Natriumgluconoat), Hydroxyflavanonen, dabei bevorzugt Eriodictyol, Sterubin (Eriodictyol-7-methylether), Homoeriodictyol, und deren Natrium-, Kalium-, Calcium-, Magnesium- oder Zinksalzen (insbesondere solche wie beschrieben in EP 1258200 A2, die bezüglich der darin offenbarten entsprechenden Verbindungen auf dem Wege der Verweisung Bestandteil dieser Anmeldung wird), Hydroxybenzoesäureamiden, dabei vorzugsweise 2,4-Dihydroxybenzoesäurevanillylamid, 2,4-Dihydroxybenzoesäure-*N*-(4-hydroxy-3-methoxybenzyl)amid, 2,4,6-Trihydroxybenzoesäure-*N*-(4-hydroxy-3-methoxybenzyl)amid, 2-Hydroxy-benzoesäure-*N*-4-(hydroxy-3-methoxybenzyl)amid, 4-Hydroxybenzoesäure-*N*-(4-hydroxy-3-methoxybenzyl)amid, 2,4-Dihydroxybenzoesäure-*N*-(4-hydroxy-3-methoxybenzyl)amid-Mono-natriumsalz, 2,4-Dihydroxybenzoesäure-*N*-2-(4-hydroxy-3-methoxyphenyl)ethylamid, 2,4-Dihydroxybenzoesäure-*N*-(4-hydroxy-3-ethoxybenzyl)amid, 2,4-Dihydroxybenzoesäure-*N*-(3,4-dihydroxybenzyl)amid und 2-Hydroxy-5-methoxy-N-[2-(4-hydroxy-3-methoxyphenyl)ethyl]amid; 4-Hydroxybenzoesäurevanillylamiden (insbesondere solche wie beschrieben in WO 2006/024587, die bezüglich der darin offenbarten entsprechenden Verbindungen auf dem Wege der Verweisung Bestandteil dieser Anmeldung wird); Hydroxydeoxybenzoinen, dabei vorzugsweise 2-(4-Hydroxy-3-methoxyphenyl)-1-(2,4,6-trihydroxyphenyl)ethanon, 1-(2,4-Dihydroxyphenyl)-2-(4-hydroxy-3-methoxyphenyl)-ethanon und 1-(2-Hydroxy-4-methoxyphenyl)-2-(4-hydroxy-3-methoxyphenyl)ethanon) (insbesondere solche wie beschrieben in WO 2006/106023 beschrieben, die bezüglich der darin offenbarten entsprechenden Verbindungen auf dem Wege der Verweisung Bestandteil dieser Anmeldung wird); Hydroxyphenylalkandionen, wie zum Beispiel Gingerdion-[2], Gingerdion-[3], Gingerdion-[4], Dehydrogingerdion-[2], Dehydrogingerdion-[3], Dehydrogingerdion-[4]) (insbesondere solche wie beschrieben in WO 2007/003527, die bezüglich der darin offenbarten entsprechenden Verbindungen auf dem Wege der Verweisung Bestandteil dieser Anmeldung wird); Diacetyltrimeren (insbesondere solche wie beschrieben in WO 2006/058893, die bezüglich der darin offenbarten entsprechenden Verbindungen auf dem Wege der Verweisung Bestandteil dieser Anmeldung wird); gamma-Aminobuttersäuren (insbesondere solche wie beschrieben in WO 2005/096841, die bezüglich der darin offenbarten entsprechenden Verbindungen auf dem Wege der Verweisung Bestandteil dieser Anmeldung wird); Divanillinen (insbesondere solche wie beschrieben in WO 2004/078302, die bezüglich der darin offenbarten entsprechenden Verbindungen auf dem Wege der Verweisung Bestandteil dieser Anmeldung wird) und 4-Hydroxydihydrochalconen (vorzugsweise wie beschrieben in US 2008/0227867 A1, die bezüglich der darin offenbarten entsprechenden Verbindungen auf dem Wege der Verweisung Bestandteil dieser Anmeldung wird), dabei insbesondere Phloretin und Davidigenin, Aminosäuren oder Gemische von Molkeproteinen mit Lecithinen, Hesperetin wie in der WO 2007/014879 offenbart, die hinsichtlich dieser Verbindungen auf dem Wege der Verweisung Bestandteil dieser Anmeldung wird, 4-Hydroxydihydrochalkonen wie in der WO 2007/107596 offenbart, die hinsichtlich dieser Verbindungen auf dem Wege der Verweisung Bestandteil dieser Anmeldung wird, oder Propenylphenylglycosiden (Chavicolglycosiden) wie in EP 1955601 A1 beschrieben, die hinsichtlich dieser Verbindungen auf dem Wege der Verweisung Bestandteil dieser Anmeldung wird, oder Extrakten aus *Rubus suavissimus,* Extrakte aus *Hydrangea macrophylla* wie in EP 2298084 A1 beschrieben, Pellitorin und abgeleiteten Aromakompositionen wie in EP 2008530 A1 beschrieben, Umami-Verbindungen wie in WO 2008/046895 A1 und EP 1989944 A1 beschrieben, Umami-Verbindungen wie beschrieben in EP 2064959 A1 bzw. EP 2135516 A1, Vanillyllignanen, Enterodiol, sowie N-Decadienoylaminosäuren und deren Gemische.

### Lebensmittelfarbstoffe

Lebensmittelfarbstoffe oder kurz Farbstoffe sind Lebensmittelzusatzstoffe zum Färben von Lebensmittel. Farbstoffe werden in die Gruppen der natürlichen Farbstoffe und synthetischen Farbstoffe unterteilt. Die naturidentischen Farbstoffe sind ebenfalls synthetischen Ursprungs. Die naturidentischen Farbstoffe sind synthetische Nachbildungen von in der Natur vorkommenden, färbenden Substanzen. Geeignete Farbstoffe für den Einsatz in der vorliegenden Zusammensetzung sind ausgewählt aus: Kurkumin, E 100 Riboflavin, Lactoflavin, Laktoflavin, Vitamin B2, E 101 Tartrazin, E 102 Chinolingelb, E 104 Gelborange S, Gelborange RGL, E 110 Cochenille, Karminsäure, echtes Karmin, E 120 Azorubin, Carmoisin, E 122 Amaranth, E 123 Cochenillerot A, Ponceau 4 R, Victoriascharlach 4 R, E 124 Erythrosin, E 127 Allurarot AC, E 129 Patentblau V, E 131 Indigotin, Indigo-Karmin, E 132 Brillantblau FCF, Patentblau AE, Amidoblau AE, E 133 Chlorophylle, Chlorophylline, E 140 Kupferkomplexe der Chlorophylle, Kupfer-Chlorophyllin-Komple, E 141 Brillantsäuregrün, Grün S, E 142 Zuckerkulör, Zuckercouleur, E 150 a Sulfitlaugen-Zuckerkulör, E 150 b Ammoniak-Zuckerkulör, E 150 c Ammoniumsulfit-Zuckerkulör, E 150 d Brillantschwarz FCF, Brillantschwarz PN, Schwarz PN, E 151 Pflanzenkohle, E 153 Braun FK, E 154 Braun HT, E 155 Carotin, Karotin, E 160 a Annatto, Bixin, Norbixin, E 160 b Capsanthin, Capsorubin, E 160 c Lycopin, E 160 d Beta-apo-8'-Carotinal, Apocarotinal, Beta-Apocarotinal, E 160 e Beta-apo-8'-Carotinsäure-Ethylester (C30), Apocarotinester, Beta-Carotinsäureester, E 160 f Lutein, Xanthophyll, E 161 b Canthaxanthin, E 161 g Betanin, Betenrot, E 162 Anthocyane, E 163 Calciumcarbonat, E 170 Titandioxid, E 171 Eisenoxide, Eisenhydroxide, E 172 Aluminium, E 173 Silber, E 174 Gold, E 175 Litholrubin BK, Rubinpigment BK, E 180.

### MEDIKAMENTE

Die beiden wesentlichen Eigenschaften der neuen Kühlstoffe besteht wie gesagt zum einen darin, als Antagonisten den TRPM8-Rezeptor zu modulieren und auf diese Weise eine physiologische Reaktion, nämlich eine Kühlwirkung auf Haut oder Schleimhaut auszulösen, und zum anderen unangenehme Geschmacksnoten zu maskieren. Die physiologische Kühlwirkung macht man sich zu Nutze etwa bei der Formulierung von Wund- und Brandsalben sowie Zubereitungen gegen Insektenstiche.

Ein weiterer Gegenstand der vorliegenden Erfindung umfasst daher die Kühlstoffe der Formeln (I) bis (V) als Medikamente, insbesondere als Medikamente zur Verwendung zur Linderung von Schmerzen und Entzündungszuständen der Haut und der Schleimhäute.

Adstringierende, bittere und/oder metallische Geschmacksnoten finden sich jedoch nicht nur wie oben beschrieben bei Aromen und Süßstoffen, sondern auch im Zusammenhang mit vielen pharmazeutischen Wirkstoffen, was deren Einnahme insbesondere bei Kindern erschwert. Typische Beispiele für solche pharmazeutische Wirkstoffe sind die folgenden:

| | |
|---|---|
| 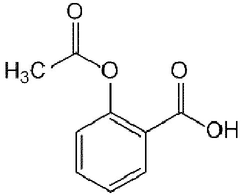 | **Aspirin** |
| | Acetylsalicylsäure |
| 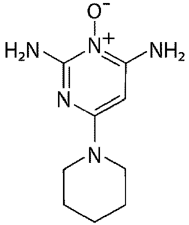 | **Minoxidil** |
| | (6-piperidin-1-ylpyrimidine-2,4-diamine 3-oxid) |
| 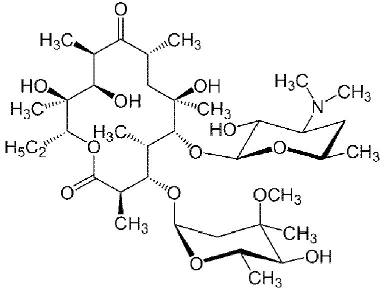 | **Erythromycin** |
| 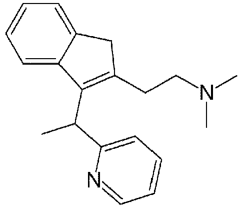 | **Dimetinden (Fenistil)** |
| | (RS-dimethyl(2-(3-[pyridin-2-yl)ethyl]-1H-inden-2-yl)ethyl)amin) |
| 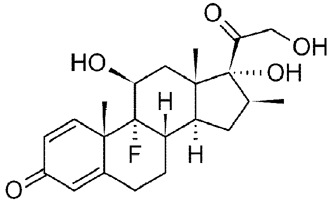 | **Betamethason** |
| | (8S,9R,10S.11S,13S,14S,16S,17R)-9-fluoro-11,17-(2-hydroxyacetyl)-10, 13,16-trimethyl-6,7,8,9,10,11,12,13,14,15,16,17-dodecahydro-3H-cyclopenta(alpha) |
| 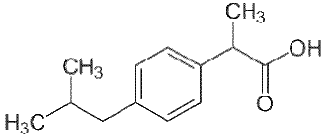 | **Ibuprofen** |
| | (RS)-2-(4-(2-methylpropyl)phenyl)propansäure) |
| 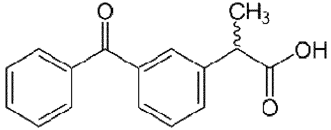 | **Ketoprofen** |
| | (RS)2-(3-benzoylphenyl)-propansäure |
| 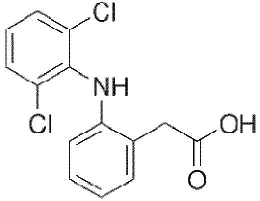 | **Dicylofenac** |
| | 2-(2,6-dichloranilino) phenylessigsäure |
| 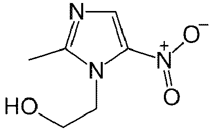 | **Metronidazol** |
| | (2-(2-methyl-5-nitro-1H-imidazol-1-yl)ethanol) |
| 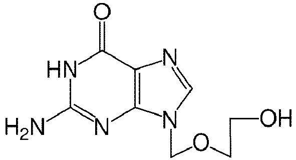 | **Acyclovir** |
| | (2-Amino-1,9-dihydro-9-((2-hydroxyethoxy)methyl)-6H-Purin-6-on), |
| 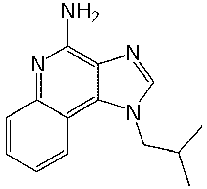 | **Imiquimod** |
| | (3-(2-methylpropyl)-3,5,8-triazatricyclo[7.4.0.0.^{2,6}]trideca-1(9),2(6),4,7,10, 12-hexaen-7-amin) |
| 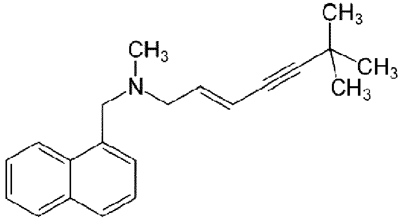 | **Terbinafin** |
| | [(2E)-6,6-dimethylhept-2-en-4-yn-1-yl](methyl)(naphthalen-1-ylmethyl)amin) |
| 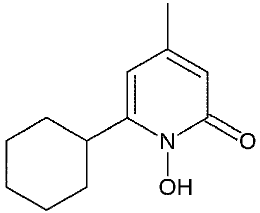 | **Cyclopiroxolamine** |
| | (6-cyclohexyl-1-hydroxy-4-methylpyridin-2(1H)-on) |

Ein weiterer Gegenstand der vorliegenden Erfindung umfasst daher Medikamente enthaltend die Kühlstoffe der Formeln (I) bis (V) sowie pharmazeutische Wirkstoffe ausgewählt aus der Gruppe, die gebildet wird von Aspirin, Minoxidil, Erythromycin, Fenistil, Betamethason, Ibuprofen, Ketoprofen, Dicyclofenac, Metronidazol, Acylovir, Imiquimod, Terbafin, Cyclopiroxolamin sowie deren Gemische.

In Probandenstudien hat sich ferner gezeigt, dass die erfindungsgemäßen Kühlstoffe der Formeln (I) bis (V) die schmerzmindernden Eigenshaften von nichtsteroidalen entzündungshemmenden Stoffen (NSAID), insbesondere von Ibuprofen und Ketoprofen über die Kühlwirkung hinaus verstärken, was für den Fachmann ebenfalls nicht zu erwarten gewesen ist.

Ein weiterer Gegenstand der vorliegenden Erfindung umfasst damit ferner Medikamente enthaltend die Kühlstoffe der Formeln (I) bis (V) sowie pharmazeutische Wirkstoffe vom Typ der NSAID insbesondere zur Verwendung in der Behandlung von Entzündungszuständen der Haut und der Schleimhaut sowie der Gelenke.

Die Medikamente können die Kühlstoffe der Formeln (I) bis (V) und die pharmazeutischen Wirkstoffe im Gewichtsverhältnis von etwa 1:99 bis etwa 10:90 und insbesondere 2:98 bis etwa 5:95 enthalten.

### GEWERBLICHE ANWENDBARKEIT

Ein weiterer Gegenstand der Erfindung betrifft ein Verfahren zur Erzeugung eines physiologischen Kühleffektes auf Haut oder Schleimhaut, umfassend die folgenden Schritte:
(i) Bereitstellen von mindestens einem erfindungsgemäßen Kühlstoff oder einer entsprechenden Kühlstoffmischung,
(ii) gegebenenfalls Formulierung der Kühlstoffe in eine kosmetische, pharmazeutische oder orale Zusammensetzung; und
(iii) in Kontaktbringen der Zubereitungen aus Schritt (i) oder Schritt (ii) mit menschlicher Haut oder Schleimhaut.

Ebenfalls beansprucht wird ein Verfahren zur Verbesserung der geschmacklichen Eigenschaften von Aromastoffen umfassend die folgenden Schritte:
(i) Bereitstellen von mindestens einem erfindungsgemäßen Kühlstoff und mindestens einem Aromastoff;
(ii) vermischen der beiden Komponenten sowie gegebenenfalls
(iii) Einarbeitung der Mischung in eine orale Zusammensetzung.

Zwei weitere Gegenstände der vorliegenden Erfindung betreffen zu einen
- die Verwendung der erfindungsgemäßen Kühlstoffe oder der entsprechenden Kühlstoffmischungen zur Erzeugung eines physiologischen Kühleffektes auf Haut oder Schleimhaut sowie zum anderen
- die Verwendung der erfindungsgemäßen Kühlstoffe oder der entsprechenden Kühlstoffmischung zur Verbesserung der geschmacklichen Eigenschaften von Aromastoffen.

Soweit bevorzugte Ausführungsformen betroffen sind, wird auf die vorstehenden Erläuterungen verwiesen, die für die erfindungsgemäßen Anwendungsverfahren und Verwendungen in gleicher Weise gelten, ohne dass es hierzu einer Wiederholung bedarf.

### BEISPIELE

### A. STABILITÄT

### Beispiele 8 bis 14, Vergleichsbeispiele V5 bis V8

Verschiedene klare O/W-Sonnenschutzemulsionen wurden nach der PIT-Methode durch Vermischen der Komponenten gemäß **Tabelle 1A** hergestellt:

**Tabelle 1A**

| Zusammensetzung O/W-Sonnenschutzlotionen | | |
|---|---|---|
| **Komponente** | **Handelsprodukt** | **Menge [Gew.-%]** |
| Polyglyceryl-2-Polyhydroxystearate (and) Lauryl Glucosides (and) Glycerin | Eumulgin^{®} VL 75 | 2,5 |
| Glyceryl Stearate | Cutina^{®} GMS | 2,0 |
| Cetearyl Alcohol | Lanette^{®} O | 4,0 |
| PVP/Hexadecen Copolymer | Antaron^{®} V216 | 3,0 |
| Cocoglycerides | Myritol^{®} 818 | 6,0 |
| Oleyl Erucate | Cetiol^{®} J600 | 3,0 |
| Dicaprylyl Ether | Cetiol^{®} OE | 5,0 |
| Mineralöl | | 2,0 |
| Bisabolol | | 1,2 |
| Tocopherol | Copherol^{®} F 1300 | 1,0 |
| Octocrylene | Neo Heliopan^{®} 303 | 4,0 |
| Isoamyl-p-methoxycinnamat | Neo Heliopan^{®} E 1000 | 2,0 |
| Octyl-methoycinnamate | Neo Heliopan^{®} AV | 3,0 |
| Octyl Triazon | Uvinul^{®} T15 | 1,0 |
| Kühlstoff | | 0,5 |
| Glycerin | | 5,0 |
| Wasser | | Ad 100 |

Die Sonnenschutzlotionen unterschieden sich lediglich im Hinblick auf den Kühlstoff, bei dem es sich zum einen um verschiedene Mentholverbindungen alleine und in Abmischungen handelte.

Nach der Herstellung wurden die Lotionen in klare PET-Flaschen abgefüllt und diese bei 30 °C gelagert. Anschließend wurden die Lotionen nach 12, 24 und 48 h hinsichtlich ihres Erscheinungsbildes beurteilt. Dabei bedeutet bezüglich der Stabilität: (+) = unverändert; (#) geringfügige Tröpfchenbildung und (-) Abscheidung von Öltröpfchen an der Oberfläche sowie leicht gelbliche Verfärbung.

Die Ergebnisse sind in **Tabelle 1B** zusammengefast. Die Beispiele 1 bis 7 sind erfindungsgemäß, die Beispiele V1 bis V4 dienen zum Vergleich. Die erfindungsgemäßen Zubereitungen zeigen dabei eine deutlich verbesserte Stabilität.

**Tabelle 1B**

| Bewertung von O/W-Sonnenschutzlotionen in Abhängigkeit der Kühlstoffe | | | | | | | | | | | |
|---|---|---|---|---|---|---|---|---|---|---|---|
| **Kühlstoff** | **V1** | **V2** | **V3** | **V4** | 1 | **2** | **3** | **4** | **5** | **6** | **7** |
| Menthol | 100 | - | - | - | - | - | 50 | - | - | - | - |
| Menthon Glyceyl Acetat | - | 100 | - | - | - | - | - | 50 | - | - | - |
| Menthyl Lactat | - | - | 100 | - | - | - | - | - | 50 | - | - |
| 2-(p-tolyloxy)-N-(1H-pyrazol-5-yl)-N-((thiophen-2-yl)methyl)acetamid | - | - | - | 100 | - | - | - | - | - | 50 | 50 |
| Kühlstoff Struktur 1 | - | - | - | - | 100 | - | 50 | - | 50 | 50 | - |
| Kühlstoff Struktur 4 | - | - | - | - | - | 100 | - | 50 | - | - | 50 |

| **Bewertung der Stabilität** | | | | | | | | | | | |
|---|---|---|---|---|---|---|---|---|---|---|---|
| Nach 12 h | + | + | + | + | + | + | + | + | + | + | + |
| Nach 24 h | # | + | # | + | + | + | + | + | + | + | + |
| Nach 48 h | - | # | # | # | + | # | + | + | + | + | + |

Die Beispiele und Vergleichsbeispiele zeigen, dass Emulsionen unter Verwendung der erfindungsgemäßen Kühlstoffe eine erhöhte Stabilität gegen Entmischung aufweisen. Diese lässt sich weiter verbessern, wenn man Mischungen der erfindungsgemäßen Kühlstoffe mit weiteren Kühlstioffen einsetzt.

### B. KÜHLLEISTUNG

### Beispiele 8 bis 14, Vergleichsbeispiele V5 bis V8

Es wurden verschiedene Mundwasserzubereitungen hergestellt, jeweils enthaltend 42 Gew.-% Alkohol, 5 Gew.-% ethoxyliertes Castoröl, 0,2 Gew.-% Allantoin, 0,1 Gew.-% Natrium Saccharin 450, 1 Gew.-% Kühlstoff(e) sowie ad 100 Gew.-% deminealisiertes Wasser. Zur Bestimmung der Kühlleistung wurden jeweils 10 ml der Lösungen mit Wasser auf 100 ml verdünnt und die Verdünnungen dann von 10 Probanden bestimmungsgemäß angewendet. Die Kühlwirkung in der Mundhöhle und auf den Schleimhäuten wurde sofort, nach 30 Sekunden sowie nach 10, 20 und 30 Minuten auf einer Skala von (10) = sehr intensive bis (1) = kaum mehr wahrnehmbar beurteilt. Die Ergebnisse finden sich in **Tabelle** 2; angegeben sind jeweils die Mittelwerte pro Testreihe. Die Beispiel 8 bis 14 sind erfindungsgemäß, die Beispiele V5 bis V8 dienen zum Vergleich.

**Tabelle 2**

| Bewertung von O/W-Sonnenschutzlotionen in Abhängigkeit der Kühlstoffe | | | | | | | | | | | |
|---|---|---|---|---|---|---|---|---|---|---|---|
| **Kühlstoffe** | **V5** | **V6** | **V7** | **V8** | **8** | **9** | **10** | **11** | **12** | **13** | **14** |
| Menthol | 100 | - | - | - | - | - | 50 | - | - | - | - |
| Menthon Glyceyl Acetat | - | 100 | - | - | - | - | - | 50 | - | - | - |
| Menthyl Lactat | - | - | 100 | - | - | - | - | - | 50 | - | - |
| 2-(p-tolyloxy)-N-(1H-pyrazol-5-yl)-N-((thiophen-2-yl)methyl)acetamid | - | - | - | 100 | - | - | - | - | - | 50 | 50 |
| Kühlstoff Struktur 1 | - | - | - | - | 100 | - | 50 | - | 50 | 50 | - |
| Kühlstoff Struktur 4 | - | - | - | - | - | 100 | - | 50 | - | - | 50 |

| **Bewertung der Kühlleistung** | | | | | | | | | | | |
|---|---|---|---|---|---|---|---|---|---|---|---|
| sofort | 9 | 9 | 9 | 10 | 10 | 10 | 10 | 10 | 10 | 10 | 10 |
| Nach 30 Sekunden | 6 | 6 | 7 | 6 | 8 | 8 | 8 | 8 | 8 | 8 | 8 |
| Nach 10 Minuten | 4 | 4 | 4 | 4 | 7 | 7 | 7 | 7 | 7 | 7 | 7 |
| Nach 20 Minuten | 3 | 2 | 3 | 3 | 5 | 5 | 5 | 5 | 5 | 6 | 6 |
| Nach 30 Minuten | 1 | 1 | 1 | 1 | 4 | 4 | 4 | 4 | 4 | 5 | 5 |

Die Beispiele und Vergleichsbeispiele zeigen, dass die erfindungsgemäßen Kühlstoffe, insbesondere in Kombination mit weiteren Kühlstoffen über eine signifikant längere Kühlwirkung verfügen.

### C. SENSORISCHE BEURTEILUNG

### Beispiele 15 bis 54, Vergleichsbeispiele V9 bis V23

Kaugummimassen bestehend aus 20 Gew.-% Polyisobutylen (MW 60.000), 51 Gew.-% Sorbitol, 5 Gew.-% Mannitol, 8 Gew.-% Glycerin, 8,2 Gew.-% einer 1:1-Mischung aus Lycasin und Glycerin, 0,2 Gew.-% Lecithin (ad 99,5 Gew.-% Wasser) wurden hergestellt und mit jeweils 0,5 Gew.-% verschiedener Aromen, Süßstoffe bzw. pharmazeutischer Wirkstoffe versetzt. Anschließend wurden die Kaugummimassen von einem Panel bestehend aus 5 geschulten Personen sensorisch auf einer Skala von 1 (kaum zu bemerken) bis 10 (dominant) bewertet. Als Standard diente dabei eine Formulierung ohne Kühlstoffe. Die Zusammensetzung der Kühlstoffe sowie die Bewertung der einzelnen Geschmacksnoten (angegeben ist jeweils der Mittelwert der Beurteilungen) sind in **Tabelle 1A bis 1E** zusammengefasst. Die Beispiele 15 bis 54 sind erfindungsgemäß, die Beispiele V9 bis V23 dienen zum Vergleich.

**Tabelle 1A**

| Sensorische Bewertung von Kaugummis mit Methylsalicylat | | | | | | | | | | | | |
|---|---|---|---|---|---|---|---|---|---|---|---|---|
| **Kühlstoffe** | **0** | **V9** | **V10** | **V11** | **15** | **16** | **17** | **18** | **19** | **20** | **21** | **22** |
| Methylsalicylat | 100 | 95 | 95 | 95 | 99 | 95 | 95 | 90 | 80 | 80 | 80 | 80 |
| Menthol | - | 5 | - | - | - | - | - | - | - | 10 | - | |
| Menthon Glyceyl Acetat | - | - | 5 | - | - | - | - | - | - | - | 10 | |
| Menthyl Lactat | - | - | - | 5 | - | - | - | - | - | - | - | 10 |
| Kühlstoff Struktur 1 | - | - | - | - | 1 | 5 | - | 10 | - | 10 | - | 10 |
| Kühlstoff Struktur 2 | - | - | - | - | - | - | 5 | - | 20 | - | 10 | |

| ***Bewertung*** | | | | | | | | | | | | |
|---|---|---|---|---|---|---|---|---|---|---|---|---|
| Süß | 2 | 2 | 2 | 2 | 3 | 3 | 3 | 3 | 3 | 3 | 3 | 3 |
| Heuartig-minzig | 2 | 2 | 2 | 2 | 3 | 3 | 3 | 3 | 3 | 3 | 3 | 3 |
| Scharf | 8 | 6 | 6 | 6 | 5 | 4 | 4 | 2 | 2 | 2 | 2 | 2 |
| Stechend | 6 | 6 | 6 | 6 | 5 | 4 | 4 | 2 | 2 | 2 | 2 | 2 |
| Bitter | 7 | 4 | 4 | 4 | 3 | 2 | 2 | 2 | 1 | 1 | 1 | 1 |
| Teerartig | 5 | 3 | 3 | 3 | 2 | 2 | 2 | 2 | 2 | 2 | 2 | 2 |

**Tabelle 1B**

| Sensorische Bewertung von Kaugummis mit Isopulegol | | | | | | | | | | | | |
|---|---|---|---|---|---|---|---|---|---|---|---|---|
| **Kühlstoffe** | **0** | **V12** | **V13** | **V14** | **23** | **24** | **25** | **26** | **27** | **28** | **29** | **30** |
| Isopulegol | 100 | 95 | 95 | 95 | 99 | 95 | 95 | 90 | 80 | 80 | 80 | 80 |
| Menthol | - | 5 | - | - | - | - | - | - | - | 10 | - | |
| Menthon Glyceyl Acetat | - | - | 5 | - | - | - | - | - | - | - | 10 | |
| Menthyl Lactat | - | - | - | 5 | - | - | - | - | - | - | - | 10 |
| Kühlstoff Struktur 1 | - | - | - | - | 1 | 5 | - | 10 | - | 10 | - | 10 |
| Kühlstoff Struktur 2 | - | - | - | - | - | - | 5 | - | 20 | - | 10 | |

| ***Bewertung*** | | | | | | | | | | | | |
|---|---|---|---|---|---|---|---|---|---|---|---|---|
| Süß | 2 | 2 | 2 | 2 | 3 | 3 | 3 | 3 | 3 | 3 | 3 | 3 |
| Heuartig-minzig | 2 | 2 | 2 | 2 | 3 | 3 | 3 | 3 | 3 | 3 | 3 | 3 |
| Scharf | 7 | 6 | 6 | 6 | 5 | 4 | 4 | 2 | 2 | 2 | 2 | 2 |
| Stechend | 7 | 6 | 6 | 6 | 5 | 3 | 3 | 2 | 2 | 2 | 2 | 2 |
| Bitter | 5 | 4 | 4 | 4 | 3 | 3 | 3 | 3 | 1 | 1 | 1 | 1 |
| Teerartig | 3 | 3 | 3 | 3 | 2 | 2 | 2 | 2 | 2 | 2 | 2 | 2 |

**Tabelle 1C**

| Sensorische Bewertung von Kaugummis mit Aspartam | | | | | | | | | | | | |
|---|---|---|---|---|---|---|---|---|---|---|---|---|
| **Kühlstoffe** | **0** | **V15** | **V16** | **V17** | **31** | **32** | **33** | **34** | **35** | **36** | **37** | **38** |
| Aspartam | 100 | 95 | 95 | 95 | 99 | 95 | 95 | 90 | 80 | 80 | 80 | 80 |
| Menthol | - | 5 | - | - | - | - | - | - | - | 10 | - | - |
| Menthon Glyceyl Acetat | - | - | 5 | - | - | - | - | - | - | - | 10 | - |
| Menthyl Lactat | - | - | - | 5 | - | - | - | - | - | - | - | 10 |
| Kühlstoff Struktur 1 | - | - | - | - | 1 | 5 | - | 10 | - | 10 | | 10 |
| Kühlstoff Struktur 2 | - | - | - | - | - | - | 5 | - | 20 | - | 10 | - |

| ***Bewertung*** | | | | | | | | | | | | |
|---|---|---|---|---|---|---|---|---|---|---|---|---|
| Süß | 8 | 8 | 8 | 8 | 9 | 9 | 9 | 9 | 9 | 9 | 9 | 9 |
| Heuartig-minzig | 1 | 2 | 2 | 2 | 2 | 2 | 2 | 2 | 2 | 2 | 2 | 2 |
| Scharf | 7 | 5 | 5 | 5 | 3 | 3 | 3 | 3 | 2 | 2 | 2 | 2 |
| Stechend | 7 | 5 | 5 | 5 | 4 | 4 | 4 | 4 | 2 | 2 | 2 | 2 |
| Bitter | 5 | 4 | 4 | 4 | 2 | 2 | 2 | 2 | 2 | 2 | 2 | 2 |
| Metallisch | 6 | 4 | 4 | 4 | 2 | 2 | 2 | 2 | 2 | 1 | 1 | 1 |

**Tabelle 10**

| Sensorische Bewertung von Kaugummis mit Rebaudiosid A | | | | | | | | | | | | |
|---|---|---|---|---|---|---|---|---|---|---|---|---|
| **Kühlstoffe** | **0** | **V18** | **V19** | **V20** | **39** | **40** | **41** | **42** | **43** | **44** | **45** | **46** |
| Rebaudiosid A | 100 | 95 | 95 | 95 | 99 | 95 | 95 | 90 | 80 | 80 | 80 | 80 |
| Menthol | - | 5 | - | - | - | - | - | - | - | 10 | - | - |
| Menthon Glyceyl Acetat | - | - | 5 | - | - | - | - | - | - | - | 10 | - |
| Menthyl Lactat | - | - | - | 5 | - | - | - | - | - | - | - | 10 |
| Kühlstoff Struktur 1 | - | - | - | - | 1 | 5 | - | 10 | - | 10 | - | 10 |
| Kühlstoff Struktur 2 | - | - | - | - | - | - | 5 | - | 20 | - | 10 | - |

| ***Bewertung*** | | | | | | | | | | | | |
|---|---|---|---|---|---|---|---|---|---|---|---|---|
| Süß | 8 | 8 | 8 | 8 | 9 | 9 | 9 | 9 | 9 | 9 | 9 | 9 |
| Heuartig-minzig | 1 | 2 | 2 | 2 | 2 | 2 | 2 | 2 | 2 | 2 | 2 | 2 |
| Scharf | 6 | 5 | 5 | 5 | 3 | 3 | 3 | 3 | 1 | 1 | 1 | 1 |
| Stechend | 6 | 5 | 5 | 5 | 4 | 4 | 4 | 4 | 1 | 1 | 1 | 1 |
| Bitter | 6 | 4 | 4 | 4 | 2 | 2 | 2 | 2 | 2 | 2 | 2 | 2 |
| Metallisch | 8 | 5 | 5 | 5 | 2 | 2 | 2 | 2 | 2 | 1 | 1 | 1 |

**Tabelle 1E**

| Sensorische Bewertung von Kaugummis mit Ibuprofen | | | | | | | | | | | | |
|---|---|---|---|---|---|---|---|---|---|---|---|---|
| **Kühlstoffe** | **0** | **V21** | **V22** | **V23** | **47** | **48** | **49** | **50** | **51** | **52** | 53 | **54** |
| Ibuprofen | 100 | 95 | 95 | 95 | 99 | 95 | 95 | 90 | 80 | 80 | 80 | 80 |
| Menthol | - | 5 | - | - | - | - | - | - | - | 10 | - | - |
| Menthon Glyceyl Acetat | - | - | 5 | - | - | - | - | - | - | - | 10 | - |
| Menthyl Lactat | - | - | - | 5 | - | - | - | - | - | - | - | 10 |
| Kühlstoff Struktur 1 | - | - | - | - | 1 | 5 | - | 10 | - | 10 | - | 10 |
| Kühlstoff Struktur 2 | - | - | - | - | - | - | 5 | - | 20 | - | 10 | - |

| ***Bewertung*** | | | | | | | | | | | | |
|---|---|---|---|---|---|---|---|---|---|---|---|---|
| Süß | 1 | 1 | 1 | 1 | 1 | 1 | 1 | 1 | 1 | 1 | 1 | 1 |
| Heuartig-minzig | 1 | 1 | 1 | 1 | 1 | 1 | 1 | 1 | 1 | 1 | 1 | 1 |
| Scharf | 5 | 4 | 4 | 4 | 3 | 3 | 3 | 3 | 2 | 2 | 2 | 2 |
| Stechend | 3 | 3 | 3 | 3 | 2 | 2 | 2 | 2 | 2 | 2 | 2 | 2 |
| Bitter | 3 | 3 | 3 | 3 | 2 | 2 | 2 | 2 | 2 | 2 | 2 | 2 |
| Metallisch | 9 | 5 | 5 | 5 | 3 | 3 | 3 | 3 | 2 | 2 | 2 | 2 |

In der sensorischen Beurteilung schnitten alle erfindungsgemäßen Formulierungen deutlich besser als die Vergleichsprodukte ab. Bei den untersuchten Aromen scharfe, stehende, bittere und teerartige Noten fast völlig maskiert, bei den Süßstoffen die Süße leicht gesteigert und insbesondere der metallische Nachgeschmack vermieden. Bei den pharmazeutischen Wirkstoffen wurde vor allem der metallisch-scharfe Nahgeschmack überdeckt. Dabei erwiesen sich Kombinationen aus den erfindungsgemäßen und weiteren Kühlstoffen als besonders wirksam.

### FORMULIERUNGBEISPIELE FÜR KOSMETISCHE ZUBEREITUNGEN

Die folgenden Formulierungsbeispiele **F1 to F55** zeigen unterschiedlichste Formulierungen für Kosmetische und pharmazeutische Zubereitungen. Kühlstoff 1 bedeutet hier der erfindungsgemäße Kühlstoff gemäß Formel (I).

**Tabelle F1**

| Transparente Flüssigseife (Mengen in Gew.-%) | | |
|---|---|---|
| **Bestandteile** | **INCI Name** | **Menge** |
| Tagat O 2 | PEG-20 Glyceryl Oleate | 2.5 |
| Coconut oil diethanolamine condensate | Cocamide DEA | 5.0 |
| Abil B 8842 | Cyclomethicone | 0.5 |
| Sodium laurylethersulfate. 28% | Sodium Laureth Sulfate | 35.0 |
| Tego-Betaine L7 | Cocamidopropyl Betaine | 5.0 |
| Soap. 25% | Coconut acid. Potassium salt. Potassium Oleate | 20.0 |
| Hydrolite^{®} 5 | 1,2-propanediol | 0.4 |
| Kühlstoff 1 | | 1.0 |
| Wasser | Wasser | Ad 100 |

**Tabelle F2**

| Flüssige Syndetseife (Mengen in Gew.-%) | | |
|---|---|---|
| **Bestandteile** | **INCI Name** | **Menge** |
| Elfan OS 46 | Sodium Olefin C14-C16 Sulfonate | 35.5 |
| Armoteric LB | Lauryl Betaine | 8.0 |
| Elfan SG | | 10.0 |
| Elfacos GT 282 L | Talloweth-60 Myristyl Glycol | 3.0 |
| PCL-Liquid 100 | Cetearyl Ethylhexanoate | 4.0 |
| Hydrolite^{®} 5 Green | Hydrolite^{®} 5 Green | 0.4 |
| Preservative | Methylchloroisothiazolinone | 0.1 |
| Kühlstoff 1 | | 1.5 |
| Wasser | Wasser | Ad 100 |

**Tabelle F3**

| Körperpflegelotion (Mengen in Gew.-%) | | |
|---|---|---|
| **Bestandteile** | **INCI Name** | **Menge** |
| Lumerol K 28 | Disodium Laureth Sulfosuccinate. Co-camidopropyl Betaine. Magnesium Lauryl Sulfate | 33.0 |
| Amphotensid B 4 | Cocamidopropyl Betaine | 10.0 |
| Perlglanzmittel GM 4055 | MIPA-Pareth-25 Sulfate. Glycol Stearate | 4.0 |
| Sodium Chloride | Sodium Chloride | 2.0 |
| Avocado oil | Persea Gratissima (Avocado) Oil | 3.0 |
| Wasser | Wasser | Ad 100 |
| Hydrolite^{®} 5 | Hydrolite^{®} 5 Green | 0.5 |
| Euxyl^{®} K727 | Phenoxyethanol. Methyldibromo Glutaronitrile. Isothiazolinones | 0.3 |
| Kühlstoff 1 | | 1.0 |

**Tabelle F4**

| Körperpflegelotion mit Triclosan (Mengen in Gew.-%) | | |
|---|---|---|
| **Bestandteile** | **INCI Name** | **Menge** |
| Texapon N 25 | Sodium Laureth Sulfate | 37.5 |
| Lamepon S | Potassium Cocoyl Hydrolyzed Collagen | 28.0 |
| Lamesoft LMG | Hydrogenated Tallow Glycerides. TEA-Cocoyl Hydrolyzed Collagen | 5.0 |
| Lamesoft 156 | Glyceryl Laurate. TEA-Cocoyl Hydrolyzed Collagen | 5.0 |
| Sodium Chloride | Sodium Chloride | 1.7 |
| Irgasan DP 300 | Triclosan | 0.5 |
| Wasser | Wasser | Ad 100 |
| Hydrolite^{®} 5 | Hydrolite^{®} 5 Green | 0.3 |
| Euxyl^{®} K703 | Phenoxyethanol. Benzoic Acid. Dehy-droacetic Acid | 0.4 |
| Kühlstoff 1 | | 1.0 |

**Tabelle F5**

| Intimwäsche (Mengen in Gew.-%) | | |
|---|---|---|
| **Bestandteile** | **INCI Name** | **Menge** |
| Tegobetaine HS | Cocamidopropyl Betaine. Glyceryl Laurate | 15.0 |
| Tagat L 2 | PEG-20 Glyceryl Laurate | 2.0 |
| Arlacide G | Chlorhexidine Digluconate | 0.1 |
| Rewoquat B 50 | Benzalkonium Chloride | 0.1 |
| Lactic Acid. 80% | Lactic Acid | 0.1 |
| Wasser | Wasser | Ad 100 |
| Hydrolite^{®} 5 | Hydrolite^{®} 5 Green | 0.2 |
| Euxyl^{®} K700 | Potassium Sorbate. Benzyl Alcohol. Phenoxyethanol | 0.3 |
| Kühlstoff 2 | | 1.3 |

**Tabelle F6**

| Flüssigseife (Mengen in Gew.-%) | | |
|---|---|---|
| **Ingredient** | **INCI** | **Menge** |
| Deionisiertes Wasser | Wasser | 2.0 |
| Soap bases mix | Sodium tallowates / palmitates | 95.8 |
| Titanium dioxide | Titanium dioxide | 1.0 |
| Hydrolite^{®} 5 | Hydrolite^{®} 5 Green | 1.2 |
| Preservatives | Phenoxyethanol | 0.5 |
| Kühlstoff 1 | | 1.0 |

**Tabelle F7**

| Shampoo (Mengen in Gew.-%) | |
|---|---|
| **Bestandteile** | **Menge** |
| Sodium lauryl ether sulfate (e.g. Texapon NSO) | 12 |
| Cocamidopropyl betaine (e.g. Dehyton K) | 2 |
| Sodium chloride | 1.4 |
| Citric acid | 1.3 |
| 1,2-pentanediol (Hydrolite^{®} 5) | 0.3 |
| Phenoxyethanol. methyl-. ethyl-. butyl- and propylparaben | 0.5 |
| Kühlstoff 4 | 1.0 |
| Wasser | Ad 100 |

**Tabelle F8**

| 2-in-1 Shampoo (Mengen in Gew.-%) | | |
|---|---|---|
| **Bestandteile** | **INCI Name** | **Menge** |
| Deionisiertes Wasser | Wasser | Ad 100 |
| Plantacare PS 10 | Sodium Laureth Sulfate. Lauryl Glucoside | 20.0 |
| Euperlan PK 771 | Glycol Distearate. Sodium Lauryl Sulfate. Cocamide MEA. Laureth-10 | 6.0 |
| Sodium chloride | Sodium Chloride | 1.4 |
| Citric acid monohydrate crystalline | Citric acid | 0.1 |
| Hydrolite^{®} 5 | Hydrolite^{®} 5 Green | 0.5 |
| Dragocid Liquid | Phenoxyethanol, Parabens | 0.5 |
| Kühlstoff 1 | | 1.0 |

**Tabelle F9**

| Anti-Schuppen Shampoo (Mengen in Gew.-%) | |
|---|---|
| **Bestandteile** | **Menge** |
| Climbazole | 0.50 |
| Sodium Laureth Sulfate | 37.00 |
| Cocamidopropyl Betaine | 8.00 |
| PEG-6 Caprylic/Capric Glycerides | 2.50 |
| Laureth-2 | 2.00 |
| Wasser (Aqua). Glycerol. Thymus Vulgaris (Thyme). Flower/Leaf Extract | 0.50 |
| Rosmarinus Officinalis (Rosemary) Leaf Wasser. Wasser (Aqua). Butylene Glycol. Pentylene Glycol | 0.50 |
| Bisabolol | 0.10 |
| Panthenol | 0.50 |
| Polyquaternium-10 | 0.40 |
| 1,2-pentanediol (Hydrolite^{®} 5) | 0.50 |
| Phenoxyethanol. Methylparaben. Ethylparaben. Butylparaben. Propylparaben. Isobutylparaben | 0.70 |
| Kühlstoff 5 | 1.50 |
| Wasser (Aqua) | Ad 100 |

**Tabelle F10**

| Hair conditioner mit Crinipan. rinse-off (Mengen in Gew.-%) | | |
|---|---|---|
| **Bestandteile** | **INCI Name** | **Menge** |
| Lanette^{®} O | Cetearyl Alcohol | 4.00 |
| Dragoxat 89 | Ethylhexyl Isononanoate | 2.00 |
| Genamin^{®} KDM-P | Behentrimonium Chloride | 1.00 |
| SF 1550 | Phenyl Trimethicone | 0.10 |
| Neo Heliopan^{®} BB | Benzophenone-3 | 0.10 |
| Crinipan^{®} AD | Climbazole | 0.80 |
| Glycerol 99.5 P. | Glycerol | 6.00 |
| Wasser | Wasser (Aqua) | Ad 100 |
| Actipone^{®} Alpha Pulp | Wasser (Aqua). Butylene Glycol. Malic Acid. Actinidia Chinensis (Kiwi)Fruit Juice. Citrus. Aurantium Dulcis (Orange). Juice. Citrus Paradisi (Grapefruit) Juice. Pyrus Malus (Apple) Juice. Trideceth-9. PrunusAmygdalus Dulcis (Sweet Almond) Seed Extract | 0.50 |
| Extrapone^{®} Bamboo P | Propylene Glycol. Wasser (Aqua). Butylene Glycol. Bambusa Vulgaris Shoot Extract | 0.50 |
| Sodium Hydroxide 10% solution | Sodium Hydroxide | 0.40 |
| Colour I | Colour | 0.60 |
| Colour II | Colour | 0.30 |
| Hydrolite^{®} 5 | Hydrolite^{®} 5 Green | 0.40 |
| Preservative | Methylparaben | 0.20 |
| Kühlstoff 6 | | 1.2 |

**Tabelle F11**

| Sprühbarer Hair Conditioner mit Zinkpyrithrione - leave-on (Mengen in Gew.-%) | | |
|---|---|---|
| **Bestandteile** | **INCI Name** | **Menge** |
| Monomuls 60-35 C | Hydrogenated Palm Glycerides | 1.70 |
| Cetiol OE | Dicaprylyl Ether | 7.20 |
| Abil 100 | Dimethicone | 3.60 |
| Dehyquart F75 | Distearoylethyl Hydroxyethylmonium. Methosulfate. Cetearyl Alcohol | 4.00 |
| Eumulgin B1 | Ceteareth-12 | 3.50 |
| Cetiol S | Diethylhexylcyclohe xane | 7.20 |
| D-Panthenol | Panthenol | 0.10 |
| Glycerol 99.5 P. | Glycerol | 1.50 |
| Wasser | Wasser (Aqua) | Ad 100 |
| Actipone^{®} Rosemary | Wasser (Aqua). Propylene. Glycol. Rosmarinus Officinalis. (Rosemary) Leaf Extract | 0.10 |
| Frescolat^{®} ML Cryst. | Menthyl Lactate | 0.50 |
| Dragosantol100 | Bisabolol | 0.10 |
| Zinc Omadine | Zinc pyrithione | 0.10 |
| Hydrolite^{®} 5 | Hydrolite^{®} 5 | 0.40 |
| Phenonip^{®} | phenoxyethanol. methylparaben. ethylparaben. butylparaben. propylparaben. isobutylparaben | 0.30 |
| Kühlstoff 1 | | 1.50 |

**Tabelle F12**

| Hair Conditioner mit UV-Schutz (Mengen in Gew.-%) | | |
|---|---|---|
| **Bestandteile** | **I NCI Name** | **Menge** |
| Renex PEG 6000 | PEG-150 | 2.50 |
| Hair Conditioner Base | Cetyl alcohol. behentrimonium chloride. Triticum Vulgare (Wheat) bran extract. linoleic acid | 3.00 |
| PCL-Solid | Stearyl heptanoate. stearyl caprylate | 0.50 |
| Dow Corning 5200 | Laurylmethicone copolyol | 0.50 |
| Natrosol 250 HR | Hydroxyethylcellulose | 0.50 |
| Benzophenone-4 | Benzophenone-4 | 1.00 |
| Neo Heliopan AP | Disodiumphenyldibenz-imidazole tetrasulphonate | 1.00 |
| Amino methyl propanol | Amino methyl propanol | 2.00 |
| Dow Corning 949 cationic emulsion | Amodimethicone. cetrimonium chloride. trideceth-12 | 2.00 |
| Hydrolite^{®} 5 | Hydrolite^{®} 5 | 0.80 |
| 1.2-hexanediol | 1.2-hexanediol | 0.50 |
| Kühlstoff 3 | | 1.50 |
| Wasser | Wasser (Aqua) | Ad 100 |

**Tabelle 13**

| Shower gel (Mengen in Gew.-%) | | |
|---|---|---|
| **Bestandteile** | **INCI** | **Menge** |
| Deionisiertes Wasser | Wasser | Ad 100 |
| Plantacare PS 10 | Sodium Laureth Sulfate. Lauryl Glucoside | 20.0 |
| Sodium chloride | Sodium Chloride | 1.4 |
| Citric acid monohydrate crystalline | Citric Acid | 1.3 |
| Hydrolite^{®} 5 | Hydrolite^{®} 5 | 0.6 |
| SymDiol^{®}68 | 1.2-hexanediol. Caprylyl glycol | 0.4 |
| Kühlstoff 2 | | 0.9 |

**Tabelle F14**

| | |
|---|---|
| Rasierschaum (Mengen in Gew.-%) | |

| **Bestandteile** | **Menge** |
|---|---|
| Dem. Wasser | 77.2 |
| Triethanolamine | 4.0 |
| Edenor L2 SM (Stearinic acid. Palmitinic acid) (Cognis) | 5.3 |
| Laureth-23 | 3.0 |
| Stearylalcohol | 0.5 |
| Sodium lauryl sulfate | 3.0 |
| Extrapone Seaweed (Wasser. propylene glycol. potassium iodide. Fucus Vesiculosus Extract) | 1.0 |
| Dragosantol (Bisabolol. Farnesol) | 0.1 |
| 1,2-pentanediol (Hydrolite^{®} 5) | 1.0 |
| Euxyl^{®} K220 (Methylisothiazolinone. Ethylhexylglyerol) | 0.6 |
| Kühlstoff 4 | 0.6 |
| Propane. butane 4.2 Bar | 4.0 |

**Tabelle F15**

| Enthaarungscreme (Mengen in Gew.-%) | |
|---|---|
| **Bestandteile** | **Menge** |
| Cetearyl alcohol | 10.0 |
| Ceteareth-12 | 2.0 |
| PCL-Liquid (Cetearylethylhexanoate. Isopropylmyristate) | 3.0 |
| Dragosantol (Bisabolol. Farnesol) | 0.1 |
| Edenor L2 SM (Stearinic acid. Palmitinic acid) | 1.0 |
| Dem. Wasser | 52.2 |
| Urea | 5.0 |
| Dem. Wasser | 10.0 |
| Calcium thioglycolate | 6.0 |
| Sodium hydroxide solution. 10 % | 10.0 |
| 1,2-pentanediol (Hydrolite^{®} 5) | 0.5 |
| Neo Dragocid Powder (Methyl parabene. sorbinic acid. Dehydro acetic acid. Propyl parabene) | 0.2 |
| Kühlstoff3 | 2.0 |

**Tabelle F16**

| After Shave Tonic (Mengen in Gew.-%) | | |
|---|---|---|
| **Bestandteile** | **INCI** | **Menge** |
| SymSol^{®} PF-3 | Wasser (Aqua). Pentylene Glycol. Sodium Lauryl Sul-foacetate. SodiumOleoyl Sarcosinate. Sodium Chloride. Disodium Sulfoacetate. SodiumOleate. Sodium Sulfate | 3.00 |
| SymSitive^{®} 1609 | Pentylene Glycol. 4-t-Butylcyclohexan ol | 1.00 |
| Frescolat^{®} ML | Menthyl Lactate | 0.30 |
| Glycerol 99.5 P. | Glycerol | 5.00 |
| Wasser | Wasser (Aqua) | Ad 100 |
| Extrapone^{®} Glacier Wasser GW | Glycerol. Wasser (Aqua) | 1.00 |
| SymCalmin^{®} | Butylene Glycol. Pentylene Glycol. Hydroxyphenyl Propamidobenzoic A cid | 0.50 |
| Dragosine^{®} | Carnosine | 0.10 |
| Hydrolite^{®} 5 | Pentylene Glycol | 5.00 |
| Ethanol 96 % | Alcohol Denat. | 5.00 |
| Colour Pigment | Colour Pigment | 0.05 |
| Hydrolite^{®} 5 | 1,2-pentanediol | 0.15 |
| Kühlstoff 1 | | 1.00 |

**Tabelle F17**

| Deodorant Formulierung als Roll-on Gel (Mengen in Gew.-%) | |
|---|---|
| **Bestandteile** | **Menge** |
| 1.3-butylene glycol | 2.00 |
| PEG-40-hydrogenated castor oil | 2.00 |
| Hydroxyethylcellulose | 0.50 |
| 1,2-pentanediol (Hydrolite^{®} 5) | 0.30 |
| 1.3-propanediol | 0.50 |
| 3-phenylpropanol | 0.40 |
| Ethylhexyl glycerin | 0.10 |
| Kühlstoff 1 | 0.50 |
| Wasser | ad 100.00 |

**Tabelle F18**

| Klares Deodorant als roll-on (Mengen in Gew.-%) | | |
|---|---|---|
| **Bestandteile** | **INCI** | **Menge** |
| Methocel E4M Premium | Hydroxypropyl Methylcellulose | 0.50 |
| Wasser | Wasser (Aqua) | Ad 100 |
| Neo-PCL Wasser Soluble N | (Aqua) Trideceth-9. PEG-5 Ethylhexanoate. Wasser | 1.00 |
| Solubilizer | PEG-40 Hydrogenated Castor Oil. Trideceth-9. Propylene Glycol. Wasser (Aqua) | 3.00 |
| Deolite | Dimethyl Phenylpropanol. Pentylene Glycol | 0.50 |
| Locron LW | Aluminium Chlorohydrate | 25.00 |
| Aloe Vera Gel Concentrate 10/1 | Aloe Barbadensis Leaf Juice | 1.00 |
| Propylene Glycol -1.2 99 P GC | Propylene Glycol | 4.00 |
| Ethanol 96 % | Alcohol Denat. | 30.00 |
| Hydrolite^{®} 5 | 1,2pentanediol | 1.00 |
| Kühlstoff 1 | | 1.25 |

**Tabelle F19**

| Deodorant stick (Mengen in Gew.-%) | |
|---|---|
| **Bestandteile** | **Menge** |
| Sodium stearate | 8.00 |
| PPG-3 Myristyl ether | 70.00 |
| 1.2-propylene glycol | 10.00 |
| 1.1-dimethyl-3-phenylpropanol | 0.20 |
| 2-butyloctanoic acid | 0.20 |
| 1,2-pentanediol (Hydrolite^{®} 5) | 0.60 |
| Heptoxy-1.2-propanediol | 0.20 |
| Phenoxyethanol | 0.30 |
| Kühlstoff 1 | 1.50 |
| Wasser | Ad 100 |

**Tabelle F20**

| Antiperspirantstift (Mengen in Gew.-%) | | |
|---|---|---|
| **Bestandteile** | **INCI** | **Menge** |
| PCL Liquid 100 | Cetearyl ethylhexanonate | to 100 |
| Silicone Fluid 345 | Cyclomethicone | 10.00 |
| CRODACOL C90 | Cetyl Alcohol | 8.00 |
| SYNCROWAX HGLC | C18-36 Triglyceride | 8.00 |
| CRODAMOL PTC | Pentaerythritol Tetracaprylate/Caprate | 5.00 |
| SYNCROWAX HRC | Tribehenin | 4.00 |
| VOLPO N5 | Oleth-5 | 1.00 |
| Titanium Dioxide | | 1.00 |
| Rezal 36GP | Aluminium Tetrachlorohydrex GLY | 20.00 |
| Dry Flo C | Aluminium Starch Octenyl Succinate | 22.50 |
| Hydrolite^{®} 5 | 1,2-pentanediol | 0.60 |
| Preservative | Phenoxyethanol | 0.40 |
| Hexoxy-1.2-propanediol | | 0.10 |
| Kühlstoff 1 | | 1.40 |

**Tabelle F21**

| Pumpspray (Mengen in Gew.-%) | | |
|---|---|---|
| **Bestandteile** | **INCI** | **Menge** |
| SymClariol^{®} | Decylene Glycol | 0.50 |
| Solubilizer | PEG-40 Hydrogenated Castor Oil. Trideceth-9. Propylene Glycol. Wasser (Aqua) | 4.00 |
| Neo-PCL Wasser Soluble N | Trideceth-9. PEG-5 Ethylhexanoate. Aqua | 1.50 |
| SymRelief^{®} | Bisabolol. Zingiber Officinale (Ginger) Root Extract | 0.10 |
| Wasser | Wasser (Aqua) | Ad 100 |
| 1.2 Propylene Glycol | Propylene Glycol | 6.00 |
| Hydrolite^{®} 5 | 1,2-pentanediol | 0.40 |
| SymDiol^{®} 68 | 1.2-Hexanediol. Caprylyl Glycol | 0.20 |
| Kühlstoff 2 | | 1.40 |

**Tabelle F22**

| Antiperspirant (Mengen in Gew.-%) | |
|---|---|
| **Bestandteile** | **Mengen** |
| Reach AZP-908 SUF | 24.00 |
| Cyclomethicone (Pentamer) | Ad 100 |
| Polydecene (Silkflo 364 NF) | 17.50 |
| Neo Heliopan OS (ethylhexyl salicylate) | 2.50 |
| L-Menthyl lactate (Frescolate ML) | 0.25 |
| Polyethylene | 3.00 |
| Hydrogenated castor oil | 2.00 |
| Promyristyl PM-3 | 7.00 |
| PEG-8 Distearate | 3.00 |
| Silicon dioxide (Cab-O-Sil M-5) | 1.00 |
| Stearyl alcohol | 15.00 |
| Octyldodecanol | 0.10 |
| 1,2-pentanediol (Hydrolite^{®} 5) | 0.80 |
| 3-Phenylpropanol | 0.40 |
| Kühlstoff 3 | 1.60 |

**Tabelle F23**

| Sprühdeodorant mit Triclosan (Mengen in Gew.-%) | |
|---|---|
| **Bestandteile** | **Menge** |
| PEG-40-hydrogenated castor oil | 3.00 |
| Ethylhexylglycerol (Octoxyglycerol) | 0.80 |
| Ethanol | 40.00 |
| Citrate buffer | 0.50 |
| Triclosan^{®} (5-chloro-2-(2.4-dichlorophenoxy)phenol) | 0.25 |
| 1,2-pentanediol (Hydrolite^{®} 5) | 0.75 |
| Phenoxyethanol | 0.40 |
| Kühlstoff 1 | 1.40 |
| Wasser | Ad 100 |

**Tabelle F24**

| O/W Lotion (Mengen in Gew.-%) | |
|---|---|
| **Bestandteile** | **Menge** |
| Paraffin oil | 5.00 |
| Isopropyl palmitate | 5.00 |
| Cetyl alcohol | 2.00 |
| Beeswax | 2.00 |
| Ceteareth-20 | 2.00 |
| PEG-20-glyceryl stearate | 1.50 |
| Glycerol | 3.00 |
| 1,2-pentanediol (Hydrolite^{®} 5) | 0.30 |
| Methylparaben | 0.30 |
| Kühlstoff 3 | 1.00 |
| Wasser | ad 100.00 |

**Tabelle F25**

| Body Lotion (Mengen in Gew.-%) | |
|---|---|
| **Bestandteile** | **Menge** |
| Cetearyl Alcohol | 2.00 |
| Ethylhexyl Isononanoate | 5.00 |
| Cetearyl Ethylhexanoate. Isopropyl Myristate | 3.00 |
| Glyceryl Oleate Citrate. Caprylic/Capric Triglyceride | 4.00 |
| Wasser (Aqua) | 79.50 |
| Carbomer | 0.30 |
| Sodium Benzoate | 0.100 |
| Propylene Glycol | 5.00 |
| Sodium Hydroxide 30% solution | 0.30 |
| 1,2-pentanediol (Hydrolite^{®} 5) | 0.30 |
| Triethylene Glycol. Imidazolidinyl Urea. Methylparaben. Propylparaben. Dehydro-acetic Acid | 0.30 |
| Kühlstoff 5 | 1.30 |

**Tabelle F26**

| Creme (Mengen in Gew.-%) | |
|---|---|
| **Bestandteile** | **Menge** |
| Paraffin oil | 10.00 |
| Ozokerite | 4.00 |
| Vaseline | 4.00 |
| VegeTabelle oil | 10.00 |
| Wool wax alcohol | 2.00 |
| Aluminum stearate | 0.40 |
| 1,2-pentanediol (Hydrolite^{®} 5) | 2.00 |
| Phenoxyethanol | 0.50 |
| Kühlstoff 1 | 1.50 |
| Wasser | ad 100.00 |

**Tabelle F27**

| Creme (Mengen in Gew.-%) | | |
|---|---|---|
| **Bestandteile** | **INCI** | **Menge** |
| Dracorin^{®} CE | Glyceryl Stearate Citrate | 1.00 |
| Lanette^{®} O | Cetearyl Alcohol | 2.00 |
| Cutina^{®} GMS-V | Glyceryl Stearate | 1.00 |
| Tegosoft^{®} MM | Myristyl Myristate | 1.00 |
| Xiameter^{®} PMX-0246. Cyclosiloxane | Cyclohexasiloxane (and) Cyclopentasiloxane | 0.50 |
| Dragoxat^{®} 89 | Ethylhexyl Isononanoate | 2.00 |
| PCL-Liquid 100 | Cetearyl Ethylhexanoate | 4.00 |
| Neutral Oil | Caprylic/Capric Triglyceride | 4.00 |
| Carbopol^{®} Ultrez 21 | Acrylates/C10-30 Alkyl Acrylate Crosspolymer | 0.20 |
| Keltrol^{®} CG-T | Xanthan Gum | 0.10 |
| Wasser | Wasser (Aqua) | Ad 100 |
| Glycerol 99.5 P. | Glycerol | 3.00 |
| Propylene Glycol -1.2 99 P GC | Propylene Glycol | 2.00 |
| Sodium Benzoate | Sodium Benzoate | 0.10 |
| Sodium Hydroxide 10% solution | Sodium Hydroxide | 0.50 |
| Hydrolite^{®} 5 | 1,2-pentanediol | 0.30 |
| Euxyl^{®} K702 | Dehydroacetic Acid. Benzoic Acid. Phenoxyetha-nol.Polyaminopropyl Biguanide. Ethylhexylglycerin | 0.30 |
| Kühlstoff 1 | | 2.00 |

**Tabelle F28**

| Hand- und Körpercreme (Mengen in Gew.-%) | | |
|---|---|---|
| **Bestandteile** | **INCI** | **Menge** |
| Dracorin^{®} GOC | Glyceryl Oleate Citrate. Caprylic/Capric Triglyceride | 2.00 |
| PCL-Solid | Stearyl Heptanoate. Stearyl Caprylate | 2.50 |
| Lanette^{®} O | Cetearyl Alcohol | 1.50 |
| Cutina^{®} GMS-V | Glyceryl Stearate | 1.00 |
| Dragoxat^{®} 89 | Ethylhexyl Isononanoate | 3.00 |
| PCL-Liquid 100 | Cetearyl Ethylhexanoate | 7.00 |
| Isodragol^{®} | Triisononanoin | 4.00 |
| Xiameter^{®} PMX-0345 Cyclosiloxane | Cyclopentasiloxane (and) Cyclohexasiloxane | 0.50 |
| Wasser | Wasser (Aqua) | Ad 100 |
| Carbopol^{®} Ultrez 21 | Acrylates/C10-30 Alkyl Acrylate Crosspolymer | 0.20 |
| Keltrol^{®} CG-RD | Xanthan Gum | 0.10 |
| Glycerol 85 P. | Glycerol | 3.00 |
| DragoBetaGlucan | Wasser (Aqua). Butylene Glycol. Glycerol. Avena Sativa (Oat) Kernel Extract | 1.50 |
| Potassium Sorbat | Potassium Sorbate | 0.10 |
| Sodium Hydroxide 10% solution | Sodium Hydroxide | 0.50 |
| Hydrolite^{®} 5 | 1,2-pentanediol | 0.20 |
| Euxyl^{®} K300 | Methyl-. Butyl-. Ethyl-. Propyl. Isobutylparaben. Phenoxyethanol. | 0.30 |
| Kühlstoff 4 | | 1.00 |

**Tabelle F29**

| Gesichtscreme (Mengen in Gew.-%) | | |
|---|---|---|
| **Bestandteile** | **INCI** | **Menge** |
| Emulsiphos^{®} | Potassium Cetyl Phosphate. Hydrogenated Palm Glycerides | 1.50 |
| Cutina^{®} GMS-V | Glyceryl Stearate | 1.70 |
| Lanette^{®} O | Cetearyl Alcohol | 3.00 |
| Tegosoft^{®} MM | Myristyl Myristate | 1.00 |
| PCL-Liquid 100 | Cetearyl Ethylhexanoate | 1.00 |
| Isodragol^{®} | Triisononanoin | 3.00 |
| Dragoxat^{®} 89 | Ethylhexyl Isononanoate | 4.00 |
| Avocado Oil | Persea Gratissima (Avocado) Oil | 3.00 |
| Abil^{®} 350 | Dimethicone | 0.50 |
| Covi-ox^{®} T-70 | Tocopherol | 0.10 |
| Edeta^{®} BD | Disodium EDTA | 0.10 |
| Carbopol^{®} Ultrez 21 | Acrylates/C10-30 Alkyl Acrylate Crosspolymer | 0.30 |
| Keltrol^{®} CG-RD | Xanthan Gum | 0.150 |
| Wasser | Wasser (Aqua) | Ad 100 |
| Glycerol 99.5 P. | Glycerol | 4.00 |
| Propylene Glycol -1.2 99 P GC | Propylene Glycol | 3.00 |
| SymMatrix^{®} | Maltodextrin. Rubus Fruticosus (Blackberry) Leaf Extract | 0.50 |
| Sodium Hydroxide 10% solution | Sodium Hydroxide | 0.50 |
| Hydrolite^{®} 5 | 1,2-pentanediol | 0.30 |
| Euxyl^{®} K712 | Sodium Benzoate. Potassium Sorbate | 0.20 |
| Kühlstoff 1 | | 1.00 |

**Tabelle F30**

| Feuchtigkeitscreme (Mengen in Gew.-%) | |
|---|---|
| **Ingredient** | **Menge** |
| PEG-7 hydrogenated castor oil | 6.00 |
| Cetearyl ethyl hexanoate | 10.00 |
| Isopropyl myristate | 5.00 |
| Mineral oil | 7.00 |
| Shea Butter (*Butyrospermum parkii*) | 0.50 |
| Aluminum stearate | 0.50 |
| Magnesium stearate | 0.50 |
| Bisabolol | 0.20 |
| Quaternium-18-Hectorit | 0.70 |
| Dipropylene glycol | 5.0 |
| Magnesium sulfate | 0.70 |
| 1,2-pentanediol (Hydrolite^{®} 5) | 4.00 |
| Preservative (Phenoxyethanol) | 0.20 |
| Kühlstoff 1 | 1.00 |
| Aqua dem. | 58.90 |

**Tabelle F31**

| Antifaltencreme (Mengen in Gew.-%) | |
|---|---|
| **Bestandteile** | **Menge** |
| Glyceryl Stearate Citrate | 1.00 |
| Glyceryl Laurate | 1.00 |
| Cetearyl Alcohol (and) Myristyl Myristate | 3.00 |
| Cetearyl Ethylhexanoate | 4.00 |
| Mineral oil | 4.00 |
| Cyclohexasiloxane | 0.50 |
| Acrylates/C10-30 Alkyl Acrylate Crosspolymer | 0.20 |
| Wasser | Ad 100 |
| 1.2-Hexanediol | 2.00 |
| Sodium Hydroxide 10% solution | 0.10 |
| Narcissus Tazetta Bulb Extract | 1.00 |
| 1,2-pentanediol (Hydrolite^{®} 5) | 0.30 |
| Preservative (Phenoxyethanol) | 0.50 |
| Kühlstoff 3 | 1.50 |

**Tabelle F32**

| Desinfizierendes Hautpflegeöl (Mengen in Gew.-%) | | |
|---|---|---|
| **Bestandteile** | **INCI** | **Menge** |
| Neutral Oil | Caprylic/Capric Triglyceride | Ad 100 |
| Sweet Almond Oil | Prunus Dulcis | 20.00 |
| Dragoxat^{®} 89 | Ethylhexyl Isononanoate | 4.00 |
| Isopropyl Palmitate | Isopropyl Palmitate | 6.00 |
| PCL-Liquid 100 | Cetearyl Ethylhexanoate | 15.00 |
| Dragosantol^{®} 100 | Bisabolol | 0.20 |
| Retinyl Acetate In Oil (1 Mio. le/G) | Retinyl Acetate | 0.50 |
| Vitamin E Acetate | Tocopheryl Acetate | 0.50 |
| Covi-ox^{®} T-70 | Tocopherol | 0.10 |
| Hydrolite^{®} 5 | 1,2-pentanediol | 0.30 |
| Preservative | Methyl-. Butyl-. Ethyl-. Propylparaben | 0.30 |
| Kühlstoff 1 | | 1.00 |

**Tabelle F33**

| Antiseptische Wundcreme (Mengen in Gew.-%) | |
|---|---|
| **Bestandteile (INCI)** | **Menge** |
| Sorbitan Isostearate. Hydrogenated Castor Oil. Ceresin. Beeswax (Cera Alba) | 6.00 |
| Petrolatum | 21.00 |
| Cera Alba | 5.00 |
| Cetearyl Alcohol | 7.00 |
| Prunus Dulcis | 7.00 |
| Lanolin | 5.00 |
| Paraffinum Liquidum | 12.00 |
| Wasser (Aqua) | Ad 100 |
| Panthenol | 7.00 |
| Magnesium Sulfate | 0.70 |
| 1,2-pentanediol (Hydrolite^{®} 5) | 1.00 |
| Tocopheryl Acetate | 1.00 |
| Octenidine dihydrochloride | 0.10 |
| Phenoxyethanol | 0.50 |
| Kühlstoff 6 | 1.50 |

**Tabelle F34**

| Feuchtigkeitsmaske (Mengen in Gew.-%) | | |
|---|---|---|
| **Bestandteile** | **INCI** | **Menge** |
| Wasser | Wasser (Aqua) | Ad 100 |
| Stabileze QM | PVM / Ma Decadiene Crosspolymer | 0.50 |
| Biotive^{®} L-Arginine | Arginine | 0.75 |
| Actipone^{®} Laminaria Saccharina GW | Glycerol. Wasser (Aqua). Laminaria Saccharina Extract | 1.00 |
| Extrapone^{®} Cucumber | Wasser (Aqua). Propylene Glycol. Cucumis Sativus (Cucumber) Juice | 1.00 |
| Glycerol 99.5 P. | Glycerol | 7.00 |
| Neo Actipone^{®} Soap Nutshell | Sapindus Mukurossi Peel Extract | 0.50 |
| Colour I | Colour | 0.01 |
| Hydrolite^{®} 5 | Pentylene Glycol | 5.00 |
| Solubilizer | PEG-40 Hydrogenated Castor Oil. Trideceth-9. Wasser (Aqua) | 0.60 |
| Hydrolite^{®} 5 | 1,2-pentanediol | 0.08 |
| Preservative | Phenoxyethanol | 0.40 |
| Kühlstoff 1 | | 1.00 |

**Tabelle F35**

| Sprühbares Desinfektionsgel (Mengen in Gew.-%) | | |
|---|---|---|
| **Bestandteile** | **INCI** | **Menge** |
| Wasser | Wasser (Aqua) | Ad 100 |
| Stabileze QM | PVM / Ma Decadiene Crosspolymer | 0.25 |
| Sodium Hydroxide 10% solution | Sodium Hydroxide | 0.40 |
| Coffein pure | Caffeine | 0.50 |
| Extrapone^{®} Horse Chestnut | Propylene Glycol. Wasser (Aqua). Glucose. Aesculus Hippocastanum (Horse Chestnut) Seed Extract. Lactic Acid | 1.00 |
| Hydrolite^{®} 5 | 1,2-pentanediol | 3.00 |
| 1.3 Butylene Glycol | Butylene Glycol | 5.00 |
| Biotive^{®} Esculin Sesquihydrate | Esculin | 0.30 |
| Ethanol 96 % | Alcohol Denat. | 10.00 |
| Solubilizer | PEG-40 Hydrogenated Castor Oil. Trideceth-9. Wasser (Aqua) | 0.50 |
| Octenidine dihydrochloride | | 0.10 |
| Preservative | Phenoxyethanol | 0.70 |
| Kühlstoff 1 | | 1.00 |

**Tabelle F36**

| Mineralisches Wasch- und Reinigungsgel (Mengen in Gew.-%) | | |
|---|---|---|
| **Bestandteile** | **INCI** | **Menge** |
| Wasser | Wasser (Aqua) | Ad 100 |
| Pionier^{®} NP 37 G | Sodium Carbomer | 1.50 |
| SymSol^{®} PF-3 | Wasser (Aqua). Pentylene Glycol. Sodium Lau-ryl Sulfoacetate. SodiumOleoyl Sarcosinate. Sodium Chloride. Disodium Sulfoacetate. SodiumOleate. Sodium Sulfate | 5.00 |
| Hydroviton^{®} 24 | Wasser (Aqua). Pentylene Glycol. Glycerol. Sodium Lactate. Lactic Acid. Serine. Urea. Sorbitol. Sodium Chloride. Allantoin | 1.00 |
| Extrapone^{®} Silk GW | Wasser (Aqua). Glycerol. Hydrolyzed Silk | 1.00 |
| Hydrolite^{®} 5 Green | Hydrolite^{®} 5 Green | 4.00 |
| Actipearls Red Star # DH10402/6 | Wasser (Aqua). Propylene Glycol. Algin. Gellan Gum. Xanthan Gum. CalciumChloride. CI 12490 (Pigment Red 5). Mica (CI 77019). Titanium Dioxide (CI 77891) | 1.00 |
| 1,2-pentanediol (Hydrolite^{®} 5) | 1,2-pentanediol | 0.50 |
| 3-Phenylpropanol | | 0.70 |
| Kühlstoff 5 | | 0.30 |

**Tabelle F37**

| Anti-Akne Wäsche (Mengen in Gew.-%) | |
|---|---|
| **Bestandteile (INCI)** | **Menge** |
| Wasser (Aqua) | 45.70 |
| Polyquaternium-7 | 0.50 |
| Cocamidopropyl Betaine 9.000 | 9.00 |
| Coco Glucoside 2.000 | 2.00 |
| Polysorbate 80. Glycerol. Gossypium Herbaceum. (Cotton) Seed Oil. Wasser (Aqua) | 1.00 |
| Trideceth-9. PEG-5 Ethylhexanoate. Wasser (Aqua) | 1.00 |
| Glycereth-90 Isostearate. Laureth-2 | 0.50 |
| Sodium Laureth Sulfate 37.000 | 37.00 |
| Glycerol. Triticum Vulgare (Wheat) Gluten. Wasser (Aqua) | 1.00 |
| Sodium Chloride | 0.30 |
| 1,2-pentanediol (Hydrolite^{®} 5) | 1.00 |
| Phenoxyethanol. Methylparaben. Ethylparaben. Butylparaben. Propylparaben. Isobutylparaben | 0.30 |
| Kühlstoff 5 | 1.50 |

**Tabelle F38**

| Sonnenschutzformulierung (Mengen in Gew.-%) | |
|---|---|
| **Ingredient** | **Menge** |
| Ethylhexyl cinnamic acid | 7.50 |
| Benzophenon-3 | 2.00 |
| Polyglyceryl dimer soyate | 0.80 |
| Sorbitane stearate | 1.00 |
| Tocopheryl acetate | 0.50 |
| Glyceryl stearate. PEG-100 Stearate | 3.00 |
| PEG-40. hydrogenated castor oil | 1.00 |
| Titanium dioxide. aluminum oxide hydrate. Dimethicon/Methicon Copolymer | 3.00 |
| *Butyrospermum parkii* (Shea Butter) | 1.00 |
| C₁₂₋₁₅alkyl benzoate | 6.50 |
| Butylene glycol | 5.00 |
| Xanthan gum | 0.30 |
| Disodium EDTA | 0.10 |
| Allantoin | 0.10 |
| Polyacryl amide. C₁₃₋₁₄ isoparaffin. Laureth-7 | 1.00 |
| 1,2-pentanediol (Hydrolite^{®} 5) | 5.00 |
| 4-t Butylcyclohexanol | 1.00 |
| Preservatives (Methyl-. Butyl-. Ethyl-. Propylparaben. Phenoxyethanol) | 0.30 |
| Kühlstoff 3 | 2.00 |
| Aqua dem. | Ad 100 |

**Tabelle F39**

| Sonnenschutzspray (Mengen in Gew.-%) | | |
|---|---|---|
| **Bestandteile** | **INCI** | **Menge** |
| Wasser. demineralized | Wasser (aqua) | 69.50 |
| Glycerol | Glycerol | 4.00 |
| 1.3 butylene glycol | Butylene glycol | 5.00 |
| D-Panthenol | Panthenol | 0.50 |
| Lara Care A-200 | Galactoarabinan | 0.25 |
| Baysilone oil M 10 | Dimethicone | 1.00 |
| Edeta BD | Disodium EDTA | 0.10 |
| Copherol 1250 | Tocopheryl acetate | 0.50 |
| Cetiol OE | Dicaprylyl ether | 3.00 |
| Neo Heliopan^{®} HMS | Homosalate | 5.00 |
| Neo Heliopan^{®} AV | Ethylhexyl methoxycinnamate | 6.00 |
| Neo Heliopan^{®} 357 | Butyl methoxydibenzoylmethane | 1.00 |
| Corapan TQ | Diethylhexylnaphthalate | 2.00 |
| Alpha Bisabolol | Bisabolol | 0.10 |
| Pemulen TR-2 | Acrylates/C10-30 alkyl acrylate crosspolymer | 0.25 |
| NaOH. 10% | Sodium hydroxide | 0.60 |
| Hydrolite^{®} 5 | 1,2-pentanediol | 0.20 |
| Phenoxyethanol | Phenoxyethanol | 0.40 |
| Solbrol M | Methylparaben | 0.10 |
| Solbrol P | Propylparaben | 0.10 |
| Kühlstoff 1 | | 1.00 |

**Tabelle F40**

| Sonnenschutzspray O/W. SPE 15-20 (Mengen in Gew.-%) | | |
|---|---|---|
| **Bestandteile** | **INCI** | **Menge** |
| Dracorin^{®} GOC | Glyceryl Oleate Citrate. Caprylic/Capric Triglyceride | 2.00 |
| Corapan^{®} TQ | Diethylhexyl 2.6-Naphthalate | 3.00 |
| Neo Heliopan^{®} HMS | Homosalate | 7.00 |
| Neo Heliopan^{®} OS | Ethylhexyl Salicylate | 5.00 |
| Neo Heliopan^{®} 357 | Butyl Methoxydibenzoylmethane | 3.00 |
| Isoadipate | Diisopropyl Adipate | 6.00 |
| Baysilone^{®} Oil M10 | Dimethicone | 1.00 |
| Edeta^{®} BD | Disodium EDTA | 0.10 |
| Vitamin E Acetate | Tocopheryl Acetate | 0.50 |
| Dragosantol^{®} 100 | Bisabolol | 0.10 |
| Pemulen^{®} TR-2 | Acrylates/C10-30 Alkyl Acrylate Crosspolymer | 0.25 |
| Wasser | Wasser (Aqua) | Ad 100 |
| Glycerol 99.5 P. | Glycerol | 4.00 |
| Butylene Glycol | Butylene Glycol | 5.00 |
| Neo Heliopan^{®} Hydro (103089). used as 25% aq. solution | Phenylbenzimidazole Sulfonic Acid | 8.00 |
| Biotive^{®} L-Arginine | Arginine | 0.55 |
| Hydrolite^{®} 5 | 1,2-pentanediol | 0.40 |
| Sobrol M | Methylparaben | 0.30 |
| Kühlstoff 3 | | 1.00 |

**Tabelle F41**

| Sonnenschutzsoftcreme (W/O). SPF 40 (Mengen in Gew.-%) | | |
|---|---|---|
| **Bestandteile** | **INCI** | **Menge** |
| Dehymuls PGPH | Polyglyceryl-2 dipolyhydroxystearate | 5.00 |
| Copherol 1250 | Tocopheryl acetate | 0.50 |
| Permulgin 3220 | Ozocerite | 0.50 |
| Zinc stearate | Zinc stearate | 0.50 |
| Tegosoft TN | C12-15 Alkyl benzoate | 10.00 |
| Neo Heliopan^{®} E1000 | Isoamyl-p-methoxycinnamate | 2.00 |
| Neo Heliopan^{®} 303 | Octocrylene | 5.00 |
| Neo Heliopan^{®} MBC | 4-Methylbenzylidene camphor | 3.00 |
| Zinc oxide. neutral | Zinc oxide | 5.00 |
| Wasser. distilled | Wasser (aqua) | Add 100 |
| EDETA BD | Disodium EDTA | 0.10 |
| Glycerol | Glycerol | 4.00 |
| Magnesium sulfate | Magnesium sulfate | 0.50 |
| Hydrolite^{®} 5 | 1,2-pentanediol | 0.30 |
| Symdiol^{®} 68 | 1.2-Hexanediol. Caprylylglycol | 0.30 |
| Kühlstoff 1 | | 1.00 |

**Tabelle F42**

| Sonnenschutzmilch (W/O) (Mengen in Gew.-%) | | |
|---|---|---|
| **Bestandteile** | **INCI** | **Menge** |
| Dehymuls PGPH | Polyglyceryl-2 dipolyhydroxystearate | 3.00 |
| Beeswax 8100 | Beeswax | 1.00 |
| Monomuls 90-0-18 | Glyceryl oleate | 1.00 |
| Zinc stearate | Zinc stearate | 1.00 |
| Cetiol SN | Cetearyl isononanoate | 5.00 |
| Cetiol OE | Dicaprylyl ether | 5.00 |
| Tegosoft TN | C12-15 alkyl benzoate | 4.00 |
| Vitamin E | Tocopherol | 0.50 |
| Neo Heliopan^{®} OS | Ethylhexyl salicylate | 5.00 |
| Neo Heliopan^{®} AV | Ethylhexyl methoxycinnamate | 7.50 |
| Uvinul^{®} T150 | Ethylhexyl triazone | 1.50 |
| Wasser. distilled | Wasser (Aqua) | To 100 |
| Trilon BD | Disodium EDTA | 0.10 |
| Glycerol | Glycerol | 5.00 |
| Neo Heliopan^{®} AP 10% solution. neutralized with NaOH | Disodium phenyl dibenzimidazole tetrasulfonate | 15.00 |
| Hydrolite^{®} 5 | 1,2-pentanediol | 0.25 |
| Alpha bisabolol | Bisabolol | 0.10 |
| SymOcide^{®} PT | Phenoxyethanol. Tropolone | 0.25 |
| Kühlstoff 3 | | 1.00 |

**Tabelle F43**

| After Sun Gel (Mengen in Gew.-%) | | |
|---|---|---|
| **Bestandteile** | **INCI** | **Menge** |
| SymSol^{®} PF-3 | Wasser (Aqua). Pentylene Glycol. Sodium Lauryl Sulfoacetate. SodiumOleoyl Sarcosinate. Sodium Chloride. Disodium Sulfoacetate. SodiumOleate. Sodium Sulfate | 3.000 |
| Glycerol 99.5 P. | Glycerol | 5.000 |
| SymHelios^{®} 1031 | Benzylidene Dimethoxydimethylin danone | 0.100 |
| Wasser | Wasser (Aqua) | Ad 100 |
| Pemulen^{®} TR-2 | Acrylates/C10-30 Alkyl Acrylate Crosspolymer | 1.000 |
| D-Panthenol 75 W | Panthenol | 0.500 |
| SymFinity^{®} 1298 | Echinacea Purpurea Extract | 0.100 |
| Extrapone^{®} Pearl GW | Wasser (Aqua). Glycerol. Hydrolyzed Pearl. Xanthan Gum | 1.000 |
| Sodium Hydroxide 10% solution | Sodium Hydroxide | 2.500 |
| Ethanol 96 % | Alcohol Denat. | 15.000 |
| Hydrolite^{®} 5 | 1,2-pentanediol | 0.20 |
| SymOcide^{®} PS | Phenoxyethanol. 1.2-Hexanediol. Decyleneglycol | 0.50 |
| Kühlstoff 1 | | 1.00 |

**Tabelle F44**

| After Sun Lotion (Mengen in Gew.-%) | |
|---|---|
| **Bestandteile** | **Menge** |
| Acrylate/C10-30 alkylacrylate crosspolymer | 0.4 |
| Cetearylethyl hexanoate | 15.0 |
| Bisabolol | 0.2 |
| Tocopheryl acetate | 1.0 |
| Panthenol | 1.0 |
| Alcohol | 15.0 |
| Glycerol | 3.0 |
| 1.2-Hexanediol | 0.60 |
| Kühlstoff 3 | 1.50 |
| 1,2-pentanediol (Hydrolite^{®} 5) | 4.0 |
| Aqua dem. | Ad 100 |
| Triethanolamine | 0.2 |

**Tabelle F45**

| Hair Styling Gel (Mengen in Gew.-%) | |
|---|---|
| **Bestandteile** | **Menge** |
| Wasser | Ad 100 |
| PVM/MA Decadiene Crosspolymer | 0.60 |
| PVP | 3.00 |
| Isocetyl Stearate | 4.00 |
| Ethylhexyl Methoxycinnamate | 0.50 |
| Aminomethyl Propanol | 0.40 |
| 1,2-pentanediol (Hydrolite^{®} 5) | 0.60 |
| SymDiol^{®} 68T (1.2-Hexanediol. 1.2-Octanediol. Tropolone) | 0.30 |
| Phenoxyethanol | 0.20 |
| Kühlstoff 2 | 1.50 |

**Tabelle F46**

| Silikonemulsion (Mengen in Gew.-%) | |
|---|---|
| **Bestandteile** | **Menge** |
| Potassium Cetyl Phosphate. Hydrogenated Palm Glycerides | 1.00 |
| Cyclohexasiloxane | 4.00 |
| Cetearyl Alcohol | 1.50 |
| Phenyl Trimethicone | 3.00 |
| Stearyl Heptanoate. Stearyl Caprylate | 3.00 |
| Dimethicone | 1.00 |
| Xanthan Gum | 0.20 |
| Isoamyl p-Methoxycinnamate | 5.00 |
| Wasser | Ad 100 |
| Methylpropanediol | 3.00 |
| 1,2-pentanediol (Hydrolite^{®} 5) | 0.30 |
| Diazolidinyl urea | 0.10 |
| Kühlstoff 5 | 1.50 |

**Tabelle F47**

| Microemulsionsgel (Mengen in Gew.-%) | |
|---|---|
| **Ingredient** | **Menge** |
| Glycerol isostearate | 1.80 |
| Octoxyglycerol | 1.00 |
| Ceteareth-15 | 5.20 |
| PEG-150 Distearate | 1.00 |
| Aluminium chlorohydrate | 5.00 |
| Isotridecylisononanoate | 3.30 |
| Cyclomethicone | 6.60 |
| 1,2-pentanediol (Hydrolite^{®} 5) | 0.70 |
| Euxyl^{®} K145 (Methylchloroisothiazolinone. Methylisothiazlinone. Bronopol) | 0.10 |
| Kühlstoff 1 | 1.00 |
| Wasser | Ad 100 |

**Tabelle F48**

| Lufterfrischer in Gelform (Mengen in Gew.-%) | |
|---|---|
| **Ingredient** | **Menge** |
| Demineralisiertes Wasser | Ad 100 |
| Genugel^{®} X-6424 (carrageenan) | 2.00 |
| Arkopal^{®} N 100 or Tergitol^{®} NP 10 (Emulsifer) | 3.50 |
| 1,2-pentanediol (Hydrolite^{®} 5) | 0.60 |
| Preventol^{®} D 7 (5-chloro-2-methyl-4-isothiazolin-3-one. 2-methyl-2H isothiazol-3-one) | 0.10 |
| Kühlstoff 1 | 1.00 |

**Tabelle F49**

| Textilreiniger (Mengen in Gew.-%) | |
|---|---|
| **Bestandteile (INCI)** | **Menge** |
| Aqua | 72.10 |
| Dialkylester ammomium methosulfate | 16.60 |
| Polydimethylsiloxane | 0.30 |
| Magnesiumchloride | 10.00 |
| 1,2-pentanediol (Hydrolite^{®} 5) | 0.60 |
| Mixture of 5-Chloro-2-methyl-2H-isothiazol-3-one and 2-Methyl-2H-isothiazol-3-one | 0.10 |
| Kühlstoff 1 (verkapselt) | 0.40 |

## Patentansprüche

1. Kühlstoffe ausgewählt aus der Gruppe, die gebildet wird von:

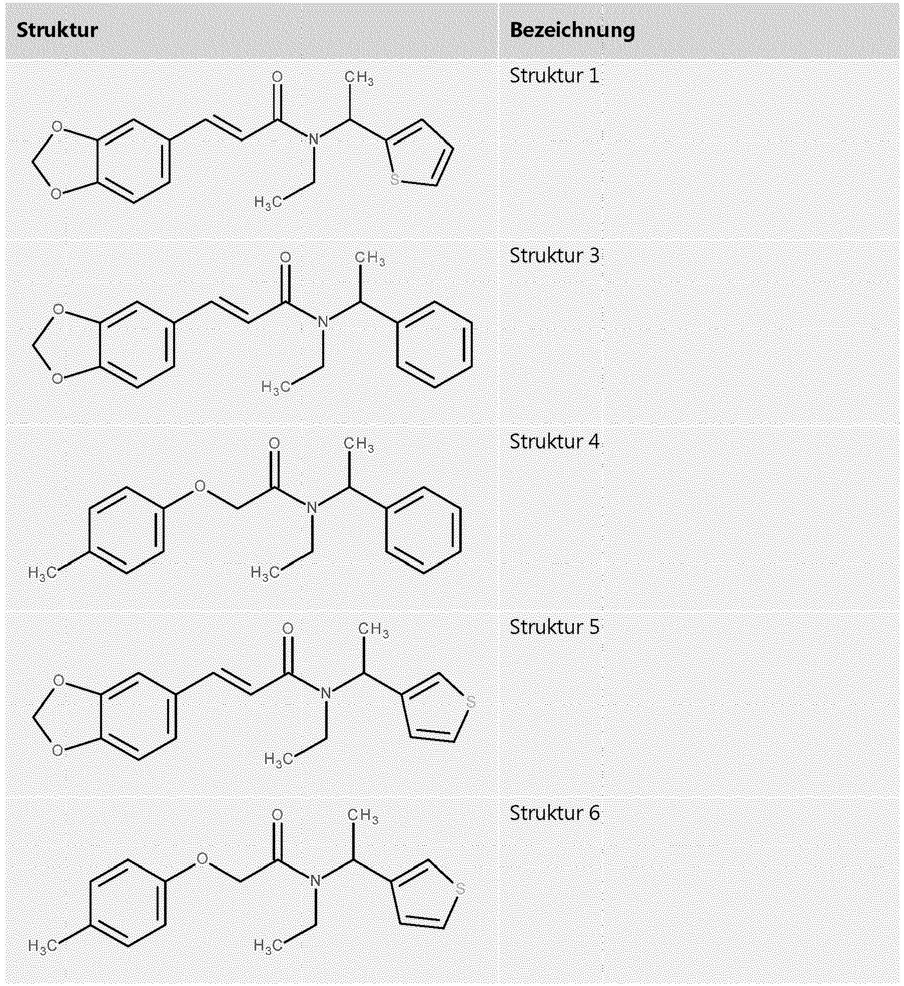

2. Physiologische Kühlstoffmischung enthaltend, bestehend aus oder im Wesentlichen bestehend aus:
(a) ein, zwei, drei oder mehreren Kühlstoffen gemäß Anspruch 1 und
(b1) mindestens einem weiteren physiologischen Kühlstoff.

3. Mischung nach Anspruch 2, **dadurch gekennzeichnet, dass** die physiologischen Kühlstoffe, die die Komponente (b1) bilden, ausgewählt aus der Gruppe, die gebildet wird von Menthol, Menthol Methyl Ether, Menthone Glyceryl Acetal (FEMA GRAS 3807), Menthone Glyceryl Ketal (FEMA GRAS 3808), Menthyl Lactate (FEMA GRAS 3748), Menthol Ethylene Glycol Carbonate (FEMA GRAS 3805), Menthol Propylene Glycol Carbonate (FEMA GRAS 3806), Menthyl-N-ethyloxamat, Monomethyl Succinate (FEMA GRAS 3810), Monomenthyl Glutamate (FEMA GRAS 4006), Menthoxy-1,2-propanediol (FEMA GRAS 3784), Menthoxy-2-methyl-1,2-propandiol (FEMA GRAS 3849) sowie den Menthancarbonsäureestern und -amiden WS-3, WS-4, WS-5, WS-12, WS-14 und WS-30 sowie deren Gemischen.

4. Mischung nach Anspruch 2, **dadurch gekennzeichnet, dass** die Komponenten (a) und (b1) im Gewichtsverhältnis von etwa 1:99 bis etwa 99:1 enthalten sind.

5. Aromazubereitung enthaltend, bestehend aus oder im Wesentlichen bestehend aus
(a) ein, zwei, drei oder mehreren Kühlstoffen gemäß Anspruch 1 und
(b2) mindestens einem Aromastoff.

6. Zubereitung nach Anspruch 5, **dadurch gekennzeichnet, dass** die Aromastoffe, die die Komponente (b2) bilden, ausgewählt sind aus der Gruppe, die gebildet wird von Acetophenon, Allylcapronat, alpha-Ionon, beta-Ionon, Anisaldehyd, Anisylacetat, Anisylformiat, Benzaldehyd, Benzothiazol, Benzylacetat, Benzylalkohol, Benzylbenzoat, beta-Ionon, Butylbutyrat, Butylcapronat, Butylidenphthalid, Carvon, Camphen, Caryophyllen, Cineol, Cinnamylacetat, Citral, Citronellol, Citronellal, Citronellylacetat, Cyclohexylacetat, Cymol, Damascon, Decalacton, Dihydrocumarin, Dimethylanthranilat, Dimethylanthranilat, Dodecalacton, Ethoxyethylacetat, Ethylbuttersäure, Ethylbutyrat, Ethylcaprinat, Ethylcapronat, Ethylcrotonat, Ethylfuraneol, Ethylguajakol, Ethylisobutyrat, Ethylisovalerianat, Ethyllactat, Ethylmethylbutyrat, Ethylpropionat, Eucalyptol, Eugenol, Ethylheptylat, 4-(p-Hydroxyphenyl)-2-butanon, gamma-Decalacton, Geraniol, Geranylacetat, Geranylacetat, Grapefruitaldehyd, Methyldihydrojasmonat (z.B. Hedion^{®}), Heliotropin, 2-Heptanon, 3-Heptanon, 4-Heptanon, trans-2-Heptenal, cis-4-Heptenal, trans-2-Hexenal, cis-3-Hexenol, trans-2-Hexensäure, trans-3-Hexensäure, cis-2-Hexenylacetat, cis-3-Hexenylacetat, cis-3-Hexenylcapronat, trans-2-Hexenylcapronat, cis-3-Hexenylformiat, cis-2-Hexylacetat, cis-3-Hexylacetat, trans-2-Hexylacetat, cis-3-Hexylformiat, para-Hydroxybenzylaceton, Isoamylalkohol, Isoamylisovalerianat, Isobutylbutyrat, Isobutyraldehyd, Isoeugenolmethylether, Isopropylmethylthiazol, Laurinsäure, Leavulinsäure, Linalool, Linalooloxid, Linalylacetat, Menthol, Menthofuran, Methylanthranilat, Methylbutanol, Methylbuttersäure, 2-Methylbutylacetat, Methylcapronat, Methylcinnamat, 5-Methylfurfural, 3,2,2-Methylcyclopentenolon, 6,5,2-Methylheptenon, Methyldihydrojasmonat, Methyljasmonat, 2-Methylmethylbutyrat, 2-Methyl-2-Pentenolsäure, Methylthiobutyrat, 3,1-Methylthiohexanol, 3-Methylthiohexylacetat, Nerol, Nerylacetat, trans,trans-2,4-Nonadienal, 2,4-Nonadienol, 2,6-Nonadienol, 2,4-Nonadienol, Nootkaton, delta Octalacton, gamma Octalacton, 2-Octanol, 3-Octanol, 1,3-Octenol, 1-Octylacetat, 3-Octylacetat, Palmitinsäure, Paraldehyd, Phellandren, Pentandion, Phenylethylacetat, Phenylethylalkohol, Phenylethylalkohol, Phenylethylisovalerianat, Piperonal, Propionaldehyd, Propylbutyrat, Pulegon, Pulegol, Sinensal, Sulfurol, Terpinen, Terpineol, Terpinolen, 8,3-Thiomenthanon, 4,4,2-Thiomethylpentanon, Thymol, delta-Undecalacton, gamma-Undecalacton, Valencen, Valeriansäure, Vanillin, Acetoin, Ethylvanillin, Ethylvanillinisobutyrat (= 3-Ethoxy-4-isobutyryloxybenzaldehyd), 2,5-Dimethyl-4-hydroxy-3(2H)-furanon und dessen Abkömmlinge (dabei vorzugsweise Homofuraneol (= 2-Ethyl-4-hydroxy-5-methyl-3(2H)-furanon), Homofuronol (= 2-Ethyl-5-methyl-4-hydroxy-3(2H)-furanon und 5-Ethyl-2-methyl-4-hydroxy-3(2H)-furanon), Maltol und Maltol-Abkömmlinge (dabei vorzugsweise Ethylmaltol), Cumarin und Cumarin-Abkömmlinge, gamma-Lactone (dabei vorzugsweise gamma-Undecalacton, gamma-Nonalacton, gamma-Decalacton), delta-Lactone (dabei vorzugsweise 4-Methyldeltadecalacton, Massoilacton, Deltadecalacton, Tuberolacton), Methylsorbat, Divanillin, 4-Hydroxy-2(oder 5)-ethyl-5(oder 2)-methyl-3(2H)furanon, 2-Hydroxy-3-methyl-2-cyclopentenon, 3-Hydroxy-4,5-dimethyl-2(5H)-furanon, Essigsäureisoamylester, Buttersäureethylester, Buttersäure-n-butylester, Buttersäureisoamylester, 3-Methyl-buttersäureethylester, n-Hexansäureethylester, n-Hexansäureallylester, n-Hexansäure-n-butylester, n-Octansäureethylester, Ethyl-3-methyl-3-phenylglycidat, Ethyl-2-trans-4-cis-decadienoat, 4-(p-Hydroxyphenyl)-2-butanon, 1,1-Dimethoxy-2,2,5-trimethyl-4-hexan, 2,6-Dimethyl-5-hepten-1-al und Phenylacetaldehyd, 2-Methyl-3-(methylthio)furan, 2-Methyl-3-furanthiol, bis(2-Methyl-3-furyl)disulfid, Furfurylmercaptan, Methional, 2-Acetyl-2-thiazolin, 3-Mercapto-2-pentanon, 2,5-Dimethyl-3-furanthiol, 2,4,5-Trimethylthiazol, 2-Acetylthiazol, 2,4-Dimethyl-5-ethylthiazol, 2-Acetyl-1-pyrrolin, 2-Methyl-3-ethylpyrazin, 2-Ethyl-3,5-dimethylpyrazin, 2-Ethyl-3,6-dimethylpyrazin, 2,3-Diethyl-5-methylpyrazin, 3-Isopropyl-2-methoxypyrazin, 3-Isobutyl-2-methoxypyrazin, 2-Acetylpyrazin, 2-Pentylpyridin, (E,E)-2,4-Decadienal, (E,E)-2,4-Nonadienal, (E)-2-Octenal, (E)-2-Nonenal, 2-Undecenal, 12-Methyltridecanal, 1-Penten-3-on, 4-Hydroxy-2,5-dimethyl-3(2H)-furanon, Guajakol, 3-Hydroxy-4,5-dimethyl-2(5H)-furanon, 3-Hydroxy-4-methyl-5-ethyl-2(5H)-furanon, Zimtaldehyd, Zimtalkohol, Methylsalicylat, Isopulegol sowie (hier nicht explizit genannte) Stereoisomere, Enantiomere, Stellungsisomere, Diastereomere, cis/trans-Isomere bzw. Epimere dieser Substanzen.

7. Zubereitung nach Anspruch 5, **dadurch gekennzeichnet, dass** die Aromastoffe, die die Komponente (b2) bilden, ausgewählt sind aus der Gruppe, die gebildet wird von Erythritol, Threitol, Arabitol, Ribotol, Xylitol, Sorbitol, Mannitol, Dulcitol, Lactitol, Miraculin, Monellin, Thaumatin, Curculin, Brazzein, Magap, Natriumcyclamat, Acesulfam K, Neohesperidin Dihydrochalcon, Saccharin Natriumsalz, Aspartam, Superaspartam, Neotam, Alitam, Sucralose, Stevioside, Rebaudioside, Lugduname, Carrelame, Sucrononate, Sucrooctate, Monatin, Phenylodulcin, Glycin, D-Leucin, D-Threonin, D-Asparagin, D-Phenylalanin, D-tryptophn, L-Prolin, Hernandulcin, Dihydrochalconglykoside, Glycyrrhizin, Glycerrhetinsäure, ihre Derivate und Salze, Extrakte von Lakritz (Glycyrrhizza glabra ssp.), Lippia dulcis Extrakte, Momordica ssp. Extrakte, Mogrosiden, Hydrangea dulcis und Steviosiden sowie deren Mischungen.

8. Zubereitung nach Anspruch 5, **dadurch gekennzeichnet, dass** die Komponenten (a) und (b2) im Gewichtsverhältnis von etwa 1:99 bis etwa 99:1 enthalten sind.

9. Kühlstoffe nach Anspruch 1 oder Mischungen nach Anspruch 2 oder Zubereitungen nach Anspruch 5, **dadurch gekennzeichnet, dass** diese verkapselt vorliegen.

10. Kosmetische oder pharmazeutische Zusammensetzung enthaltend mindestens einen Kühlstoff nach Anspruch 1 oder eine Mischung nach Anspruch 2.

11. Orale Zusammensetzung enthaltend mindestens einen Kühlstoff nach Anspruch 1 oder eine Mischung nach Anspruch 2 oder eine Zubereitung nach Anspruch 5.

12. Medikamente enthalten mindestens einen der Kühlstoffe gemäß Anspruch 1.

13. Medikamente enthalten mindestens einen der Kühlstoffe gemäß Anspruch 1 zur Verwendung zur Linderung von Schmerzen und Entzündungszuständen der Haut und der Schleimhäute.

14. Medikamente enthaltend die Kühlstoffe gemäß Anspruch 1 sowie pharmazeutische Wirkstoffe ausgewählt aus der Gruppe, die gebildet wird von Aspirin, Minoxidil, Erythromycin, Fenistil, Betamethason, Ibuprofen, Ketoprofen, Dicyclofenac, Metronidazol, Acylovir, Imiquimod, Terbafin, Cyclopiroxolamin sowie deren Gemische.

15. Medikamente enthaltend die Kühlstoffe gemäß Anspruch 1 sowie pharmazeutische Wirkstoffe vom Typ der NSAID.

16. Medikamente enthaltend die Kühlstoffe gemäß Anspruch 1 sowie pharmazeutische Wirkstoffe vom Typ der NSAID zur Verwendung in der Behandlung von Entzündungszuständen der Haut und der Schleimhaut sowie der Gelenke.

17. Medikamente nach den Ansprüche 13 bis 16, **dadurch gekennzeichnet, dass** sie die Kühlstoffe gemäß Anspruch 1 und die pharmazeutischen Wirkstoffe im Gewichtsverhältnis von etwa 1:99 bis etwa 10:90 enthalten.

18. Verfahren zur Erzeugung eines physiologischen Kühleffektes auf Haut oder Schleimhaut, umfassend die folgenden Schritte:
(i) Bereitstellen von mindestens einem Kühlstoff gemäß Anspruch 1 oder einer Mischung nach Anspruch 2,
(ii) gegebenenfalls Formulierung der Kühlstoffe in eine kosmetische, pharmazeutische oder orale Zusammensetzung; und
(iii) Inkontaktbringen der Zubereitungen aus Schritt (i) oder Schritt (ii) mit menschlicher Haut oder Schleimhaut.

19. Verfahren zur Verbesserung der geschmacklichen Eigenschaften von Aromastoffen umfassend die folgenden Schritte:
(i) Bereitstellen von mindestens einem Kühlstoff nach Anspruch 1 oder einer Kühlstoffmischung nach Anspruch 2 und mindestens einem Aromastoff;
(ii) Vermischen der beiden Komponenten sowie gegebenenfalls
(iii) Einarbeitung der Mischung in eine orale Zusammensetzung.

20. Verwendung von Kühlstoffen nach Anspruch 1 oder Mischungen nach Anspruch 2 zur Erzeugung eines physiologischen Kühleffektes auf Haut oder Schleimhaut.

21. Verwendung von Kühlstoffen nach Anspruch 1 zur Verbesserung der geschmacklichen Eigenschaften von Aromastoffen.

## Claims

1. Cooling agents selected from the group consisting of:
| **Structure** | **Reference** |
|---|---|
| 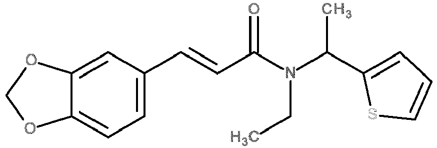 | Formula (I) |
| 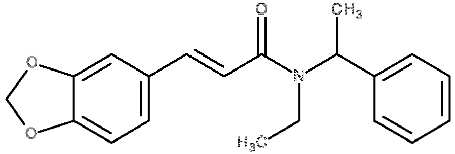 | Formula (II) |
| 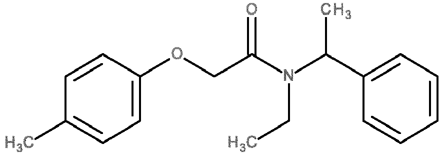 | Formula (III) |
| 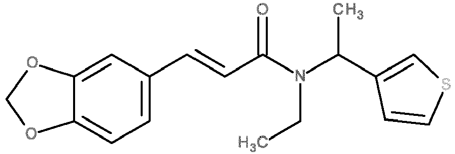 | Formula (IV) |
| 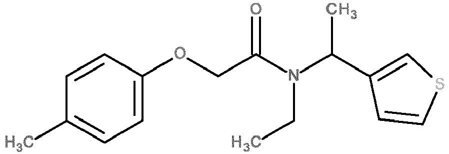 | Formula (V) |

2. Physiological coolant mixture comprising, consisting of or consisting essentially of:
(a) one, two, three or more refrigerants according to claim 1 and
(b1) at least one further physiological refrigerant.

3. The mixture according to claim 2, **characterized in that** the physiological refrigerants forming component (b1) are selected from the group consisting of menthol, menthol methyl ether, menthone glyceryl acetal (FEMA GRAS 3807), menthone glyceryl ketal (FEMA GRAS 3808), menthyl lactate (FEMA GRAS 3748), menthol ethylene glycol carbonate (FEMA GRAS 3805), Menthol Propylene Glycol Carbonate (FEMA GRAS 3806), Menthyl-N-ethyloxamate, Monomethyl Succinate (FEMA GRAS 3810), Monomenthyl Glutamate (FEMA GRAS 4006), Menthoxy-1, 2-propanediol (FEMA GRAS 3784), menthoxy-2-methyl-1,2-propanediol (FEMA GRAS 3849) and the menthanecarboxylic acid esters and amides WS-3, WS-4, WS-5, WS-12, WS-14 and WS-30 and mixtures thereof.

4. The mixture according to claim 2, **characterized in that** the components (a) and (b1) are contained in a weight ratio of about 1:99 to about 99:1.

5. A flavoring preparation comprising, consisting of or essentially consisting of
(a) one, two, three or more refrigerants according to claim 1 and
(b2) at least one flavoring agent.

6. The reparation according to claim 5, **characterized in that** the flavoring substances forming component (b2) are selected from the group formed by acetophenone, allyl capronate, alpha-ionone, beta-ionone, anisaldehyde, anisyl acetate, anisyl formate, benzaldehyde, benzothiazole, benzyl acetate, benzyl alcohol, benzyl benzoate, beta-ionone, butyl butyrate, butyl capronate, butylidene phthalide, carvone, camphene, caryophyllene, cineole, cinnamyl acetate, citral, citronellol, citronellal, citronellyl acetate, cyclohexyl acetate, cymol, damascone, decalactone, dihydrocoumarin, dimethyl anthranilate, dimethyl anthranilate, dodecalactone, ethoxyethyl acetate, ethyl butyric acid, ethyl butyrate, ethyl caprinate, ethyl capronate, ethyl crotonate, ethyl furaneol, ethyl guaiacol, ethyl isobutyrate, ethyl isovalerate, ethyl lactate, ethyl methyl butyrate, ethyl propionate, eucalyptol, eugenol, ethyl heptylate, 4-(p-hydroxyphenyl)-2-butanone, gamma-decalactone, geraniol, geranyl acetate, geranyl acetate, grapefruit aldehyde, methyl dihydrojasmonate, heliotropin, 2-heptanone, 3-heptanone, 4-heptanone, trans-2-heptenal, cis-4-heptenal, trans-2-hexenal, cis-3-hexenol, trans-2-hexenoic acid, trans-3-hexenoic acid, cis-2-hexenyl acetate, cis-3-hexenyl acetate, cis-3-hexenyl capronate, trans-2-hexenyl capronate, cis-3-hexenyl formate, cis-2-hexyl acetate, cis-3-hexyl acetate, trans-2-hexyl acetate, cis-3-hexyl formate, para-hydroxybenzylacetone, isoamyl alcohol, isoamyl isovalerate, isobutyl butyrate, isobutyraldehyde, isoeugenol methyl ether, isopropyl methyl thiazole, lauric acid, leavulinic acid, linalool, linalool oxide, linalyl acetate, menthol, menthofuran, methyl anthranilate, methyl butanol, methyl butyric acid, 2-methyl butyl acetate, methyl capronate, methyl cinnamate, 5-methyl furfural, 3,2,2-methyl cyclopentenolone, 6,5,2-methyl heptenone, methyl dihydrojasmonate, methyl jasmonate, 2-methyl methyl butyrate, 2-methyl-2-pentenolic acid, methyl thiobutyrate, 3,1-methyl thiohexanol, 3-methyl thiohexyl acetate, nerol, neryl acetate, trans,trans-2,4-nonadienal, 2,4-nonadienol, 2,6-nonadienol, 2,4-nonadienol, nootkatone, delta octalactone, gamma octalactone, 2-octanol, 3-octanol, 1,3-octenol, 1-octyl acetate, 3-octyl acetate, palmitic acid, paraldehyde, phellandrene, pentanedione, phenylethyl acetate, phenylethyl alcohol, phenylethyl alcohol, phenylethyl isovalerate, piperonal, propionaldehyde, propyl butyrate, pulegone, pulegol, sinensal, sulfurol, terpinene, terpineol, terpinolene, 8,3-thiomenthanone, 4,4,2-thiomethylpentanone, thymol, delta-undecalactone, gamma-undecalactone, valencene, valeric acid, vanillic acid, vanillin, acetoin, ethyl vanillin, ethyl vanillin isobutyrate (= 3-ethoxy-4-isobutyryloxybenzaldehyde), 2, 5-dimethyl-4-hydroxy-3(2H)-furanone and its derivatives (preferably homofuraneol (= 2-ethyl-4-hydroxy-5-methyl-3(2H)-furanone), homofuronol (= 2-ethyl-5-methyl-4-hydroxy-3(2H)-furanone and 5-ethyl-2-methyl-4-hydroxy-3(2H)-furanone), maltol and maltol derivatives (preferably ethylmaltol), coumarin and coumarin derivatives, gamma-lactones (preferably gamma-undecalactone, gamma-nonalactone, gamma-decalactone), delta-lactones (preferably 4-methyldeltadecalactone, massoilactone, deltadecalactone, tuberolactone), methyl sorbate, divanillin, 4-hydroxy-2(or 5)-ethyl-5(or 2)-methyl-3(2H)furanone, 2-hydroxy-3-methyl-2-cyclopentenone, 3-hydroxy-4,5-dimethyl-2(5H)-furanone, acetic acid isoamyl ester, butyric acid ethyl ester, butyric acid n-butyl ester, butyric acid isoamyl ester, 3-methyl-butyric acid ethyl ester, n-hexanoic acid ethyl ester, n-hexanoic acid allyl ester, n-hexanoic acid n-butyl ester, n-octanoic acid ethyl ester, ethyl 3-methyl-3-phenylglycidate, ethyl 2-trans-4-cis-decadienoate, 4-(p-hydroxyphenyl)-2-butanone, 1,1-dimethoxy-2,2,5-trimethyl-4-hexane, 2,6-dimethyl-5-hepten-1-al and phenylacetaldehyde, 2-methyl-3-(methylthio)furan, 2-methyl-3-furanethiol, bis(2-methyl-3-furyl)disulfide, furfuryl mercaptan, methional, 2-acetyl-2-thiazoline, 3-mercapto-2-pentanone, 2,5-dimethyl-3-furanethiol, 2,4,5-trimethylthiazole, 2-acetylthiazole, 2,4-dimethyl-5-ethylthiazole, 2-acetyl-1-pyrroline, 2-methyl-3-ethylpyrazine, 2-ethyl-3,5-dimethylpyrazine, 2-ethyl-3,6-dimethylpyrazine, 2,3-diethyl-5-methylpyrazine, 3-isopropyl-2-methoxypyrazine, 3-isobutyl-2-methoxypyrazine, 2-acetylpyrazine, 2-pentylpyridine, (E,E)-2,4-decadienal, (E,E)-2,4-nonadienal, (E)-2-octenal, (E)-2-nonenal, 2-undecenal, 12-methyltridecanal, 1-penten-3-one, 4-hydroxy-2,5-dimethyl-3(2H)-furanone, guaiacol, 3-hydroxy-4,5-dimethyl-2(5H)-furanone, 3-hydroxy-4-methyl-5-ethyl-2(5H)-furanone, cinnamaldehyde, cinnamic alcohol, methyl salicylate, isopulegol as well as stereoisomers, enantiomers, positional isomers, diastereomers, cis/trans isomers or epimers of these substances (not explicitly mentioned here). epimers of these substances.

7. The preparation according to claim 5, **characterized in that** the flavouring substances forming component (b2) are selected from the group formed by erythritol, threitol, arabitol, ribotol, xylitol, sorbitol, mannitol, dulcitol, lactitol, miraculin, monellin, thaumatin, curculin, brazzein, magap, sodium cyclamate, acesulfame K, neohesperidin dihydrochalcone, saccharin sodium salt, Aspartame, Superaspartame, Neotame, Alitame, Sucralose, Stevioside, Rebaudioside, Lugduname, Carrelame, Sucrononate, Sucrooctate, Monatine, Phenylodulcin, Glycine, D-Leucine, D-Threonine, D-Asparagine, D-phenylalanine, D-tryptophene, L-proline, hernandulcine, dihydrochalcone glycosides, glycyrrhizin, glycerrhetinic acid, its derivatives and salts, extracts of licorice (Glycyrrhizza glabra ssp.), Lippia dulcis extracts, Momordica ssp. extracts, mogrosides, Hydrangea dulcis and steviosides and mixtures thereof.

8. The preparation according to claim 5, **characterized in that** the components (a) and (b2) are contained in a weight ratio of about 1:99 to about 99:1.

9. The cooling agents according to claim 1 or mixtures according to claim 2 or preparations according to claim 5, **characterized in that** they are encapsulated.

10. A cosmetic or pharmaceutical composition comprising at least one cooling agent according to claim 1 or a mixture according to claim 2.

11. An oral composition comprising at least one cooling agent according to claim 1 or a mixture according to claim 2 or a preparation according to claim 5.

12. A medicament containing at least one of the cooling agents according to claim 1.

13. The medicaments containing at least one of the cooling agents according to claim 1 for use in alleviating pain and inflammatory conditions of the skin and mucous membranes.

14. The medicaments containing the cooling agents according to claim 1 and pharmaceutical active ingredients selected from the group formed by aspirin, minoxidil, erythromycin, fenistil, betamethasone, ibuprofen, ketoprofen, dicyclofenac, metronidazole, acylovir, imiquimod, terbafine, cyclopiroxolamine and mixtures thereof.

15. The medicaments containing the cooling agents according to claim 1 and active pharmaceutical ingredients of the NSAID type.

16. The medicaments containing the cooling agents according to claim 1 and pharmaceutical active ingredients of the NSAID type for use in the treatment of inflammatory conditions of the skin and mucous membrane and of the joints.

17. The medicaments according to claims 13 to 16, **characterized in that** they contain the cooling agents according to claim 1 and the active pharmaceutical ingredients in a weight ratio of about 1:99 to about 10:90.

18. A method for producing a physiological cooling effect on skin or mucous membrane, comprising the following steps:
(i) providing at least one cooling agent according to claim 1 or a mixture according to claim 2,
(ii) optionally formulating the cooling agents into a cosmetic, pharmaceutical or oral composition; and
(iii) bringing the compositions of step (i) or step (ii) into contact with human skin or mucosa.

19. A method for improving the taste properties of flavoring agents comprising the following steps:
(i) providing at least one cooling agent according to claim 1 or a cooling agent mixture according to claim 2 and at least one flavoring agent;
(ii) mixing the two components and optionally
(iii) incorporating the mixture into an oral composition.

20. The use of cooling agents according to claim 1 or mixtures according to claim 2 for producing a physiological cooling effect on the skin or mucous membrane.

21. The use of cooling agents according to claim 1 to improve the taste properties of flavoring agents.

## Revendications

1. Agents de refroidissement choisis dans le groupe constitué par :
| **Structure** | **Reference** |
|---|---|
| 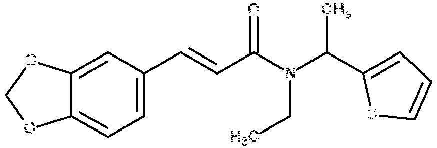 | Formula (I) |
| 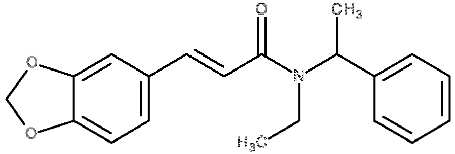 | Formula (II) |
| 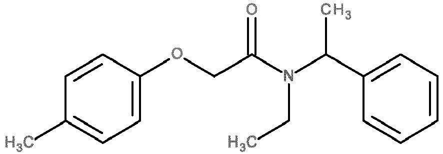 | Formula (III) |
| 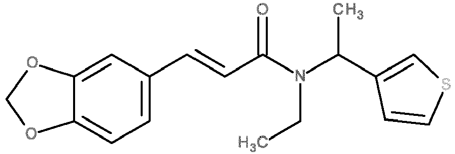 | Formula (IV) |
| 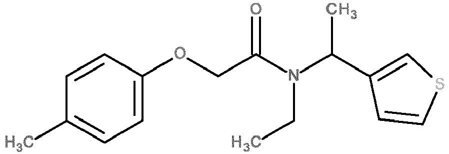 | Formula (V) |

2. Mélange de réfrigérants physiologiques comprenant, consistant en ou consistant essentiellement en :
(a) un, deux, trois ou plus réfrigérants selon la revendication 1 et
(b1) au moins un autre réfrigérant physiologique.

3. Mélange selon la revendication 2, **caractérisé par le fait que** les réfrigérants physiologiques formant le composant (b1) sont choisis dans le groupe constitué par le menthol, l'éther méthylique de menthol, l'acétal de glycéryle de menthone (FEMA GRAS 3807), le cétal de glycéryle de menthone (FEMA GRAS 3808), le lactate de menthyle (FEMA GRAS 3748), le car-bonate d'éthylène glycol de menthol (FEMA GRAS 3805), Menthol Propylène Glycol Carbonate (FEMA GRAS 3806), Menthyl-N-ethyloxamate, Monomethyl Succinate (FEMA GRAS 3810), Monomenthyl Glutamate (FEMA GRAS 4006), Menthoxy-1, 2-propanediol (FEMA GRAS 3784), menthoxy-2-méthyl-1,2-propanediol (FEMA GRAS 3849) et les esters et amides d'acide menthanecarboxylique WS-3, WS-4, WS-5, WS-12, WS-14 et WS-30 et leurs mélanges.

4. Mélange selon la revendication 2, **caractérisé par le fait que** les composants (a) et (b1) sont contenus dans un rapport en poids d'environ 1:99 à environ 99:1.

5. Préparation aromatique comprenant, consistant en ou essentiellement constituée de
(a) un, deux, trois ou plus réfrigérants selon la revendication 1 et
(b2) au moins un agent aromatisant.

6. La préparation selon la revendication 5, **caractérisée en ce que** les substances aromatisantes formant le composant (b2) sont choisies dans le groupe formé par l'acétophénone, le capronate d'allyle, l'alpha-ionone, la bêta-ionone, l'anisaldéhyde, l'acétate d'anisyle, le formiate d'anisyle, le benzaldéhyde, benzothiazole, acétate de benzyle, alcool benzylique, benzoate de benzyle, bêta-ionone, butyrate de butyle, capronate de butyle, phtalide de butylidène, carvone, camphène, caryophyllène, cinéole, acétate de cinnamyle, citral, citronellol, citronellal, acétate de citronellyl, acétate de cyclohexyle, cymol, damascone, décalactone, dihydrocoumarine, anthranilate de diméthyle, anthranilate de diméthyle, dodécalactone, acétate d'éthoxyéthyle, acide butyrique d'éthyle, butyrate d'éthyle, caprinate d'éthyle, capronate d'éthyle, crotonate d'éthyle, furanéol d'éthyle, gaïacol d'éthyle, isobutyrate d'éthyle, isovalérate d'éthyle, lactate d'éthyle, butyrate d'éthyle, propionate d'éthyle, eucalyptol, eugénol, heptylate d'éthyle, 4-(p-hydroxyphényl)-2-butanone, gamma-décalactone, géraniol, acétate de géranyle, acétate de géranyle, aldéhyde de pamplemousse, di-hydrojasmonate de héliotropine, 2-heptanone, 3-heptanone, 4-heptanone, trans-2-hepténal, cis-4-hepténal, trans-2-hexénal, cis-3-hexénol, acide trans-2-hexénoïque, acide trans-3-hexénoïque, acétate de cis-2-hexényle, acétate de cis-3-hexényle, capronate de cis-3-hexényle, capronate de cis-3-hexényle, capronate de trans-2-hexényle, formate de cis-3-hexényle, acétate de cis-2-hexyle, acétate de cis-3-hexyle, formate de cis-3-hexyle, parahydroxybenzylacétone, alcool isoamylique, isovalérate d'isoamyle, butyrate d'isobutyle, aldéhyde d'isobutyle, éther méthylique d'isoeugénol, thiazole méthylique d'isopropyle, acide laurique, acide lévulinique, linalol, oxyde de linalol, acétate de linalol, menthol, menthofurane, anthranilate de méthyle, butanol de méthyle, acide butyrique de méthyle, acétate de 2-méthylbutyle, capronate de méthyle, cinnamate de méthyle, 5-méthyl furfural, 3,2,2-méthyl cyclopentenolone, 6,5,2-méthyl heptenone, methyl dihydrojasmonate, méthyl jasmonate, 2-méthyl méthyl butyrate, acide 2-méthyl-2-penténolique, méthyl thiobutyrate, 3,1-méthyl thiohexanol, 3-méthyl thiohexyl acétate, nérol, acétate de nérol, trans,trans-2,4-nonadienal, 2,4-nonadienol, 2,6-nonadienol, 2,4-nonadienol, nootkatone, delta octalactone, gamma octalactone, 2-octanol, 3-octanol, 1,3-octénol, acétate de 1-octyle, acétate de 3-octyle, acide palmitique, paraldéhyde, phellandrène, pentanedione, acétate de phényléthyle, alcool phényléthylique, alcool phényléthylique, isovalérate de phényléthyle, pipéronal, propionaldéhyde, butyrate de propyle, pulegone, pulegol, sinensal, sulfurol, terpinène, terpinéol, terpinolène, 8,3-thiomenthanone, 4,4,2-thiométhylpentanone, thymol, delta-undécalactone, gamma-undécalactone, valencène, acide valérique, acide vanillique, vanilline, acétoïne, éthylvanilline, isobutyrate d'éthylvanilline (= 3-éthoxy-4-isobuty-ryloxybenzaldéhyde), 2, 5-diméthyl-4-hydroxy-3(2H)-furanone et ses dérivés [de préférence homofuranéol (= 2-éthyl-4-hydroxy-5-méthyl-3(2H)-furanone), homofuronol (= 2-éthyl-5-méthyl-4-hydroxy-3(2H)-furanone et 5-éthyl-2-méthyl-4-hydroxy-3(2H)-fura-none)], maltol et dérivés du maltol (de préférence éthylmaltol), cou-marine et dérivés de la coumarine, gamma-lactones (de préférence gamma-undécalactone, gamma-nonalactone, gamma-décalactone), delta-lactones (de préférence 4-méthyldeltadécalactone, massoilactone, delta-lactone, etc, massoilactone, deltadécalactone, tubérolactone), sorbate de méthyle, divanilline, 4-hydroxy-2(ou 5)-éthyl-5(ou 2)-méthyl-3(2H)furanone, 2-hydroxy-3-méthyl-2-cyclopenténone, 3-hydroxy-4,5-diméthyl-2(5H)-furanone, ester isoamylique de l'acide acétique, ester éthylique de l'acide butyrique, ester n-butylique de l'acide butyrique, ester isoamylique de l'acide butyrique, ester éthylique de l'acide 3-méthyl-butyrique, ester éthylique de l'acide n-hexanoïque, ester allylique de l'acide n-hexanoïque, ester n-butylique de l'acide n-hexanoïque, ester éthylique de l'acide n-oc-tanoïque, 3-méthyl-3-phénylglycidate d'éthyle, 2-trans-4-cis-décadiénoate d'éthyle, 4-(p-hydroxyphényl)-2-butanone, 1,1-diméthoxy-2,2,5-triméthyl-4-hexane, 2,6-diméthyl-5-heptène-1-al et phénylacétal-déhyde, 2-méthyl-3-(méthylthio)furane, 2-méthyl-3-fu-ranethiol, bis(2-méthyl-3-furyl)disulfure, furfuryl mercaptan, méthional, 2-acétyl-2-thia-zoline, 3-mercapto-2-pentanone, 2,5-diméthyl-3-furanethiol, 2,4,5-triméthylthiazole, 2-acétylthiazole, 2,4-diméthyl-5-éthylthiazole, 2-acétyl-1-pyrroline, 2-méthyl-3-éthylpyra-zine, 2-éthyl-3,5-diméthylpyrazine, 2-éthyl-3,6-diméthylpyrazine, 2,3-diéthyl-5-méthyl-pyrazine, 3-isopropyl-2-méthoxypyrazine, 3-isobutyl-2-méthoxypyrazine, 2-acétylpyra-zine, 2-pentylpyridine, (E, E)-2,4-décadiénal, (E,E)-2,4-nonadiénal, (E)-2-octénal, (E)-2-nonénal, 2-undécénal, 12-méthyltridécanal, 1-pentène-3-one, 4-hydroxy-2,5-diméthyl-3(2H)-furanone, gaïacol, 3-hydroxy-4,5-diméthyl-2(5H)-furanone, 3-hydroxy-4-méthyl-5-éthyl-2(5H)-furanone, cinnamaldéhyde, alcool cinnamique, salicylate de méthyle, isopulegol ainsi que les stéréoisomères, énantiomères, isomères de position, diastéréoisomères, isomères cis/trans ou épimères de ces substances (non explicitement mentionnés ici), et d'épimères de ces substances.

7. La préparation selon la revendication 5, **caractérisée par le fait que** les substances aro-matisantes formant le composant (b2) sont choisies dans le groupe formé par l'érythritol, le thréitol, l'arabitol, le ribotol, le xylitol, le sorbitol, le mannitol, l'acide acétylsalicylique et l'acide acétylsalicylique, sorbitol, mannitol, dulcitol, lactitol, miraculine, monelline, thau-matine, curculine, brazzéine, magap, cyclamate de sodium, acésulfame K, néohes-péridine dihy-drochalcone, sel de sodium de saccharine, aspartame, Superaspartame, Neotame, Alitame, Sucralose, Stevioside, Rebaudioside, Lugduname, Carrelame, Sucrononate, Sucrooctate, Monatine, Phénylodulcine, Glycine, D-Leucine, D-Thréonine, D-Asparagine, D-phénylalanine, D-tryptophène, L-proline, hernandulcine, glycosides de dihydrochalcone, glycyrrhizine, acide glycérhétinique, ses dérivés et ses sels, extraits de réglisse (Glycyrrhizza glabra ssp.), d'extraits de Lippia dulcis, d'extraits de Momordica ssp., de mogrosides, d'Hydrangea dulcis et de stéviosides et leurs mélanges.

8. La préparation selon la revendication 5, **caractérisée par le fait que** les composants (a) et (b2) sont contenus dans un rapport de poids d'environ 1:99 à environ 99:1.

9. Agents réfrigérants selon la revendication 1 ou mélanges selon la revendication 2 ou préparations selon la revendication 5, **caractérisés par le fait qu'**ils sont encapsulés.

10. Composition cosmétique ou pharmaceutique comprenant au moins un agent réfrigérant selon la revendication 1 ou un mélange selon la revendication 2.

11. Composition orale comprenant au moins un agent réfrigérant selon la revendication 1 ou un mélange selon la revendication 2 ou une préparation selon la revendication 5.

12. Médicament contenant au moins un des agents réfrigérants selon la revendication 1.

13. Le médicament contenant au moins un des agents rafraîchissants selon la revendication 1, destinés à soulager la douleur et les états inflammatoires de la peau et des membranes muqueuses.

14. Le médicament contenant les agents réfrigérants selon la revendication 1 et des principes actifs pharmaceutiques choisis dans le groupe formé par l'aspirine, le minoxidil, l'érythromycine, le fenistil, la bétaméthasone, l'ibuprofène, le kétoprofène, le dicyclofénac, le métronidazole, l'acylovir, l'imiquimod, la terbafine, la cyclopiroxolamine et leurs mélanges.

15. Le médicament contenant les agents réfrigérants selon la revendication 1 et des principes actifs pharmaceutiques du type AINS.

16. Le médicament contenant les agents réfrigérants selon la revendication 1 et des principes actifs pharmaceutiques du type AINS pour le traitement des affections inflammatoires de la peau, des muqueuses et des articulations.

17. Le médicament selon les revendications 13 à 16, **caractérisés en ce qu'**ils contiennent les agents réfrigérants selon la revendication 1 et les principes pharmaceutiques actifs dans un rapport de poids d'environ 1:99 à environ 10:90.

18. Méthode pour produire un effet de refroidissement physiologique sur la peau ou les muqueuses, comprenant les étapes suivantes :
(i) fournir au moins un agent réfrigérant selon la revendication 1 ou un mélange selon la revendication 2,
(ii) formuler éventuellement les agents réfrigérants dans une composition cosmétique, pharmaceutique ou orale ; et
(iii) mettre les compositions de l'étape (i) ou de l'étape (ii) en contact avec la peau ou les muqueuses humaines.

19. Méthode d'amélioration des propriétés gustatives des agents aromatisants comprenant les étapes suivantes :
(i) fournir au moins un agent réfrigérant selon la revendication 1 ou un mélange d'agents réfrigérants selon la revendication 2 et au moins un agent aromatique ;
(ii) mélanger les deux composants et éventuellement
(iii) incorporer le mélange dans une composition orale.

20. Utilisation d'agents réfrigérants selon la revendication 1 ou de mélanges selon la revendication 2 pour produire un effet physiologique de refroidissement sur la peau ou les muqueuses.

21. Utilisation d'agents réfrigérants selon la revendication 1 pour améliorer les propriétés gustatives des agents aromatisants.
